# EUROPEAN PATENT SPECIFICATION

(11) **EP 4 370 687 B1**
(45) Date of publication and mention of the grant of the patent: **09.04.2025**
(21) Application number: 22740363.1
(22) Date of filing: 28.06.2022
(51) Int. Cl.: C12N 15/76, C12P 21/00

(54) **NEW SYSTEMS FOR PRODUCING RECOMBINANT PROTEINS**
NEUE SYSTEME ZUR HERSTELLUNG REKOMBINANTER PROTEINE
NOUVEAUX SYSTÈMES DE PRODUCTION DE PROTÉINES RECOMBINANTES

(30) Priority: 12.07.2021 IT 202100018254
(43) Date of publication of application: 22.05.2024
(73) Proprietor: Nemysis Ltd., Dublin 7 D07 F6DC (IE)
(72) Inventor: CAVALETTI, Linda, 22069 Rovellasca (CO) (IT); CARENZI, Giacomo Enrico, 21052 Busto Arsizio (VA) (IT); KORMANEC, Jan, 84551 Bratislava (SK); HOMEROVA, Dagmar, 84551 Bratislava (SK)
(74) Representative: Manfredi, Irene
(86) International application number: PCT/EP2022/067738
(87) International publication number: WO 2023/285135

(56) References cited:
- WO-A1-2021/013553
- CN-A- 109 554 415
- CN-A- 109 897 862
- BAI CHAOXIAN ET AL: "Exploiting a precise design of universal synthetic modular regulatory elements to unlock the microbial natural products in Streptomyces", vol. 112, no. 39, 29 September 2015 (2015-09-29), pages 12181 - 12186, XP055906170, ISSN: 0027-8424, Retrieved from the Internet <URL:https://www.ncbi.nlm.nih.gov/pmc/articles/PMC4593075/pdf/pnas.201511027.pdf> DOI: 10.1073/pnas.1511027112

## Description

### FIELD OF THE INVENTION

The present invention is directed to new systems for the production of recombinant proteins in *Streptomyces* host strain.

The new system includes vectors and new recombinant host strains, in particular for the production of *Actinoallomurus* endopeptidases.

### BACKGROUND

Recombinant DNA (rDNA) technology offers a very potent set of technical platforms for the controlled and scalable production of polypeptides of interest by relatively inexpensive procedures. Recombinant proteins are typically obtained through recombinant DNA technology in *Escherichia coli, Saccharomyces cerevisiae,* in insect, hamster and mammalian cells. There is however a demand for improving the production of big quantities of recombinant proteins; furthermore, there are very strict requirements to be fulfilled when proteins are produced for human use, for instance for use as food supplements and/or as medicaments in the prevention and/or treatment of human diseases. Streptomycetes are regarded as a safe source of proteins for human alimentary use. Two examples of food enzymes sourced from *Streptomyces spp* are: glucose isomerases used for fructose syrup production (Anné, et al. Molecular Cell Research, 2014), and the widely exploited transglutaminase from S. *mobaraensis,* used in food industry for its properties in improving the texture and overall quality of final food products, such as processed meat and fish products, as well as diary and baked food (Kieliszek, et al. Folia Microbiologica, 2013).

*S. lividans* cells are known to be efficient host cells for the production of recombinant proteins, since recombinant proteins expressed by said cells can be directly secreted and released in the culture medium. However, different proteins are obtained at very different yields in *S. lividans,* this result being unpredictable (Anné, et al. Molecular Cell Research, 2014).

To improve the yield of heterologous proteins, a collection of derivative strains of *S. lividans* TK24 (TK24 Taxonomy ID: 457428) have been constructed by sequential deletion of known potentially interfering secondary metabolite gene clusters. Novakova et al. (Applied Microbiology and Biotechnology, 2018) disclose efficient production of mithramycin A, with yields close to 3 g/L, using this optimized collection of strains.

Still reproducibility of high yields with any polypeptide-encoding polynucleotide is not guaranteed; moreover, there is still room for further improvements in the provision of recombinant proteins production systems.

The present invention is aimed at providing improved systems for efficient manufacturing of recombinant proteins in *Streptomyces,* preferably *Streptomyces lividans* (*S. lividans*) host strains.

In particular, the system of the invention has been found to be particularly useful for the production of big quantities of a recombinant endopeptidase (endopeptidase 40 or E40) of the *Actinoallomurus* strain.

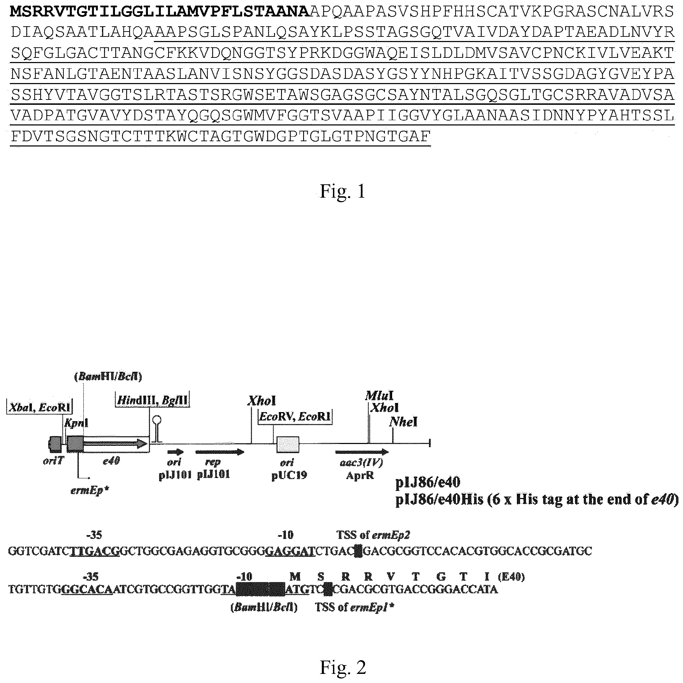

E40 native protein consists of 398 amino acid residues (see SEQ ID NO: 3); the N-terminal signal peptide has been identified between positions 1 and 27, and it has been predicted that the mature form of the protein (E40mat) is a 32.5 kDa polypeptide of sequence SEQ ID NO: 1, starting from position 74 of the native protein (see Fig. 1).

"E40pre-pro" is hereafter used to identify the native pre-pro-endopeptidase, including its native signal peptide, consisting of sequence SEQ ID NO: 3, or derivatives thereof, such as tagged E40pre-pro having sequence SEQ ID NO:4 (his-tagged E40pre-pro); "E40pre" is hereafter used to identify E40 pro-enzyme, without the signal peptide, consisting of aminoacidic sequence SEQ ID NO: 9; "E40mat" is hereafter used to identify the mature endopeptidase of aminoacidic sequence consisting of SEQ ID NO: 1, or derivatives thereof, such as tagged E40mat having aminoacidic sequence of SEQ ID NO: 2 (E40Hismat).

Said endopeptidase has been first disclosed in WO2013083338 and it has shown to provide very rapid and efficient degradation of gluten peptides into non-toxic peptides, being active at the whole pH range of the gastric and intestinal environment (enzymatic activity is shown in the range of pH 3-6 with optimum at pH 5) and being moreover resistant to degradation by gastrointestinal endogenous enzymes. This makes said *Actinoallomurus* endopeptidases very suitable for being used in the treatment and/or prevention of Celiac Disease (CD) and CD-associated disorders. There is therefore a strong interest in developing efficient and inexpensive methods of manufacturing said *Actinoallomurus* endopeptidase.

WO2021013553 discloses a method for manufacturing recombinant *Actinoallomurus* endopeptidases in a *S. lividans* host cell, wherein expression of E40 is preferably driven in *S. lividans* TK24 strain by a high-copy replicative expression vector (pIJ86, SEQ ID NO:7).

Several replicative plasmids have been successfully used, especially for their high-copy number, for overproduction of many biotechnologically relevant heterologous proteins in Streptomycetes. Replicative vectors have however some drawbacks; for instance, they can only be transferred to Streptomycetes by laborious protoplast transformation and they need a permanent antibiotic selection for stable maintenance in Streptomycetes.

Integrating vectors containing a DNA segment encoding attachment/integration functions (Att/Int) for site-specific integration into specific sites of the *Streptomyces* genome (such as an *attB* site for specific integrase) have also been employed for expressing recombinant proteins in *Streptomyces.* Most of the site-specific integration systems are based on various actinomycete temperate bacteriophages (actinophages) identified in *Streptomyces* species. Temperate bacteriophages (and integration vectors prepared using their functional elements) are integrated into the host chromosome at a specific site by a recombination process that requires specialized attachment sites, *attP* in the phage (or in the integration vector) and *attB* in the host chromosome.

Despite several benefits, these Att/Int systems also have some limitations, such as the fact that they integrate the entire integration plasmid with the *E. coli* replicon, the integrase gene and the resistance marker gene. This can be a significant biotechnological obstacle, as the stability of the constructs can be affected. In fact, the instability of several Att/Int systems has been reported in several *Streptomyces* strains (Kormanec J. et al., Applied Microbiology and Biotechnology 103:5463-5482, 2019). Therefore, these integrating vectors method have been considered unsuitable for the construction of stable biotechnological production strains.

Moreover, several other elements also participate in the efficiency of a recombinant protein production system, such as the regulatory elements in the expression cassettes cloned into the expression vector.

Therefore, the achievement of a satisfying yield of protein and the avoidance of undesired drawbacks is never an easy and clear-cut task in the field of recombinant proteins production.

The present invention provides a surprisingly improved system for producing recombinant proteins, and preferably recombinant *Actinoallomurus* endopeptidases, in *Streptomyces,* preferably *S. lividans,* host cells.

### BRIEF DESCRIPTION OF THE INVENTION

The system of the invention for the production of recombinant proteins in *Streptomyces* host cells, preferably of *S. lividans* strains, includes new recombinant vectors bearing an expression cassette comprising a polynucleotide encoding for the recombinant protein of interest under the regulation of a strong promoter, which is an engineered *kasO* promoter (*kasOp**)*,* of sequence SEQ ID NO: 10, and a regulatory element, which is a synthetic variant SR40 ribosome-binding site (RBS) based on PhiC31 capsid protein gene (Bai et al. 2015, Proc Natl Acad Sci USA 112, 12181-12186) of sequence SEQ ID NO: 11. The recombinant vectors of the invention are single-site integrating vectors based on the phage PhiBT1, preferably integrating at its *attB* site in *Streptomyces* genomes.

The polynucleotide encoding for the recombinant protein of interest encodes for a recombinant pre-protein that includes a signal peptide. In preferred aspects, said signal peptide is a heterologous signal peptide, more preferably signal peptide vsi of subtilisin inhibitor of *Streptomyces venezuelae* (Lammertyn et al. 1997 Appl Environ Microbiol 63, 1808- 1813), having sequence SEQ ID NO: 12.

Preferably, the recombinant protein of interest is an *Actinoallomurus* endopeptidase, preferably *Actinoallomurus* endopeptidases 40 (E40), a biologically active fragment, a variant, or a derivative thereof.

The systems of the invention also include new recombinant host strains comprising the new vectors. The present invention is also directed to the use of the new systems of the invention for the production of recombinant proteins by secretion of the same in the culture medium of the host cell, in particular for the production of *Actinoallomurus* endopeptidases, and to methods of production of the recombinant protein by means of the new systems of the invention.

Surprisingly, despite the use of a single copy vector, the system of the invention provides expression of recombinant protein that is stable and in high amounts.

### BRIEF DESCRIPTION OF FIGURES

Figure 1: Sequence of native E40 pre-pro-enzyme (E40pre-pro). Its signal peptide is shown in bold and the mature enzyme is underlined.
Figure 2: Scheme of the prior art recombinant vector pIJ86/e40 containing the *e40* gene encoding for E40pre endopeptidase (with and without 6xHis tag), inserted between *Bam*HI and *HindIII* sites of the cloning vector pIJ86. The sequence of the *ermEp** promoter region (SEQ ID NO: 30) with -10 and -35 regions of promoters (bold and underlined), TSS (bold, underlined and highlighted), and the *Bam*HI/*Bcl*I fusion with *e40* is indicated below the scheme of the plasmid. Amino acids of E40 are in the second position of each codon.
Figure 3: Scheme of the expression vector pMU1s-kasOpSR40vsi3mRFPA. The sequence of vsi-mRFP fusion with inserted restriction sites (bold and underlined) is indicated below the scheme of the plasmid. Amino acids of Vsi signal peptide (in bold) and mRFP (red font) are in the second position of each codon.
Figure 4: 13.5% SDS-PAGE of 20 ul samples of medium after secretion of mRFP in S. *lividans* TK24 with pMU1s-kasOpSR40vsi3mRFPA grown in TSB medium to various time points (two individual clones). Lanes: 1=LMW standard; 2-5=TK24 pMU1s-kasOpSR40vsi3mRFPA grown for 24h, 48h, 72h, 146h respectively; 10=TK24 grown for 48h.
Figure 5: Scheme of the cloning vector pLit-kasOpSR40vsi3mRFP containing the strong *kasOp** promoter, optimized strong SR40 RBS, and inserted single *Nde*I site in ATG codon of the vsi-mRFP reporter fusion gene.
Figure 6: Scheme of the recombinant plasmids pLit-e40 and pLit-e40His, containing the entire *e40* gene with its original signal peptide sequence (with and without 6 x His tag, respectively) inserted between *Nde*I and AvrII sites of the cloning vector pLit kasOpSR40vsi3mRFP. The sequence of the *kasOp** promoter with -10 and -35 regions of the promoter (bold and underlined), its TSS (bold, underlined and highlighted), RBS (bold and underlined), and *Nde*I fusion with *e40* is indicated below the scheme of the plasmid. Amino acids of E40 are in the second position of each codon.
Figure 7: Scheme of the cloning expression vector pMU1s-kasOpSR40gusA containing the strong *kasOp** promoter, optimized strong SR40 RBS, and inserted single *Nde*I site in ATG codon of the *gusA* reporter gene. The sequence of this promoter and RBS region with -10 and -35 regions of the promoter (bold and underlined), its TSS (bold, underlined and -highlighted) and RBS (bold and underlined) is indicated below the scheme of the plasmid.

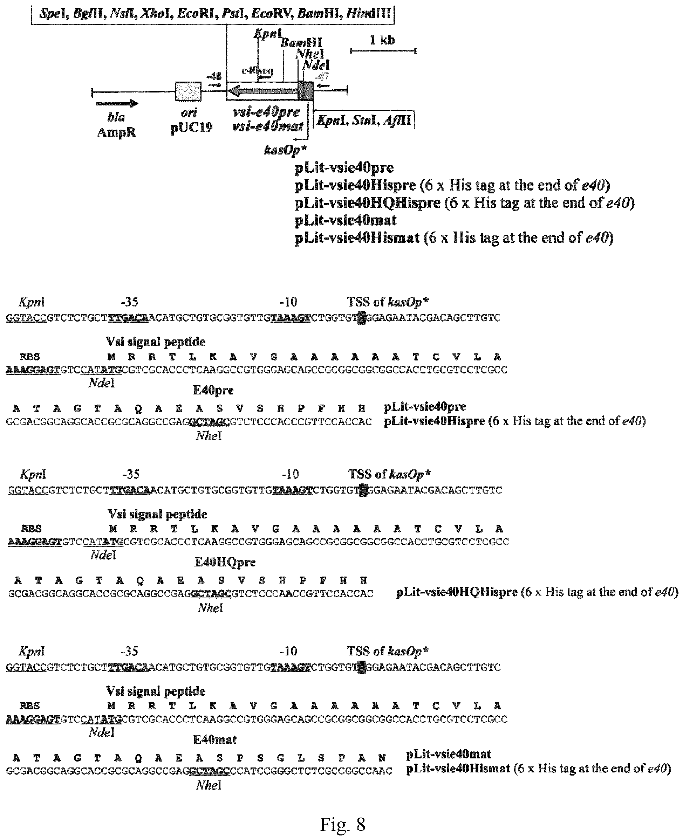

Figure 8: Scheme of the recombinant plasmids pLit-vsie40pre, pLit-e40Hispre, pLitvsie40HQHispre, pLit-vsie40mat, pLit-vsie40Hismat, containing the *vsi-e40pre* gene fusion of Vsi with pro enzyme E40pre and the *vsi-e40mat* gene fusion of Vsi with mature enzyme E40mat (with and without 6 x His tag, respectively), inserted between *Nhe*I and *HindIII* sites of the cloning vector pLit-kasOpSR40vsi3mRFP. The sequence of the *kasOp** promoter with -10 and -35 regions of the promoter (bold and underlined), its TSS (bold, underlined and -highlighted), RBS (bold and underlined), *Nde*I and *Nhe*I of the vsi-E40 fusion site is indicated below plasmid.
Figure 9: Schemes of the recombinant plasmids pMU1s-e40, pMU1s-e40His, pMU1svsie40pre, pMU1s-vsie40Hispre, pMU1s-vsie40HQHispre, pMU1s vsie40mat, pMU1s-vsie40Hismat, containing all the forms of *e40* gene fusions as *Kpn*I *Eco*RV DNA fragments from pLit plasmids (see Figs. 6, 8) inserted between *Kpn*I and *EcoRV* sites of the cloning vector pMU1s-kasOpSR40gusA (Fig. 7).
Figure 10: Schemes of the recombinant plasmids pIJ86-kasOpe40, pIJ86-kasOpe40His, pIJ86-vsie40pre, pIJ86-vsie40Hispre, pIJ86-vsie40HQHispre, pIJ86-vsie40mat, pIJ86-vsie40Hismat, containing all the forms of *e40* gene fusions (as indicated in Fig. 6, 8) (with and without 6 x His tag, respectively) as *Kpn*I-*Eco*RV DNA fragments from pLit plasmids (Fig. 6, 8) inserted between *KpnI* and *HindIII* sites of the cloning vector pIJ86 (Fig. 2).
Figure 11: Scheme of the recombinant plasmid pMU1s-ermEe40, containing *ermEp*-e40His* fusion from the plasmid pIJ86/e40His containing *e40* gene with 6 x His tag.
Figure 12: E40 activity in wild-type and mutant *S. lividans* RedStrep 1.3 strain conjugated with high copy replicative plasmids of the prior art.
Figure 13: E40 activity in wild-type and mutant *S. lividans* RedStrep strains conjugated with single-site integrating plasmids expressing native E40 under the *ermEp** promoter.
Figure 14: E40 activity in wild-type *S. lividans* strains conjugated with single-site integrating plasmids expressing native E40 (A) or His-tagged E40 (B) under the *kasOp** promoter and optimized strong SR40 RBS according to the invention.
Figure 15: E40 activity in wild-type S. *lividans* strains conjugated with high copy pIJ86 plasmids expressing E40 (A) or E40 His-tagged (B) under the *kasOp** promoter and optimized strong SR40 RBS.
Figure 16: E40 activity in wild-type *S. lividans* strains conjugated with high copy pIJ86 plasmids pIJ86 comprising *vsi-e40pre* (A), His-tagged *vsi-e40pre* (B), *vsi-e40mat* (C), or His-tagged *vsi-e40mat* fusion genes (D), under the *kasOp** promoter and optimized strong SR40 RBS.
Figure 17: E40 activity in wild-type *S. lividans* strains conjugated with single-site integrating plasmids comprising *vsi-e40pre* (A) or His-tagged *vsi-e40pre* (B), under the *kasOp** promoter and optimized strong SR40 RBS.
Figure 18: E40 activity in wild-type or mutant *S. lividans* RedStrep strains conjugated with single-site integrating plasmids expressing native E40 (A) or native E40 His-tagged (B) under the *kasOp** promoter and optimized strong SR40 RBS.
Figure 19: E40 activity in wild-type or mutant *S. lividans* RedStrep strains conjugated with single-site integrating plasmids expressing *vsi-e40pre* (A) or His-tagged *vsi-e40pre* (B), under the *kasOp** promoter and optimized strong SR40 RBS.

### DETAILED DESCRIPTION OF THE INVENTION

The present invention is first directed to a recombinant vector bearing an expression cassette for heterologous expression of a recombinant protein in a *Streptomyces* host cell, said cassette comprising: a promoter, a regulatory element down-stream said promoter, and a polynucleotide encoding a recombinant protein, down-stream said promoter and said regulatory element, and operably linked to the same.

The terms "vector", "expression vector" and "plasmid" are used herein interchangeably. The expression vector for heterologous expression of a recombinant protein in a *Streptomyces* host cell of the invention is a single-site integrating vector containing the *attP* site and integrase gene from PhiBT1 phage, more preferably a vector integrating at its *attB* site of *Streptomyces,* having sequence SEQ ID NO: 13.

Examples of single-site integrating vectors according to the present invention include those described in Kormanec J. et al., Applied Microbiology and Biotechnology (2019) 103:5463-5482.

In preferred embodiments, the single-site integrating vector is a vector having the backbone of pMU1 vector (see Craney et al. 2007, Nucleic Acids Res 35, e46).

The promoter of said expression cassette is an engineered *kasO* promoter *(kasOp*)* of sequence SEQ ID NO: 10; the regulatory element of the expression cassette is a synthetic ribosome-binding site (SR40 RBS) of sequence SEQ ID NO: 11; and the polynucleotide encoding for the recombinant protein of interest is a polynucleotide encoding for a recombinant protein that includes the signal peptide.

The expression cassette of the invention is particularly suitable for heterologous expression of recombinant proteins in *Streptomyces* host cells, wherein the recombinant protein is expressed by the host cell, cultured in a medium under suitable conditions for the growth of the host cell, and it is secreted in said culture medium. The signal peptide drives secretion of the protein in the medium.

Optionally the signal peptide is a heterologous signal peptide, more preferably Vsi signal peptide of subtilisin inhibitor of *Streptomyces venezuelae* having sequence SEQ ID NO: 12. Surprisingly, the use of a heterologous signal peptide fused to the protein of interest, according to the invention, provides secretion of the recombinant protein as good as the protein's native signal peptide.

In preferred embodiments of the present invention, the recombinant protein encoded by the polynucleotide of the expression cassette is a recombinant *Actinoallomurus* endopeptidase with glutenase activity; more preferably said endopeptidase is endopeptidase 40 (E40) of sequence comprising SEQ ID NO: 1, a biologically active fragment of E40, a naturally occurring allelic variant of E40; or an endopeptidase of sequence having at least 60%, 70%, 80%, 90% or 95% of identity to SEQ ID NO: 1. SEQ ID NO: 1 is the polypeptide sequence of the mature form of E40.

Therefore, preferably the expression cassette comprises a polynucleotide encoding for the endopeptidase E40 of sequence comprising SEQ ID NO: 1, for a biologically active fragment of E40, for a naturally occurring allelic variant of E40, or for an endopeptidase of sequence having at least 60%, 70%, 80%, 90% or 95% of identity to SEQ ID NO: 1. The term "biologically active fragment" of E40 refers to portions of the endopeptidase which maintain its specific glutenase activity. A polynucleotide encoding for a "biologically-active fragment" of E40 can be identified as disclosed by WO2021013553. The polynucleotide encoding for the recombinant protein of interest can have sequence that differs from the annotated nucleic acid sequence due to degeneracy of the genetic code and thus it encodes the same protein encoded by a polynucleotide having the annotated nucleic acid sequence.

The terms "identity" or "homology" when referred to a nucleotide or aminoacidic sequence are herein used interchangeably and refer to the degree to which two polynucleotide or polypeptide sequences are identical or homologous on a residue-by-residue basis over a particular region of comparison. The alignment and the percent identity or homology can be determined using any suitable software program known in the art, for example those described in Current Protocols in Molecular Biology (Ausubel F.M. et al., "Commercially Available Software", Current Protocols in Molecular, 1987, Supplement 30, Section 7.7.18, Table 7.7.1). Preferred programs include the GCG Pileup program, FASTA (Pearson R. and Lipman D. J. "Improved Tools for Biological Sequence Analysis" Proc. Natl., Acad. Sci. USA, 1988, 85, 2444-2448), and BLAST (Altschul S.F., Gish W., Miller W., Myers E.W., Lipman D.J. "Basic local alignment search tool" J. Mol. Biol., 1990, 215, 403-410).

The term "allelic variant" denotes any of two or more alternative forms of a gene occupying the same chromosomal locus. Allelic variation arises naturally through mutation, and may result in phenotypic polymorphism within populations. Gene mutations can be silent (no change in the encoded polypeptide) or may encode polypeptides having altered aminoacidic sequence. The term allelic variant refers also to a protein encoded by an allelic variant of a gene.

The polynucleotide encoding for the recombinant protein of interest can encode for a protein that is operatively-fused to another polypeptide, for instance a tag, such as a histidine-tag. For instance, said polynucleotide can be a polynucleotide encoding for a tagged protein, such as for the tagged E40 of sequence comprising or consisting of SEQ ID NO: 2. In preferred embodiments, said polynucleotide encoding for the protein of interest is a polynucleotide of sequence comprising or consisting of sequence SEQ ID NO: 5, 6, 15, 16, 17, or 18 and the expression cassette is of sequence SEQ ID NO: 20, 21, 22 or 23.

Therefore, in preferred embodiment, the present invention is directed to single-site integrating vector, more preferably a vector integrating at *attB* site for PhiBT1 phage of *Streptomyces,* bearing an expression cassette for the expression in *Streptomyces* host cells, preferably of *S. lividans* strains, of the *Actinoallomurus* endopeptidase, preferably of the *Actinoallomurus* endopeptidase 40 (E40), a biologically active fragment, a variant, or a derivative thereof, under the control of an engineered *kasO* promoter *(kasOp*)* of sequence SEQ ID NO: 10 and a ribosome-binding site (RBS) of sequence SEQ ID NO: 11 (SR40 RBS). Optionally the recombinant protein is the *Actinoallomurus* endopeptidase whose signal peptide is substituted with a Vsi signal peptide.

More preferably the single-site integrating vector bearing an expression cassette for the expression of the *Actinoallomurus* endopeptidase 40 (E40) is a pMU1 vector of sequence SEQ ID NO: 25, 26, 27 or 28.

The present invention is also directed to a host cell for heterologous expression of a recombinant protein comprising an expression vector according to the invention, preferably being a recombinant *Streptomyces* host cell, more preferably a *S. lividans* host cell. The host cell can be a wild-type *S. lividans* host cell or a mutant *S. lividans* host cell, such as a *S. lividans* host cell of strain *S. lividans* RedStrep 1.3 (Δact, Δred, Δcda), *S. lividans* RedStrep 1.6 (Δact, Δred, Δcda, Δmel), or *S. lividans* RedStrep 1.9 (Δact, Δred, Δcda, Δmel, ΔmatAB) (Novakova et al., 2018).

Preferably, said host cell is a cell of strain DSM 33930 (*S. lividans* 1.3 conjugated with pMU1s-e40His) DSM 33931 (*S. lividans* 1.9 conjugated with pMU1s-e40His), deposited on 24 June 2021 with the Leibniz Institute DSMZ-German Collection of Microorganisms and Cell Cultures GmbH, Inhoffenstraße 7B 38124 Braunschweig - GERMANY, under the provision of the Budapest Treaty.

The present invention is then directed also the use of the recombinant vectors or of the host cells of the invention for the production of a recombinant protein, which is secreted in the culture medium of the host cell.

Advantageously, the system of the invention provides production of the protein of interest, which is stable and in high amounts. Moreover, the protein is easily recoverable from the host cell culture medium wherein it is secreted.

In fact, the systems of the invention is also directed to a method for producing a recombinant protein in a *Streptomyces* host cell, comprising in series: culturing a recombinant *Streptomyces* host cell, preferably a *S. lividans* host cell, more preferably of the TK24 strain, in a culture medium under fermentation conditions, said recombinant host cell comprising a recombinant expression vector according to the invention; recovering the supernatant of the culture medium and purifying from said supernatant a preparation comprising the recombinant protein.

For "fermentation conditions" it is meant conditions of cultivation of the host cell strain (medium composition, stirring parameters, aeration and temperature) suitable for the strain to grow and to produce the compound of interest (the recombinant endopeptidase). Suitable fermentation conditions can be for instance those described in WO2021013553 or those described in the following examples.

It should be understood that all the possible combinations of the preferred aspects of the present invention s are also described, and therefore similarly preferred.

Examples of embodiments of the present invention are given below, provided for illustrative and non-limiting purposes.

### EXAMPLES

### Plasmids

Plasmids that have been prepared in the following examples 1-6 are summarized in Table 1. The promoter, RBS, signal peptide, gene and vector's backbone are reported for each plasmid.

**Table 1**

| **Name** | **Promoter** | **RBS** | **Signal Peptide** | **Gene** | **Vector** |
|---|---|---|---|---|---|
| **pIJ86e40** | ErmEp* | | e40 | e40pre-pro | pIJ86 |
| **pIJ86e40His** | ErmEp* | | e40 | e40pre-proHis | pIJ86 |
| **pMU1s-kasOpSR40gusA** | kasOp* | SR40 | gusA | gusA | pMU1s |
| **pMU1s-kasOpSR40vsi3mRFPA** | kasOp* | SR40 | vsi | mRFPA | pMU1s |
| **pLit-kasOpSR40vsi3mRFPA** | kasOp* | SR40 | vsi | mRFPA | LITMUS 28 |
| **pLit-e40** | kasOp* | SR40 | e40 | e40pre-pro | LITMUS 28 |
| **pLit-e40His** | kasOp* | SR40 | e40 | e40pre-proHis | LITMUS 28 |
| **pLit-vsie40pre** | kasOp* | SR40 | vsi | e40pre | LITMUS 28 |
| **pLit-vsie40Hispre** | kasOp* | SR40 | vsi | e40pre His | LITMUS 28 |
| **pLit-vsie40HQHispre** | kasOp* | SR40 | vsi | e40preH38Q | LITMUS 28 |
| **pLit-vsie40mat** | kasOp* | SR40 | vsi | e40mat | LITMUS 28 |
| **pLit-vsie40Hismat** | kasOp* | SR40 | vsi | e40mat His | LITMUS 28 |
| **pMU1s-e40** | kasOp* | SR40 | e40 | e40pre-pro | pMU1s |
| **pMU1s-e40His** | kasOp* | SR40 | e40 | e40pre-proHis | pMU1s |
| **pMU1s-vsie40pre** | kasOp* | SR40 | vsi | e40pre | pMU1s |
| **pMU1s-vsie40Hispre** | kasOp* | SR40 | vsi | e40pre | pMU1s |
| **pMU1s-vsie40HQHispre** | kasOp* | SR40 | vsi | e40preH38Q | pMU1s |
| **pMU1s-vsie40mat** | kasOp* | SR40 | vsi | e40mat | pMU1s |
| **pMU1s-vsie40Hismat** | kasOp* | SR40 | vsi | e40mat His | pMU1s |
| **pIJ86-kasOpe40** | kasOp* | SR40 | e40 | e40pre-pro | pIJ86 |
| **pIJ86-kasOpe40His** | kasOp* | SR40 | e40 | e40pre-pro His | pIJ86 |
| **pIJ86-svsie40pre** | kasOp* | SR40 | vsi | e40pre | pIJ86 |
| **pIJ86-vsie40Hispre** | kasOp* | SR40 | vsi | e40pre His | pIJ86 |
| **pIJ86-vsie40HQHispre** | kasOp* | SR40 | vsi | e40preH38Q | pIJ86 |
| **pIJ86 -vsie40mat** | kasOp* | SR40 | vsi | e40mat | pIJ86 |
| **pIJ86-vsie40Hismat** | kasOp* | SR40 | vsi | e40mat His | pIJ86 |
| **pMU1s-ermEe40** | ErmEp* | | e40 | e40pre-proHis | pMU1s |

### Host cells

*S. lividans* wild-type strain TK24 (Taxonomy ID: 457428) and *S. lividans* mutant strains RedStrep 1.3, 1.6, 1.9 (Novakova et al. 2018) have been employed in examples 7-14 to test production of recombinant protein with different plasmids.

### Example 1

**pIJ86/e40 and pIJ86/e40His** (Fig. 2) are pIJ86 high copy replicative plasmids of the prior art (see WO2021013553), including the gene encoding for e40 (pre-proe40) under the *ermEp** promoter (comprised of weaker *ermEp2* and stronger mutated *ermEp1**). These have been obtained from Fondazione Istituto Insubrico di Ricerche per la Vita (FIIRV).

### Example 2

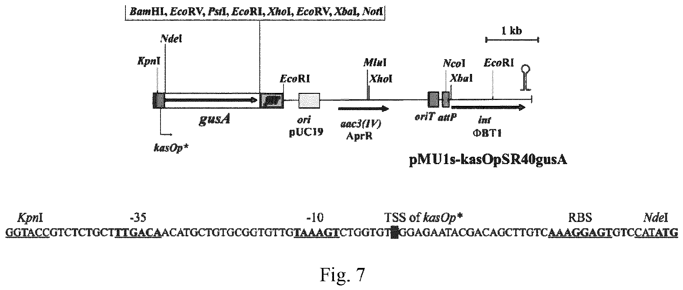

**pMU1s-kasOpSR40gusA**(Fig. 7) is a PhiBT1-based integrative vector pMU1s (Craney et al. 2007, Nucleic Acids Res 35, e46), bearing an expression cassette comprising *gusA* reporter gene (Myronovskyi et al. 2011, Appl Environ Microbiol 77, 5370-5383) under the control of the strong *kasOp** promoter and with optimized synthetic strong SR40 RBS (Bai et al. 2015, Proc Natl Acad Sci USA 112, 12181-12186). Activity of this combination was 1897 U/g of GUS, 47-fold higher than the same expression cassette under *ermEp** promoter.

### Example 3

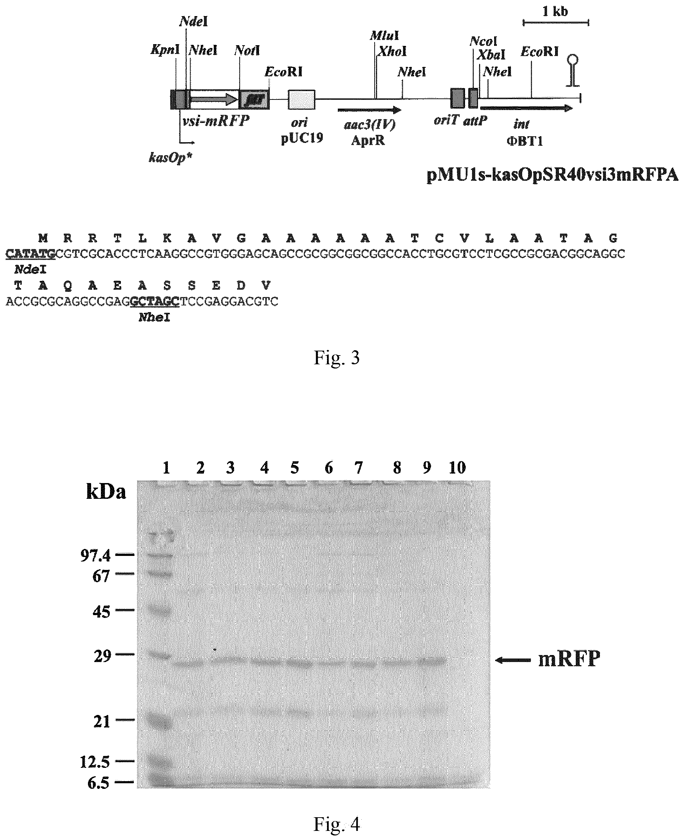

A polynucleotide encoding for a fusion polypeptide of vsi signal peptide sequence with red fluorescent protein (mRFP) reporter gene, with *Nhe*I site inserted between the signal peptide sequence and mRFP, was prepared. The whole vsi-mRFP cassette, as an 800-bp *Nde*I-*Not*I DNA fragment, was used to replace *gusA* in pMU1s-kasOpSR40gusAof Example 2, resulting in **pMU1s-kasOpSR40vsi3mRFPA** vector (Fig. 3). It similarly contained the strong *kasOp** promoter, optimized synthetic strong SR40 RBS and Vsi signal peptide. This construct had strong secretion of mRFP (27 886 FU). SDS-PAGE revealed correct band of secreted mRFP in rich TSB medium (Fig. 4).

### Example 4

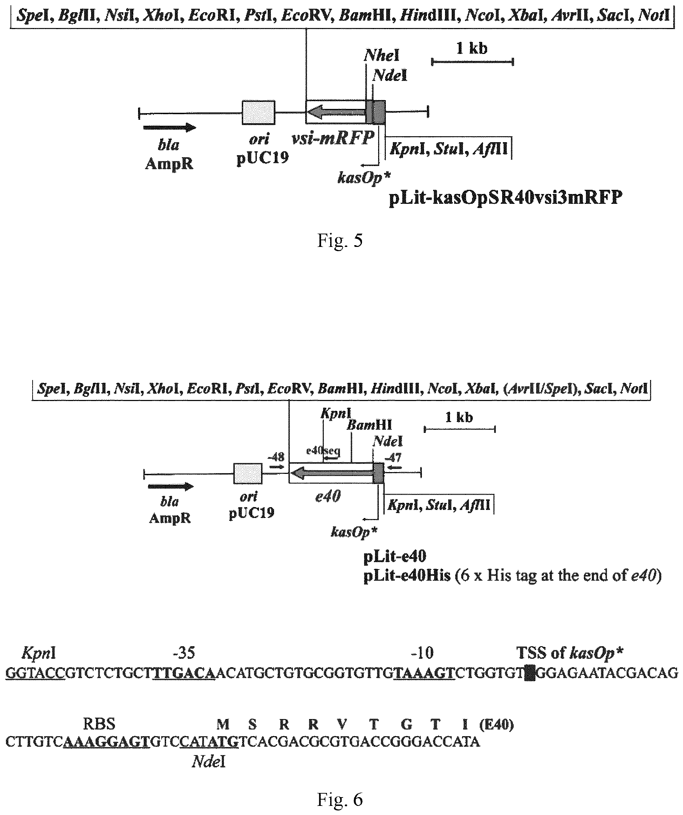

A 900-bp *Kpn*I*-Not*I expression cassette, containing the *kasOp** promoter, optimized SR40 RBS, with inserted single *Nde*I site in ATG codon of the *vsi-mRFP* reporter fusion gene, was cloned in the standard *E. coli* cloning vector pBluescript II SK (Stratagene) digested with the same enzymes, resulting in pBS-kasOpSR40vsi3mRFP. This fragment was subsequently cloned from this plasmid as a 900-bp *Kpn*I-*Sac*I fragment in the standard *E. coli* cloning vector LITMUS 28 (New England BioLabs) digested with the same enzymes, resulting in **pLit-kasOpSR40vsi3mRFP** (Fig. 5). The plasmid was verified by nucleotide sequencing and used as a vector for cloning of all e40 genes into vectors according to the invention. The entire e40 gene from its ATG initiation codon together with its signal peptide sequence (e40 pre-pro), with and without C-terminally located 6 x His tag, was PCR amplified from pIJ86/e40His plasmid, inserting an *Nde*I site in ATG initiation codon, and *Spe*I and *Hind*III sites after *e40* stop codon. The PCR amplified and purified 1200-bp DNA fragments were digested with *Nde*I and *Spe*I and cloned in pLit-kasOpSR40vsi3mRFP digested with *Avr*II and *Nde*I*,* resulting in **pLit-e40** or **pLit-e40His,** respectively (Fig. 6). Several positive clones were analyzed by restriction mapping and subsequently verified by nucleotide sequencing.

To prepare a *vsi-e40pre* gene fusion containing fusion of Vsi with a pro enzyme E40pre, we PCR amplified the e40pre gene (with and without C-terminally located 6 xHis tag), with pIJ86/e40His plasmid as a template and proof-reading Pfu DNA polymerase, using primers inserting an *Nhe*I site in Ala34 codon, and *Spe*I and *Hind*III sites after *e40* stop codon. The PCR amplified and purified 1100-bp DNA fragments were digested with *Nhe*I and *Hind*III and cloned in pLitkasOpSR40vsi3mRFP digested with *Nhe*I and *Hind*III, resulting in **pLit-vsie40pre** or **pLit-vsi40Hispre,** respectively (Fig. 8). Several positive clones were analyzed by restriction mapping and subsequently verified by nucleotide sequencing. Sequencing of the clones revealed one clone where synthesized primer produced mutation replacing the His38 CAC codon to the Gln38 CAA codon. The plasmid was also taken for further analysis and labelled as pLitvsi40HQHispre.

To prepare a vsi-e40mat gene fusion containing fusion of Vsi with a mature enzyme E40mat, we PCR amplified the e40mat gene (with and without C-terminally located 6 x His tag), with pIJ86/e40His plasmid as a template and proof-reading Pfu DNA polymerase, using primers that inserted an *Nhe*I site in Ala73 codon with changing further Ala74 codon to Ser, and *Spe*I and *Hind*III sites after *e40* stop codon. The PCR amplified and purified 1000-bp DNA fragments were digested with *NheI* and *Hind*III and cloned in pLit-kasOpSR40vsi3mRFP digested with *Nhe*I and *Hind*III, resulting in **pLit-vsie40mat** or **pLit-vsi40Hismat,** respectively (Fig. 8). Several positive clones were analyzed by restriction mapping and subsequently verified by nucleotide sequencing.

### Example 5

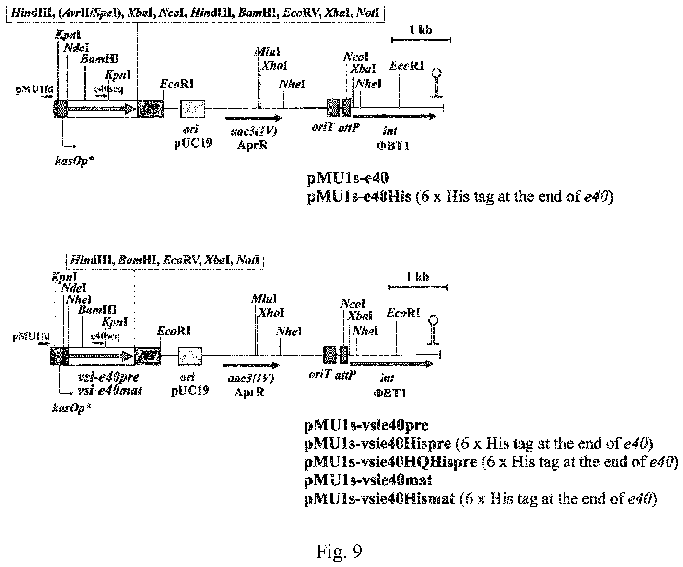

All the cassettes containing the *kasOp** promoter, RBS, and *e40* (with its original signal peptide or under Vsi signal peptide, with and without 6 x His tag at the end), have been cloned as 1400-bp *Kpn*I*-Eco*RV DNA fragments from pLit-e40, pLit-e40His, pLit-vsie40pre, pLit-e40Hispre, pLitvsie40HQHispre, pLit-vsie40mat, pLit-vsie40Hismat (Fig. 6, 8) into pMU1skasOpSR40gusA (Fig. 7), digested with *Kpn*I and *Eco*RV, resulting in **pMU1s-e40, pMU1s-e40His, pMU1s-vsie40pre, pMU1s-vsie40Hispre, pMU1s-vsie40HQHispre, pMU1s-vsie40mat, pMU1s-vsie40Hismat** vectors (Fig. 9). Several positive clones were analyzed by restriction mapping and subsequently verified by nucleotide sequencing. All the constructs can integrate into a single position of the chromosome of *S. lividans* TK24 using the phage PhiBT1-based integrative system after conjugation of the plasmids into *S. lividans* TK24 with apramycin resistance (AprR) selection. The final constructs are stable and no AprR selection is required.

### Example 6

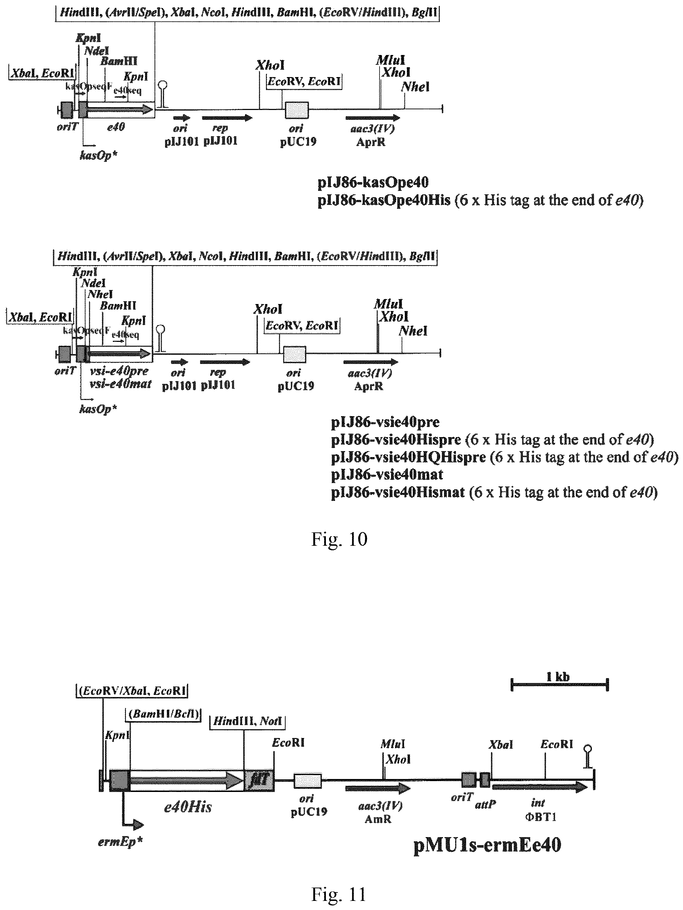

All the cassettes containing the *kasOp** promoter, RBS, and *e40* (with its original signal peptide or under Vsi signal peptide, with and without 6 x His tag at the end), have been cloned as 1400-bp *Kpn*I-*Eco*RV DNA fragments from pLit-e40, pLit-e40His, pLit-vsie40pre, pLit-e40Hispre, pLitvsie40HQHispre, pLit-vsie40mat, pLit-vsie40Hismat (Fig. 6, 8) to pIJ86, digested with *Kpn*I and *Hind*III, resulting in **pIJ86-kasOpe40, pIJ86-kasOpe40His, pIJ86-vsie40pre, pIJ86-vsie40Hispre, pIJ86-vsie40HQHispre, pIJ86-vsie40mat, pIJ86-vsie40Hismat** vectors (Fig. 10). Several positive clones were analyzed by restriction mapping and subsequently verified by nucleotide sequencing. All the constructs can replicate in *S. lividans* TK24 in high-copy number (about 100-300 copies) after conjugation of the plasmids into *S. lividans* TK24 with AprR selection. However, permanent AprR selection is required and some instability has been reported.

Last, a 1500-bp *Xba*I (filled with Klenow) - *Hind*III fragment from the high-copy number shuttle plasmid pIJ86/e40His was cloned into pMU1s-vsiRFPB vector cut with EcoRV and HindIII, resulting in **pMU1s-ermEe40** (Fig. 11). The plasmid was verified by sequencing.

### Example 7

E40 activity with the prior art strain *S. lividans*/pIJ86/e40His was determined. First, we prepared a spore stock after its sporulation on solid Bennet and Apramycin (Apr) medium. Mixed spores and spores from five independent sporulated colonies were tested for stability. Spore suspension from 1 x 1 cm sporulated region from confluent mixed spores (or whole 5 large single sporulated colonies) were inoculated in Erlenmeyer flasks (100 ml) containing 10 ml of Medium V (glucose 20 g/L, yeast extract Difco 5 g/L, soy peptone Sigma Aldrich 10 g/L, NaCl 1.5 g/L), with Apr added to final 50 ug/ml and incubated on a rotatory shaker at 200 rpm at 30°C for 1 day. 5 beads (3.5 mm) were added, then cultivation continued for another day. 2 ml of such seed culture was inoculated in Erlenmeyer flask (100 ml) containing 20 ml of the Medium P (Sucrose 340 g/L, Glucose10 g/L, Yeast extract 3 g/L, Soy peptone 5 g/L, Malt extract 3 g/L), with Apr added to final 50 µg/ml, and incubated in the same conditions. At 2d, 5d, 6d, 7d, 8d, 1 ml of culture into Eppendorf tube was taken, centrifuged (10 min at 13000 rpm), supernatant transferred into new tube and stored at - 20 °C. E40 activity was measured by SSA protocol (Standard Activity Assay, SSA, performed in 96 wells transparent microtiter plates and measured in Biotec Microplate reader at A405) with 40 x dilution of the samples. Comparable E40 activities of about 20 AU/ml was determined in all five independent colonies and mixed culture. Samples were also analyzed by SDS-PAGE, all showing the E40 38 kDa band (not shown).

### Example 8

E40 activity was tested with two *S. lividans* strains, wild-type TK24 and mutant *S. lividans* RedStrep 1.3, conjugated with the prior art plasmids pIJ86, pIJ86/e40, and pIJ86/e40His. The sporulated culture was inoculated in Erlenmeyer flasks (100 ml) containing 10 ml Niedercorn medium (3% sucrose, 2% corn steep liquor, 0.2% ammonium sulfate, 0.7% CaCO3, pH 7, in distilled water), with Apr to final 50 ug/ml and incubated on a rotatory shaker at 200 rpm at 30°C for 2 days.

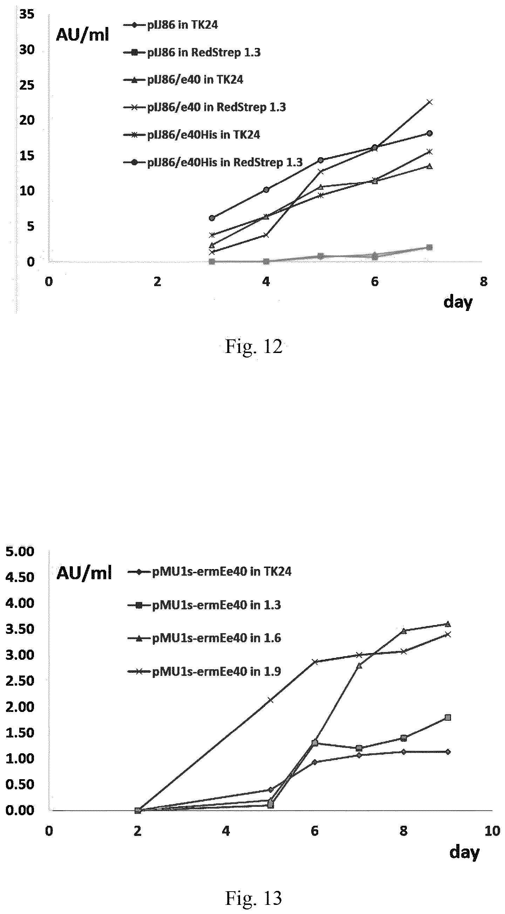

2 ml of such seed culture was inoculated in Erlenmeyer flask (100 ml) containing 20 ml of the Medium P. E40 activity was measured by SSA protocol as above with 40 x dilution of samples consisting of the supernatant of cultures taken at 3, 4, 5, 6, 7 days. In the case of vector pIJ86 alone, only low background protease activity was shown in both strains. With both constructs pIJ86/e40 and pIJ86/e40His E40 activity was of about 15 AU/ml in TK24 strain and of about 20 AU/ml in RedStrep 1.3 strain (Fig. 12)

### Example 9

E40 activity was measured in wild-type *S. lividans* TK24 strain and in three mutant strains, *S. lividans* RedStrep 1.3; 1.6; 1.9 using the control plasmid pMU1s-ermEe40 (Fig. 11). Cultures were prepared as in Example 8.

With single copy chromosomally-integrated construct pMU1s-ermEe40, E40 activity was much lower compared to the activity with high copy number pIJ86 vectors of Example 8: 1.13 AU/ml with pMU1s-ermEe40 in wild-type *S. lividans* TK24 and about 3-fold higher in RedStrep 1.6 and 1.9 strains (Fig. 13)

### Example 10

E40 activity was measured in wild-type *S. lividans* TK24 strain conjugated with four clones of the single-site integrating plasmids pMU1s-e40 and pMU1s-e40His, containing the *kasOp** promoter, strong SR40 RBS, and e40 with its original signal peptide (without and with C-terminal x His tag). Cultures were prepared as in Example 8.

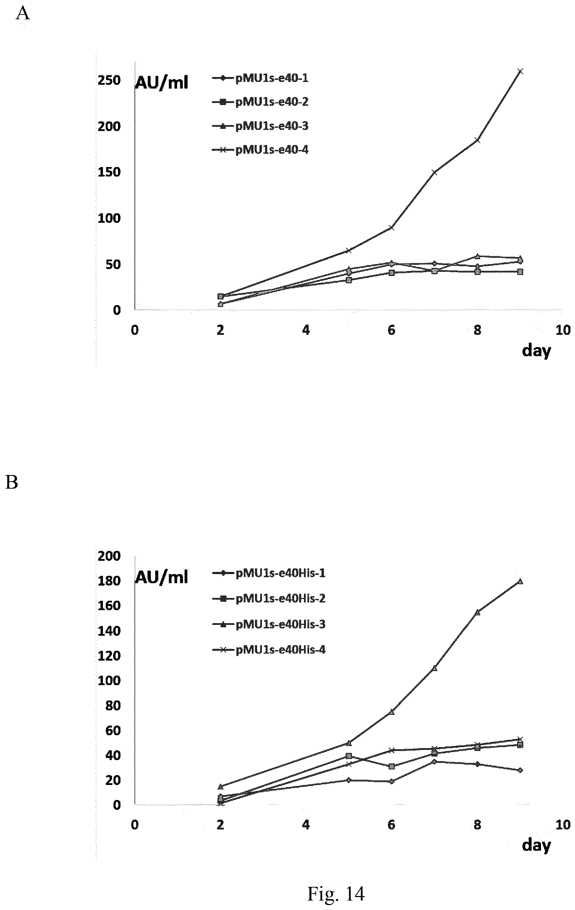

In three clones (No. 1, 2, 3) with the plasmid pMU1s-e40, there was comparable E40 activity of about 55 AU/ml, however, in the clone No.4, the activity was much higher (260 AU/ml) (Fig. 14 A). Similarly, in three clones (No. 1, 2, 4) with the plasmid pMU1s-e40His, there was comparable E40 activity of about 45 AU/ml, however, in the clone No.3, the activity was much higher (180 AU/ml) (Fig. 14B). Based on the comparison of the three similar clones with the single-copy integrative plasmid pMU1s-ermEe40 of Example 6, pMU1s-e40 and pMU1s-e40His show about 50-fold and 40-fold increase of the E40 activity, respectively.

### Example 11

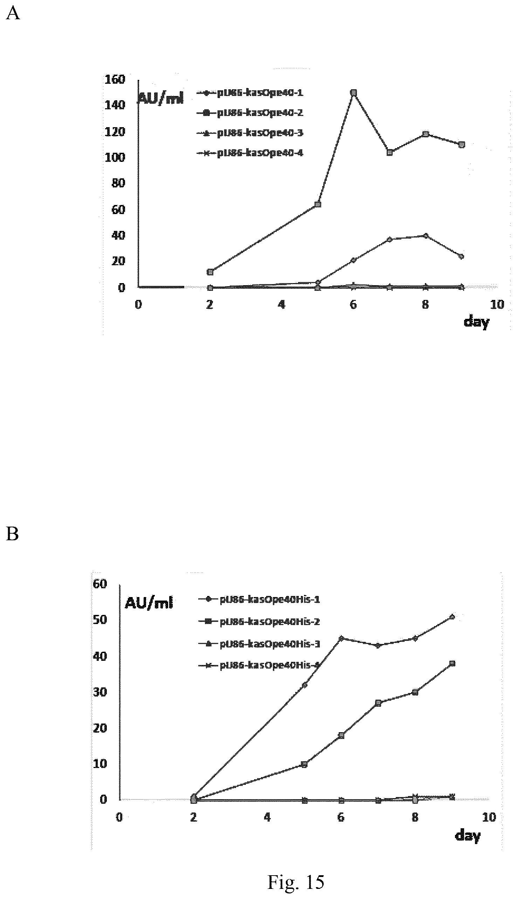

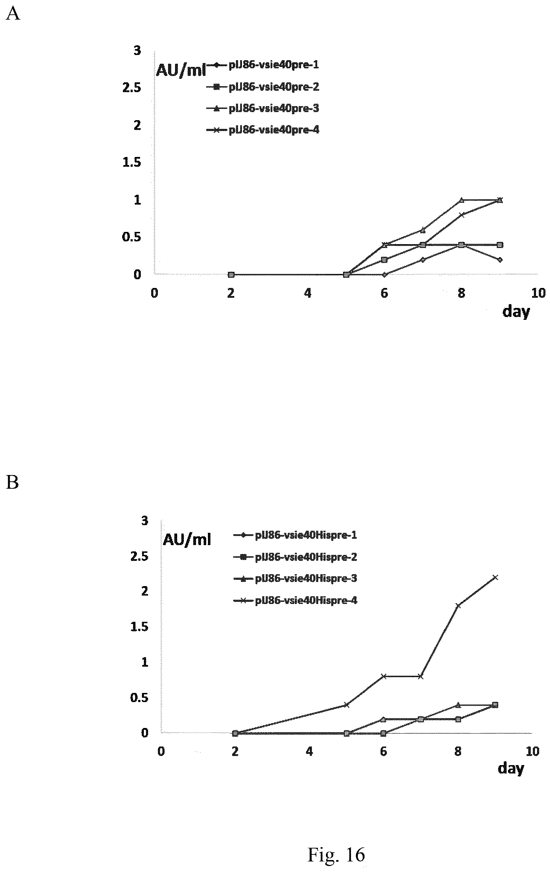

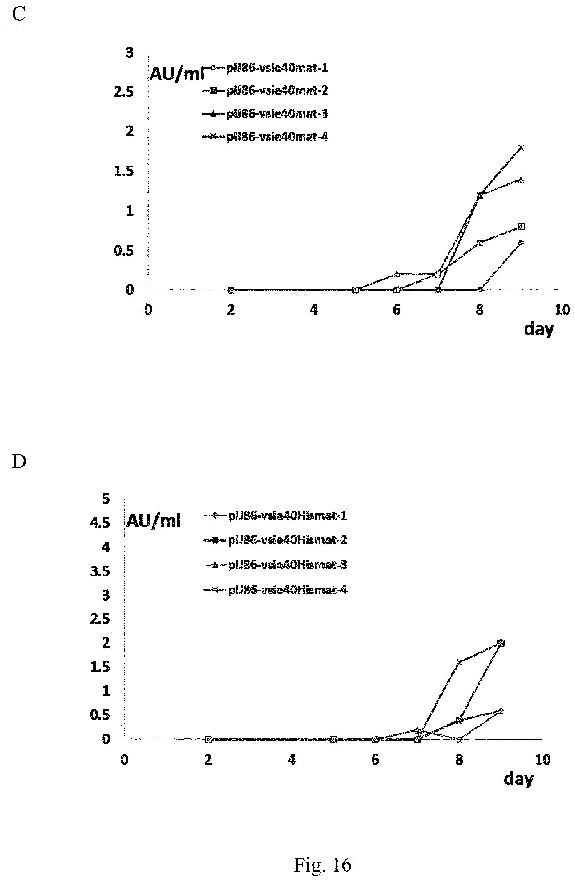

E40 activity was analyzed in four independent clones from the wild-type *S. lividans* TK24 strain conjugated with high copy vector pIJ86 containing the *kasOp** promoter, strong SR40 RBS, and e40 with its original signal peptide or with vsi signal peptide, without and with a C-terminal His tag (pIJ86-kasOpe40, pIJ86-kasOpe40His, pIJ86-vsie40pre, pIJ86-vsie40Hispre, pIJ86-vsie40HQHispre, pIJ86-vsie40mat, pIJ86-vsie40Hismat). The results indicated high instability of this high-copy number vector containing cloned cassettes *kasOp** promoter, strong SR40 RBS and e40, either with its original signal peptide (Fig. 15) or fused to Vsi signal peptide (Fig. 16), without and with C-terminal x His tag. In fact, in the case of four clones of pIJ86-kasOpe40, one out of four clones had quite high E40 activity (150 AU/ml), one had E40 activity of 40 AU/ml and two other clones had zero activity (Fig. 15 A). Similarly, in the case of four clones of pIJ86-kasOpe40His, one clone had quite high E40 activity (51 AU/ml), another clone had E40 activity of 38 AU/ml and two other clones had zero activity (Fig. 15 B).

Similarly, in the case of four clones of plasmids pIJ86-vsie40pre, pIJ86- vsie40Hispre, pIJ86-vsie40HQHispre, pIJ86-vsie40mat, pIJ86-vsie40Hismat in *S. lividans* TK24, the E40 activities were very low (between 0.5 and 2.5 AU/ml) in all the clones tested (Fig. 16 A-D). These results were surprising, since their single copy chromosomally integrated counterparts, pMU1s-e40 and pMU1s-e40His, had stable and high E40 activity (Fig. 14).

### Example 12

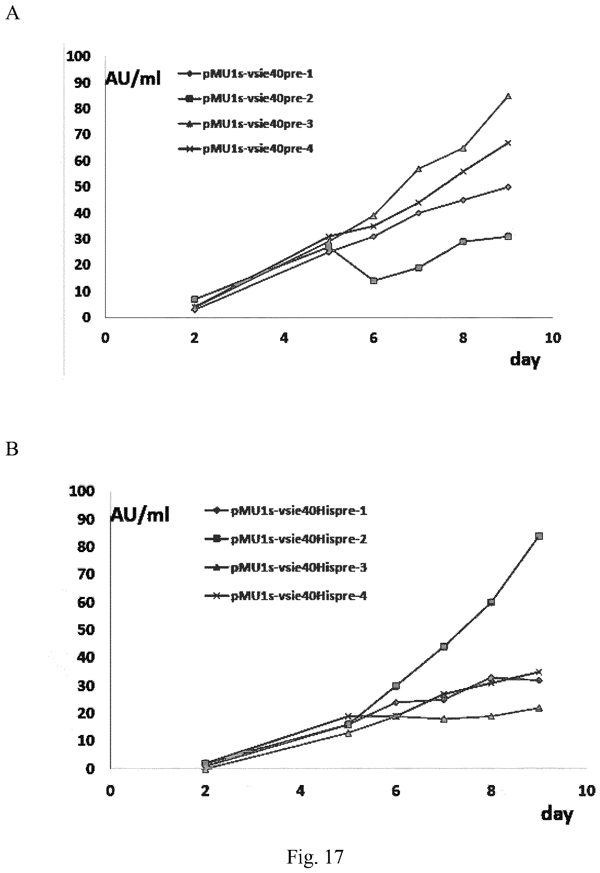

High E40 activity was found in all four clones with the plasmids pMU1s-vsie40pre and pMU1s-vsie40Hispre, containing the *kasOp** promoter, strong SR40 RBS, and pro-enzyme part of e40 fused to Vsi signal peptide (Fig. 17). In the case of the plasmid pMU1s-vsie40pre, there was E40 activity of about 50 AU/ml, and up to 85 AU/ml in one clone (Fig. 17A). Similarly, with the plasmid pMU1s-vsie40Hispre, there was comparable E40 activity of about 30 AU/ml in three clones, and 84 AU/ml in one clone (Fig. 17B). Based on the comparison of these E40 activities to the ones of E40 from cells conjugated with control single-copy integrative plasmid pMU1s-ermEe40 of Example 9, there is about 45-fold increase of the E40 activity for the construct pMU1s-vsie40pre (75-fold in the highest activity clone) and about 26- fold increase of the E40 activity for the construct pMU1s-vsie40Hispre (even 75-fold in the highest activity clone). Comparing these clones to the clones with high-copy number plasmid pIJ86/e40His in TK24, the two best clones show a 5.6-fold higher E40 activity. Moreover, these clones show high stability.

Plasmid pMU1s-vsie40HisHQpre, containing the kasOp* promoter, strong RBS, and pro-enzyme part of e40 with 38His/Gln mutation fused to Vsi signal peptide showed E40 activity partially decreased compared to its wild-type variant (about 27 AU/ml).

### Example 13

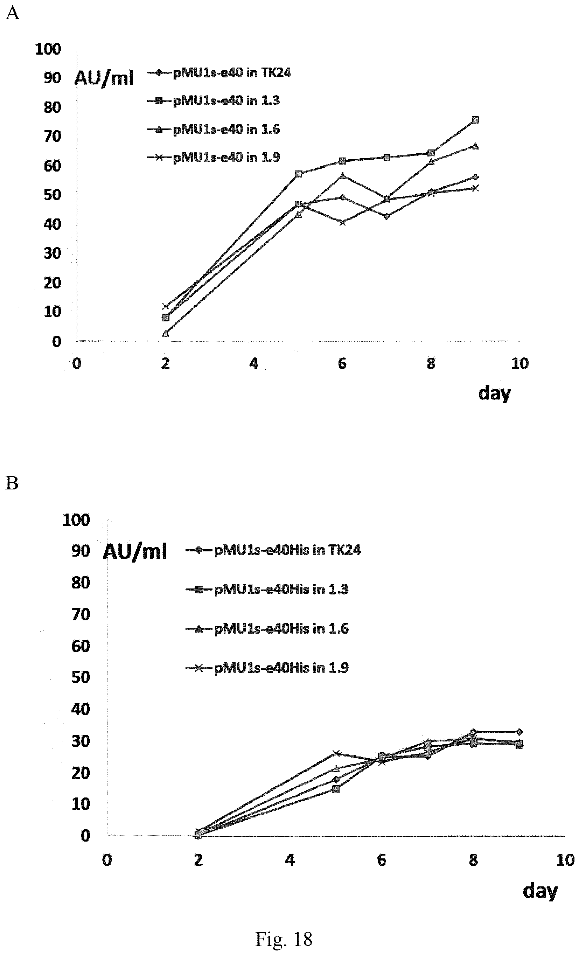

pMU1s-e40 and pMUIs-e40His were conjugated in the *S. lividans* mutant strains RedStrep1.3, 1.6 and 1.9. E40 activity was analyzed in four independent clones together with the four previously analyzed clones of example 10 in the wild-type *S. lividans* TK24 strain. In the case of pMU1s-e40, the E40 activities were clearly higher in RedStrep 1.3 (75 ± 13 AU/ml) than in TK24 (56 ± 7 AU/ml). The E40 activities were also higher in RedStrep 1.6 (67 ± 24 AU/ml), but the E40 activities in RedStrep 1.9 were similar to TK24 (52 ± 7 AU/ml) (Fig. 18A). In the case of pMUIs-e40His E40 activities were similar in all four strains; in WT TK24 (33 ± 7 AU/ml), in RedStrep 1.3 (29 ± 6 AU/ml), in RedStrep 1.6 (31 ± 7 AU/ml) and in RedStrep 1.9 (31 ± 7 AU/ml) (Fig. 18B)

### Example 14

pMU1s-vsie40pre and pMU1s-vsie40Hispre (containing the *kasOp** promoter, strong SR40 RBS, and pro-enzyme part of e40 fused to Vsi signal peptide), were conjugated into RedStrep1.3, 1.6 and 1.9 mutant strains and E40 activity was analyzed in four independent clones together with the four previously analyzed clones of example 12 in the wild-type *S. lividans* TK24 strain. Recombinant cells were grown as in example 8.

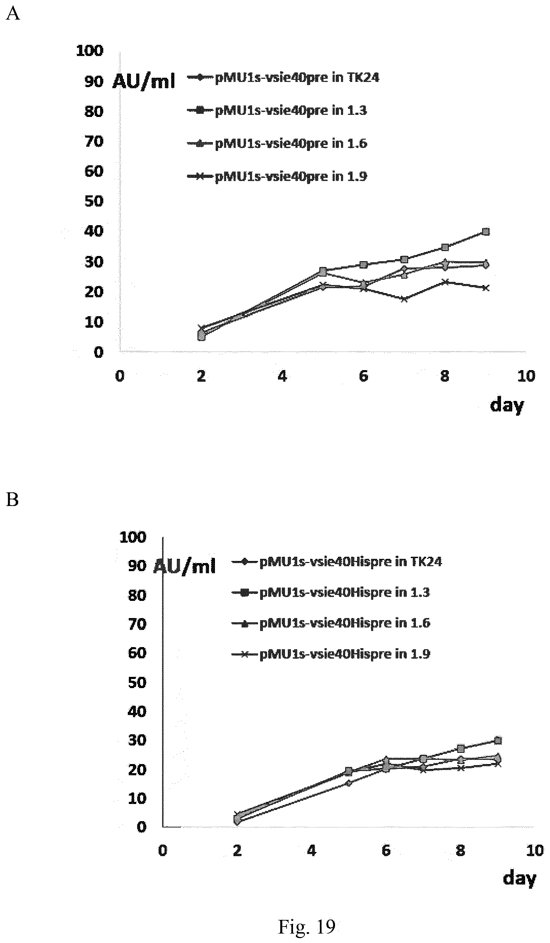

In the case of pMU1s-vsie40pre vector, E40 activities were higher in RedStrep 1.3 (40 ± 13.3 AU/ml) than in TK24 (28.75 ± 6.2 AU/ml). The E40 activities in RedStrep 1.6 (29.75 ± 3.8 AU/ml) were similar to WT TK24 strain. (Fig. 19A). In the case of pMU1s-vsie40Hispre vector, E40 activities were again higher in RedStrep 1.3 (30 ± 3.5 AU/ml), and similar in RedStrep 1.6 (24.75 ± 0.9 AU/ml), in RedStrep 1.6 (31 ± 7 AU/ml), but lower in RedStrep 1.9 (22 ± 2.9 AU/ml) (Fig. 19B).

The experimental data show that integrative vectors based on PhiBT1 phage integrase (pMU1s-e40, pMU1s-e40His, pMU1s-vsie40pre, pMU1s-vsie40Hispre, pMU1s-vsie40HQHispre, pMU1s-vsie40mat, pMU1s-vsie40Hismat) according to the invention provide stable and high production of E40. pMU1s-e40 and pMUIs-e40His vectors, containing the *kasOp** promoter, optimal strong SR40 RBS, original signal peptide sequence followed by original *e40* or *e40His* are the best vectors, producing on average 50 AU/ml or 35 AU/ml of enzyme, respectively. Moreover, the best host cell strain for E40 production is S. *lividans* RedStrep 1.3.

### SEQUENCE LISTING

SEQ ID NO: 1 
AAPSGLSPANLQSAYKLPSSTAGSGQTVAIVDAYDAPTAEADLNVYRSQFGLGACTTANGCFKKVDQNGG
TSYPRKDGGWAQEISLDLDMVSAVCPNCKIVLVEAKTNSFANLGTAENTAASLANVISNSYGGSDASDAS
YGSYYNHPGKAITVSSGDAGYGVEYPASSHYVTAVGGTSLRTASTSRGWSETAWSGAGSGCSAYNTALSG
QSGLTGCSRRAVADVSAVADPATGVAVYDSTAYQGQSGWMVFGGTSVAAPIIGGVYGLAANAASIDNNYP
YAHTSSLFDVTSGSNGTCTTTKWCTAGTGWDGPTGLGTPNGTGAF
SEQ ID NO: 2 
AAPSGLSPANLQSAYKLPSSTAGSGQTVAIVDAYDAPTAEADLNVYRSQFGLGACTTANGCFKKVDQNGG
TSYPRKDGGWAQEISLDLDMVSAVCPNCKIVLVEAKTNSFANLGTAENTAASLANVISNSYGGSDASDAS
YGSYYNHPGKAITVSSGDAGYGVEYPASSHYVTAVGGTSLRTASTSRGWSETAWSGAGSGCSAYNTALSG
QSGLTGCSRRAVADVSAVADPATGVAVYDSTAYQGQSGWMVFGGTSVAAPIIGGVYGLAANAASIDNNYP
YAHTSSLFDVTSGSNGTCTTTKWCTAGTGWDGPTGLGTPNGTGAFGHHHHHH
SEQ ID NO: 3 
MSRRVTGTILGGLILAMVPFLSTAANAAPQAAPASVSHPFHHSCATVKPGRASCNALVRSDIAQSAATLA
HQAAAPSGLSPANLQSAYKLPSSTAGSGQTVAIVDAYDAPTAEADLNVYRSQFGLGACTTANGCFKKVDQ
NGGTSYPRKDGGWAQEISLDLDMVSAVCPNCKIVLVEAKTNSFANLGTAENTAASLANVISNSYGGSDAS
DASYGSYYNHPGKAITVSSGDAGYGVEYPASSHYVTAVGGTSLRTASTSRGWSETAWSGAGSGCSAYNTA
LSGQSGLTGCSRRAVADVSAVADPATGVAVYDSTAYQGQSGWMVFGGTSVAAPIIGGVYGLAANAASIDN
NYPYAHTSSLFDVTSGSNGTCTTTKWCTAGTGWDGPTGLGTPNGTGAF
SEQ ID NO: 4 
MSRRVTGTILGGLILAMVPFLSTAANAAPQAAPASVSHPFHHSCATVKPGRASCNALVRSDIAQSAATLA
HQAAAPSGLSPANLQSAYKLPSSTAGSGQTVAIVDAYDAPTAEADLNVYRSQFGLGACTTANGCFKKVDQ
NGGTSYPRKDGGWAQEISLDLDMVSAVCPNCKIVLVEAKTNSFANLGTAENTAASLANVISNSYGGSDAS
DASYGSYYNHPGKAITVSSGDAGYGVEYPASSHYVTAVGGTSLRTASTSRGWSETAWSGAGSGCSAYNTA
LSGQSGLTGCSRRAVADVSAVADPATGVAVYDSTAYQGQSGWMVFGGTSVAAPIIGGVYGLAANAASIDN
NYPYAHTSSLFDVTSGSNGTCTTTKWCTAGTGWDGPTGLGTPNGTGAFGHHHHHH
SEQ ID NO: 5 
atgtcacgac gcgtgaccgg gaccatactg ggcgggttga tcctcgccat ggtccccttc
ctttccaccg cggccaacgc cgcaccccag gccgcgccgg cttccgtctc ccacccgttc
caccactcct gcgccacggt gaagccgggt cgggcgagct gcaatgccct cgtacgcagc
gacatcgccc agagcgcggc gaccctcgcg caccaagcgg ccgccccatc cgggctctcg
ccggccaacc tgcagagcgc ctacaagctg ccgtcctcca cggccggatc cggccagacc
gtcgcgatcg tcgacgccta tgacgccccg accgccgaag cggacttgaa cgtgtaccga
agccagttcg gactcggcgc gtgcacgacc gccaacggct gtttcaagaa ggtcgaccag
aacggcggca cgtcctatcc gaggaaggac ggcggctggg cgcaggagat ctccctggac
 
ctcgacatgg tctccgcggt ctgccccaac tgcaagatcg ttctcgtcga ggcgaagacc
aactcgttcg ccaacctggg taccgccgag aacaccgcgg cgagtctcgc gaacgtcatc
agcaacagct acggcggctc ggacgcctct gacgcgagct atggctcgta ctacaaccac
ccgggcaagg ccatcacggt cagctccggc gacgccggct acggcgtgga gtacccggcc
tcgtcccact acgtgaccgc cgtcggcggc acctcgctgc gcaccgcgag caccagccgc
ggctggagcg agaccgcgtg gagcggcgcg ggcagtggct gctcggccta caacaccgcg
ctgtccggcc agtccggcct caccggctgc tcccggcgcg ccgtcgccga cgtctccgcc
gtggccgacc cggccaccgg cgtcgccgtc tacgacagca cggcctacca gggccagagc
ggctggatgg tcttcggcgg caccagcgtc gccgcaccga tcatcggtgg cgtgtacggc
ctcgccgcca acgccgcgag catcgacaac aactacccct acgcccacac cagctcgctc
ttcgacgtca cgtcgggcag caacggcacc tgcaccacca ccaagtggtg caccgccggc
accggctggg acggccccac cggcctcgga acgccgaacg gcaccggagc cttctga
SEQ ID NO: 6 
atgtcacgac gcgtgaccgg gaccatactg ggcgggttga tcctcgccat ggtccccttc
ctttccaccg cggccaacgc cgcaccccag gccgcgccgg cttccgtctc ccacccgttc
caccactcct gcgccacggt gaagccgggt cgggcgagct gcaatgccct cgtacgcagc
gacatcgccc agagcgcggc gaccctcgcg caccaagcgg ccgccccatc cgggctctcg
ccggccaacc tgcagagcgc ctacaagctg ccgtcctcca cggccggatc cggccagacc
gtcgcgatcg tcgacgccta tgacgccccg accgccgaag cggacttgaa cgtgtaccga
agccagttcg gactcggcgc gtgcacgacc gccaacggct gtttcaagaa ggtcgaccag
aacggcggca cgtcctatcc gaggaaggac ggcggctggg cgcaggagat ctccctggac
ctcgacatgg tctccgcggt ctgccccaac tgcaagatcg ttctcgtcga ggcgaagacc
aactcgttcg ccaacctggg taccgccgag aacaccgcgg cgagtctcgc gaacgtcatc
agcaacagct acggcggctc ggacgcctct gacgcgagct atggctcgta ctacaaccac
ccgggcaagg ccatcacggt cagctccggc gacgccggct acggcgtgga gtacccggcc
tcgtcccact acgtgaccgc cgtcggcggc acctcgctgc gcaccgcgag caccagccgc
ggctggagcg agaccgcgtg gagcggcgcg ggcagtggct gctcggccta caacaccgcg
ctgtccggcc agtccggcct caccggctgc tcccggcgcg ccgtcgccga cgtctccgcc
gtggccgacc cggccaccgg cgtcgccgtc tacgacagca cggcctacca gggccagagc
ggctggatgg tcttcggcgg caccagcgtc gccgcaccga tcatcggtgg cgtgtacggc
ctcgccgcca acgccgcgag catcgacaac aactacccct acgcccacac cagctcgctc
ttcgacgtca cgtcgggcag caacggcacc tgcaccacca ccaagtggtg caccgccggc
accggctggg acggccccac cggcctcgga acgccgaacg gcaccggagc cttcggacac
caccaccacc accactga
SEQ ID NO: 7 
gccagcctcg cagagcagga ttcccgttga gcaccgccag gtgcgaataa gggacagtga
agaaggaaca cccgctcgcg ggtgggccta cttcacctat cctgcccggc tgacgccgtt
ggatacacca aggaaagtct acacgaaccc tttggcaaaa tcctgtatat cgtgcgaaaa
aggatggata taccgaaaaa atcgctataa tgaccccgaa gcagggttat gcagcggaaa
agatccgtcg acctgcaggt cgactctaga ggatctgggg aattcgagct cggtaccagc
ccgacccgag cacgcgccgg cacgcctggt cgatgtcgga ccggagttcg aggtacgcgg
cttgcaggtc caggaagggg acgtccatgc gagtgtccgt tcgagtggcg gcttgcgccc
gatgctagtc gcggttgatc ggcgatcgca ggtgcacgcg gtcgatcttg acggctggcg
agaggtgcgg ggaggatctg accgacgcgg tccacacgtg gcaccgcgat gctgttgtgg
gcacaatcgt gccggttggt aggatcctct agagtcgacc ggcatgcaag cttggctgca
gatctgatcc gcggggcgcc ggcacttcgt gctggcgccc cgcccccacc caccaggaga
ccgaccatga ccgacttcga cggacgcctg accgagggga ccgtgaacct ggtccaggac
cccaacggcg gtggctggtc cgcccactgc gctgagcccg gttgcgactg ggccgacttc
gccggaccgc tcggcttcca gggcctcgtg gccatcgctc gccgacacac gcactgaccg
cacgtcaaag ccccgccgga tcaccggcgg ggctctcttc ggccctccaa gtcacaccag
ccccaagggg cgtcgggagt ggcggaggga acctctggcc cgattggtgc caggattccc
accagaccaa agagcaacgg gccggacttc gcacctccga cccgtccgct cccagactcg
cgccccttag ccgggcgaga caggaacgtt gctcgtgccc agagtacgga gcgatgccga
ggcattgcca gatcggcccg ccgggccccg ctgccactgc gggaccgcaa ttgcccacac
accgggcaaa cggccgcgta tctactgctc agaccgctgc cggatggcag cgaagcgggc
gatcgcgcgt gtgacgcgag atgccgcccg aggcaaaagc gaacaccttg ggaaagaaac
 
aacagagttt cccgcacccc tccgacctgc ggtttctccg gacggggtgg atggggagag
cccgagaggc gacagcctct gggaagtagg aagcacgtcg cggaccgagg ctgcccgact
gcggaaagcc gcccggtaca gccgccgccg gacgctgtgg cggatcagcg ggacgccgc
gtgcaagggc tgcggccgcg ccctgatgga ccctgcctcc ggcgtgatcg tcgcccagac
ggcggccgga acgtccgtgg tcctgggcct gatgcggtgc gggcggatct ggctctgccc
ggtctgcgcc gccacgatcc ggcacaagcg ggccgaggag atcaccgccg ccgtggtcga
gtggatcaag cgcgggggga ccgcctacct ggtcaccttc acggcccgcc atgggcacac
ggaccggctc gcggacctca tggacgccct ccagggcacc cggaagacgc cggacagccc
ccggcggccg ggcgcctacc agcgactgat cacgggcggc acgtgggccg gacgccgggc
caaggacggg caccgggccg ccgaccgcga gggcatccga gaccggatcg ggtacgtcgg
catgatccgc gcgaccgaag tcaccgtggg gcagatcaac ggctggcacc cgcacatcca
cgcgatcgtc ctggtcggcg gccggaccga gggggagcgg tccgcgaagc agatcgtcgc
caccttcgag ccgaccggcg ccgcgctcga cgagtggcag gggcactggc ggtccgtgtg
gaccgccgcc ctgcgcaagg tcaaccccgc cttcacgccc gacgaccggc acggcgtcga
cttcaagcgg ctggagaccg agcgcgacgc caacgacctc gccgagtaca tcgccaagac
ccaggacggg aaggcgcccg ccctcgaact cgcccgcgcc gacctcaaga cggcgaccgg
cgggaacgtc gccccgttcg aactcctcgg acggatcggg gacctgaccg gcggcatgac
cgaggacgac gccgccgggg tcggctcgct ggagtggaac ctctcgcgct ggcacgagta
cgagcgggca acccggggac gccgggccat cgaatggacc cgctacctgc ggcagatgct
cgggctcgac ggcggcgaca ccgaggccga cgacctcgat ctgctcctgg cggccgacgc
cgacggcggg gagctgcggg ccggggtcgc cgtgaccgag gacggatggc acgcggtcac
ccgccgcgcc ctcgacctcg aggcgacccg ggccgccgaa ggcaaggacg gcaacgagnn
nncggcggcc gtgggcgaac gggtgcggga ggtcctggcg ctggccgacg cggccgacac
agtggtggtg ctcacggcgg gggaggtggc cgaggcgtac gccgacatgc tcgccgccct
cgcccagcgc cgcgaggaag caactgcacg ccgacggcga gagcaggacg acgaccagga
cgacgacgcc gacgaccgcc aggagcgggc cgcccggcac atcgcccggc tcgcaagtgg
gcccacttcg cactaactcg ctcccccccg ccgtacgtca tcccggtgac gtacggcggg
ggtcggtgac gtacgcggcg acggcggccg gggtcgaagc cgcggccgcg cgcgatatcg
aattcgtaat catggtcata gctgtttcct gtgtgaaatt gttatccgct cacaattcca
cacaacatac gagccggaag cataaagtgt aaagcctggg gtgcctaatg agtgagctaa
ctcacattaa ttgcgttgcg ctcactgccc gctttccagt cgggaaacct gtcgtgccag
ctgcattaat gaatcggcca acgcgcgggg agaggcggtt tgcgtattgg gcgctcttcc
gcttcctcgc tcactgactc gctgcgctcg gtcgttcggc tgcggcgagc ggtatcagct
cactcaaagg cggtaatacg gttatccaca gaatcagggg ataacgcagg aaagaacatg
tgagcaaaag gccagcaaaa ggccaggaac cgtaaaaagg ccgcgttgct ggcgtttttc
cataggctcc gcccccctga cgagcatcac aaaaatcgac gctcaagtca gaggtggcga
aacccgacag gactataaag ataccaggcg tttccccctg gaagctccct cgtgcgctct
cctgttccga ccctgccgct taccggatac ctgtccgcct ttctcccttc gggaagcgtg
gcgctttctc atagctcacg ctgtaggtat ctcagttcgg tgtaggtcgt tcgctccaag
ctgggctgtg tgcacgaacc ccccgttcag cccgaccgct gcgccttatc cggtaactat
cgtcttgagt ccaacccggt aagacacgac ttatcgccac tggcagcagc cactggtaac
aggattagca gagcgaggta tgtaggcggt gctacagagt tcttgaagtg gtggcctaac
tacggctaca ctagaagaac agtatttggt atctgcgctc tgctgaagcc agttaccttc
ggaaaaagag ttggtagctc ttgatccggc aaacaaacca ccgctggtag cggtggtttt
tttgtttgca agcagcagat tacgcgcaga aaaaaaggat ctcaagaaga tcctttgatc
ttttctacgg ggtctgacgc tcagtggaac gaaaactcac gttaagggat tttggtcatg
agattatcaa aaaggatctt cacctagatc cttttggttc atgtgcagct ccatcagcaa
aaggggatga taagtttatc accaccgact atttgcaaca gcgccggtga tcgtgctatg
atcgactgat gtcatcagcg gtggagtgca atgtcgtgca atacgaatgg cgaaaagccg
agctcatcgg tcagcttctc aaccttgggg ttacccccgg cggtgtgctg ctggtccaca
gctccttccg tagcgtccgg cccctcgaag atgggccact tggactgatc gaggccctgc
gtgctgcgct gggtccggga gggacgctcg tcatgccctc gtggtcaggt ctggacgacg
agccgttcga tcctgccacg tcgcccgtta caccggacct tggagttgtc tctgacacat
tctggcgcct gccaaatgta aagcgcagcg cccatccatt tgcctttgcg gcagcggggc
cacaggcaga gcagatcatc tctgatccat tgcccctgcc acctcactcg cctgcaagcc
cggtcgcccg tgtccatgaa ctcgatgggc aggtacttct cctcggcgtg ggacacgatg
ccaacacgac gctgcatctt gccgagttga tggcaaaggt tccctatggg gtgccgagac
actgcaccat tcttcaggat ggcaagttgg tacgcgtcga ttatctcgag aatgaccact
 
gctgtgagcg ctttgccttg gcggacaggt ggctcaagga gaagagcctt cagaaggaag
gtccagtcgg tcatgccttt gctcggttga tccgctcccg cgacattgtg gcgacagccc
tgggtcaact gggccgagat ccgttgatct tcctgcatcc gccagaggcg ggatgcgaag
aatgcgatgc cgctcgccag tcgattggct gagctcatga gcggagaacg agatgacgtt
ggaggggcaa ggtcgcgctg attgctgggg caacacgtgg agcggatcgg ggattgtctt
tcttcagctc gctgatgata tgctgacgct caatgccgtt tggcctccga ctaacgaaaa
tcccgcattt ggacggctga tccgattggc acggcggacg gcgaatggcg gagcagacgc
tcgtccgggg gcaatgagat atgaaaaagc ctgaactcac cgcgacgtat cgggccctgg
ccagctagct agagtcgacc tgcaggtccc cggggatcgg tcttgccttg ctcgtcggtg
atgtacttca ccagcttcgc gaagtcgctc ttcttgatgg agcgcatggg gacgtgcttg
gcaatcacgc gcaccccccg gccgttttag cggctaaaaa agtcatggct ctgccctcgg
gcggaccacg cccatcatga ccttgccaag ctcgtcctgc ttctcttcga tcttcgccag
cagggcgagg atcgtggcat caccgaaccg cgccgtgcgc gggtcgtcgg tgagccagag
tttcagcagg ccgcccaggc ggcccaggtc gccattgatg cgggccagct cgcggacgtg
ctcatagtcc acgacgcccg tgattttgta gccctggccg acggccagca ggtaggccga
caggctcatg ccggccgccg ccgccttttc ctcaatcgct cttcgttcgt ctggaaggca
gtacaccttg ataggtgggc tgcccttcct ggttggcttg gtttcatcag ccatccgctt
gccctcatct gttacgccgg cggtagccg
SEQ ID NO: 8 
gccagcctcg cagagcagga ttcccgttga gcaccgccag gtgcgaataa gggacagtga
agaaggaaca cccgctcgcg ggtgggccta cttcacctat cctgcccggc tgacgccgtt
ggatacacca aggaaagtct acacgaaccc tttggcaaaa tcctgtatat cgtgcgaaaa
aggatggata taccgaaaaa atcgctataa tgaccccgaa gcagggttat gcagcggaaa
agatccgtcg acctgcaggt cgactctaga ggatctgggg aattcgagct cggtaccagc
ccgacccgag cacgcgccgg cacgcctggt cgatgtcgga ccggagttcg aggtacgcgg
cttgcaggtc caggaagggg acgtccatgc gagtgtccgt tcgagtggcg gcttgcgccc
gatgctagtc gcggttgatc ggcgatcgca ggtgcacgcg gtcgatcttg acggctggcg
agaggtgcgg ggaggatctg accgacgcgg tccacacgtg gcaccgcgat gctgttgtgg
gcacaatcgt gccggttggt aggatcaatg tcacgacgcg tgaccgggac catactgggc
gggttgatcc tcgccatggt ccccttcctt tccaccgcgg ccaacgccgc accccaggcc
gcgccggctt ccgtctccca cccgttccac cactcctgcg ccacggtgaa gccgggtcgg
gcgagctgca atgccctcgt acgcagcgac atcgcccaga gcgcggcgac cctcgcgcac
caagcggccg ccccatccgg gctctcgccg gccaacctgc agagcgccta caagctgccg
tcctccacgg ccggatccgg ccagaccgtc gcgatcgtcg acgcctatga cgccccgacc
gccgaagcgg acttgaacgt gtaccgaagc cagttcggac tcggcgcgtg cacgaccgcc
aacggctgtt tcaagaaggt cgaccagaac ggcggcacgt cctatccgag gaaggacggc
ggctgggcgc aggagatctc cctggacctc gacatggtct ccgcggtctg ccccaactgc
aagatcgttc tcgtcgaggc gaagaccaac tcgttcgcca acctgggtac cgccgagaac
accgcggcga gtctcgcgaa cgtcatcagc aacagctacg gcggctcgga cgcctctgac
gcgagctatg gctcgtacta caaccacccg ggcaaggcca tcacggtcag ctccggcgac
gccggctacg gcgtggagta cccggcctcg tcccactacg tgaccgccgt cggcggcacc
tcgctgcgca ccgcgagcac cagccgcggc tggagcgaga ccgcgtggag cggcgcgggc
agtggctgct cggcctacaa caccgcgctg tccggccagt ccggcctcac cggctgctcc
cggcgcgccg tcgccgacgt ctccgccgtg gccgacccgg ccaccggcgt cgccgtctac
gacagcacgg cctaccaggg ccagagcggc tggatggtct tcggcggcac cagcgtcgcc
gcaccgatca tcggtggcgt gtacggcctc gccgccaacg ccgcgagcat cgacaacaac
tacccctacg cccacaccag ctcgctcttc gacgtcacgt cgggcagcaa cggcacctgc
accaccacca agtggtgcac cgccggcacc ggctgggacg gccccaccgg cctcggaacg
ccgaacggca ccggagcctt cggacaccac caccaccacc actgaagctt ggctgcagat
ctgatccgcg gggcgccggc acttcgtgct ggcgccccgc ccccacccac caggagaccg
accatgaccg acttcgacgg acgcctgacc gaggggaccg tgaacctggt ccaggacccc
aacggcggtg gctggtccgc ccactgcgct gagcccggtt gcgactgggc cgacttcgcc
ggaccgctcg gcttccaggg cctcgtggcc atcgctcgcc gacacacgca ctgaccgcac
gtcaaagccc cgccggatca ccggcggggc tctcttcggc cctccaagtc acaccagccc
caaggggcgt cgggagtggc ggagggaacc tctggcccga ttggtgccag gattcccacc
agaccaaaga gcaacggccc ggacttcgca cctccgaccc gtccgctccc agactcgcgc
cccttagccg ggcgagacag gaacgttgct cgtgcccaga gtacggagcg atgccgaggc
 
attgccagat cggcccgccg ggccccgctg ccactgcggg accgcaattg cccacacacc
gggcaaacgg ccgcgtatct actgctcaga ccgctgccgg atggcagcga agcgggcgat
cgcgcgtgtg acgcgagatg ccgcccgagg caaaagcgaa caccttggga aagaaacaac
agagtttccc gcacccctcc gacctgcggt ttctccggac ggggtggatg ggagagcccc
gagaggcgac agcctctggg aagtaggaag cacgtcgcgg accgaggctg cccgactgcg
gaaagccgcc cggtacagcc gccgccggac gctgtggcgg atcagcgggg acgccgcgtg
caagggctgc ggccgcgccc tgatggaccc tgcctccggc gtgatcgtcg cccagacggc
ggccggaacg tccgtggtcc tgggcctgat gcggtgcggg cggatctggc tctgcccggt
ctgcgccgcc acgatccggc acaagcgggc cgaggagatc accgccgccg tggtcgagtg
gatcaagcgc ggggggaccg cctacctggt caccttcacg gcccgccatg ggcacacgga
ccggctcgcg gacctcatgg acgccctcca gggcacccgg aagacgccgg acagcccccg
gcggccgggc gcctaccagc gactgatcac gggcggcacg tgggccggac gccgggccaa
ggacgggcac cgggccgccg accgcgaggg catccgagac cggatcgggt acgtcggcat
gatccgcgcg accgaagtca ccgtggggca gatcaacggc tggcacccgc acatccacgc
gatcgtcctg gtcggcggcc ggaccgaggg ggagcggtcc gcgaagcaga tcgtcgccac
cttcgagccg accggcgccg cgctcgacga gtggcagggg cactggcggt ccgtgtggac
cgccgccctg cgcaaggtca accccgcctt cacgcccgac gaccggcacg gcgtcgactt
caagcggctg gagaccgagc gcgacgccaa cgacctcgcc gagtacatcg ccaagaccca
ggacgggaag gcgcccgccc tcgaactcgc ccgcgccgac ctcaagacgg cgaccggcgg
gaacgtcgcc ccgttcgaac tcctcggacg gatcggggac ctgaccggcg gcatgaccga
ggacgacgcc gccggggtcg gctcgctgga gtggaacctc tcgcgctggc acgagtacga
gcgggcaacc cggggacgcc gggccatcga atggacccgc tacctgcggc agatgctcgg
gctcgacggc ggcgacaccg aggccgacga cctcgatctg ctcctggcgg ccgacgccga
cggcggggag ctgcggccgg gggtcgccgt gaccgaggac ggatggcacg cggtcacccg
ccgcgccctc gacctcgagg cgacccgggc cgccgaaggc aaggacggca acgagnnnnc
ggcggccgtg ggcgaacggg tgcgggaggt cctggcgctg gccgacgcgg ccgacacagt
ggtggtgctc acggcggggg aggtggccga ggcgtacgcc gacatgctcg ccgccctcgc
ccagcgccgc gaggaagcaa ctgcacgccg acggcgagag caggacgacg accaggacga
cgacgccgac gaccgccagg agcgggccgc ccggcacatc gcccggctcg caagtgggcc
cacttcgcac taactcgctc ccccccgccg tacgtcatcc cggtgacgta cggcgggggt
cggtgacgta cgcggcgacg gcggccgggg tcgaagccgc ggccgcgcgc gatatcgaat
tcgtaatcat ggtcatagct gtttcctgtg tgaaattgtt atccgctcac aattccacac
aacatacgag ccggaagcat aaagtgtaaa gcctggggtg cctaatgagt gagctaactc
acattaattg cgttgcgctc actgcccgct ttccagtcgg gaaacctgtc gtgccagctg
cattaatgaa tcggccaacg cgcggggaga ggcggtttgc gtattgggcg ctcttccgct
tcctcgctca ctgactcgct gcgctcggtc gttcggctgc ggcgagcggt atcagctcac
tcaaaggcgg taatacggtt atccacagaa tcaggggata acgcaggaaa gaacatgtga
gcaaaaggcc agcaaaaggc caggaaccgt aaaaaggccg cgttgctggc gtttttccat
aggctccgcc cccctgacga gcatcacaaa aatcgacgct caagtcagag gtggcgaaac
ccgacaggac tataaagata ccaggcgttt ccccctggaa gctccctcgt gcgctctcct
gttccgaccc tgccgcttac cggatacctg tccgcctttc tcccttcggg aagcgtggcg
ctttctcata gctcacgctg taggtatctc agttcggtgt aggtcgttcg ctccaagctg
ggctgtgtgc acgaaccccc cgttcagccc gaccgctgcg ccttatccgg taactatcgt
cttgagtcca acccggtaag acacgactta tcgccactgg cagcagccac tggtaacagg
attagcagag cgaggtatgt aggcggtgct acagagttct tgaagtggtg gcctaactac
ggctacacta gaagaacagt atttggtatc tgcgctctgc tgaagccagt taccttcgga
aaaagagttg gtagctcttg atccggcaaa caaaccaccg ctggtagcgg tggttttttt
gtttgcaagc agcagattac gcgcagaaaa aaaggatctc aagaagatcc tttgatcttt
tctacggggt ctgacgctca gtggaacgaa aactcacgtt aagggatttt ggtcatgaga
ttatcaaaaa ggatcttcac ctagatcctt ttggttcatg tgcagctcca tcagcaaaag
gggatgataa gtttatcacc accgactatt tgcaacagcg ccggtgatcg tgctatgatc
gactgatgtc atcagcggtg gagtgcaatg tcgtgcaata cgaatggcga aaagccgagc
tcatcggtca gcttctcaac cttggggtta cccccggcgg tgtgctgctg gtccacagct
ccttccgtag cgtccggccc ctcgaagatg ggccacttgg actgatcgag gccctgcgtg
ctgcgctggg tccgggaggg acgctcgtca tgccctcgtg gtcaggtctg gacgacgagc
cgttcgatcc tgccacgtcg cccgttacac cggaccttgg agttgtctct gacacattct
ggcgcctgcc aaatgtaaag cgcagcgccc atccatttgc ctttgcggca gcggggccac
aggcagagca gatcatctct gatccattgc ccctgccacc tcactcgcct gcaagcccgg
 
tcgcccgtgt ccatgaactc gatgggcagg tacttctcct cggcgtggga cacgatgcca
acacgacgct gcatcttgcc gagttgatgg caaaggttcc ctatggggtg ccgagacact
gcaccattct tcaggatggc aagttggtac gcgtcgatta tctcgagaat gaccactgct
gtgagcgctt tgccttggcg gacaggtggc tcaaggagaa gagccttcag aaggaaggtc
cagtcggtca tgcctttgct cggttgatcc gctcccgcga cattgtggcg acagccctgg
gtcaactggg ccgagatccg ttgatcttcc tgcatccgcc agaggcggga tgcgaagaat
gcgatgccgc tcgccagtcg attggctgag ctcatgagcg gagaacgaga tgacgttgga
ggggcaaggt cgcgctgatt gctggggcaa cacgtggagc ggatcgggga ttgtctttct
tcagctcgct gatgatatgc tgacgctcaa tgccgtttgg cctccgacta acgaaaatcc
cgcatttgga cggctgatcc gattggcacg gcggacggcg aatggcggag cagacgctcg
tccgggggca atgagatatg aaaaagcctg aactcaccgc gacgtatcgg gccctggcca
gctagctaga gtcgacctgc aggtccccgg ggatcggtct tgccttgctc gtcggtgatg
tacttcacca gcttcgcgaa gtcgctcttc ttgatggagc gcatggggac gtgcttggca
atcacgcgca ccccccggcc gttttagcgg ctaaaaaagt catggctctg ccctcgggcg
gaccacgccc atcatgacct tgccaagctc gtcctgcttc tcttcgatct tcgccagcag
ggcgaggatc gtggcatcac cgaaccgcgc cgtgcgcggg tcgtcggtga gccagagttt
cagcaggccg cccaggcggc ccaggtcgcc attgatgcgg gccagctcgc ggacgtgctc
atagtccacg acgcccgtga ttttgtagcc ctggccgacg gccagcaggt aggccgacag
gctcatgccg gccgccgccg ccttttcctc aatcgctctt cgttcgtctg gaaggcagta
caccttgata ggtgggctgc ccttcctggt tggcttggtt tcatcagcca tccgcttgcc
ctcatctgtt acgccggcgg tagccg
SEQ ID NO: 9 
APQAAPASVSHPFHHSCATVKPGRASCNALVRSDIAQSAATLAHQAAAPSGLSPANLQSAYKLPSSTAGS
GQTVAIVDAYDAPTAEADLNVYRSQFGLGACTTANGCFKKVDQNGGTSYPRKDGGWAQEISLDLDMVSAV
CPNCKIVLVEAKTNSFANLGTAENTAASLANVISNSYGGSDASDASYGSYYNHPGKAITVSSGDAGYGVE
YPASSHYVTAVGGTSLRTASTSRGWSETAWSGAGSGCSAYNTALSGQSGLTGCSRRAVADVSAVADPATG
VAVYDSTAYQGQSGWMVFGGTSVAAPIIGGVYGLAANAASIDNNYPYAHTSSLFDVTSGSNGTCTTTKWC
TAGTGWDGPTGLGTPNGTGAF
SEQ ID NO: 10
   gtctctgctt tgacaacatg ctgtgcggtg ttgtaaagtc tggtgta
SEQ ID NO: 11
   cttgtcaaag gagtgtccat
SEQ ID NO: 12 
atgcgtcgca ccctcaaggc cgtgggagca gccgcggcgg cggccacctg cgtcctcgcc
gcgacggcag gcaccgcgca ggccgag
SEQ ID NO: 13 
tcagacgttt cgggtgctgg gttgttgtct ctggacagtg atccatggga aactactcag
caccaccaat gttcccaaaa gaaag
SEQ ID NO: 14 
tggcgccgga cggggcttca gacgtttcgg gtgctgggtt gttgtctctg gacagtgatc
catgggaaac tactcagcac caccaatgtt cccaaaagaa agcgcaggtc agcgcccatg
agccaagatc taggcatgtc gcccttcatc gctcccgacg tccctgagca ccttctagac
actgttcgcg tcttcctgta cgcgcgtcag tctaagggcc ggtccgacgg ctcagacgtg
tcgaccgaag cacagctagc ggccggtcgt gcgttggtcg cgtctcgcaa cgcccagggg
ggtgcgcgct gggtcgtggc aggtgagttc gtggacgtcg ggcgctccgg ctgggacccg
aacgtgaccc gtgccgactt cgagcgcatg atgggcgaag tccgcgccgg cgaaggtgac
gttgtcgttg tgaatgagct ttcccggctc actcgcaagg gcgcccatga cgcgctcgaa
atcgacaacg aattgaagaa gcacggcgtg cgcttcatgt cggttcttga gccgttcctt
gacacgtcta cccctatcgg cgtcgccatt ttcgcgctga tcgctgccct tgcgaaacag
gacagtgacc tgaaggcgga gcgcctgaag ggtgcgaaag acgagattgc cgcgctgggt
ggcgttcact cgtcttccgc cccgttcgga atgcgcgccg tgcgcaagaa ggtcgataat
ctcgtgatct ccgttcttga gccggacgaa gacaacccgg atcacgtcga gctagttgag
 
cgcatggcga aaatgtcgtt cgaaggcgtg tccgacaacg ccattgcaac gaccttcgag
aaggaaaaga tcccgtcgcc cggaatggct gagagacgcg ccacggaaaa gcgtcttgcg
tccgtcaagg cacgtcgcct gaacggcgct gaaaagccga tcatgtggcg cgctcaaacg
gtccgatgga ttctcaacca tcccgcaatc ggcggtttcg cattcgagcg tgtgaagcac
ggtaaggcgc acatcaacgt catacggcgc gaccccggcg gcaagccgct aacgccccac
acgggcattc tcagcggctc gaagtggctt gagcttcaag agaagcgttc cgggaagaat
ctcagcgacc ggaagcctgg ggccgaagtc gaaccgacgc ttctgagcgg gtggcgtttc
ctggggtgcc gaatctgcgg cggctcaatg ggtcagtccc agggtggccg taagcgcaac
ggcgaccttg ccgaaggcaa ttacatgtgc gccaacccga aggggcacgg cggcttgtcg
gtcaagcgca gcgaactgga cgaattcgtt gcttcgaagg tgtgggcacg gctccgcaca
gccgacatgg aagatgaaca cgatcaggca tggattgccg ccgctgcgga gcgcttcgcc
cttcagcacg acctagcggg ggtggccgat gagcggcgcg aacaacaggc gcacctagac
aacgtgcggc gctccatcaa ggaccttcag gcggaccgta agcccggtct gtacgtcggg
cgtgaagagc tggaaacgtg gcgctcaacg gtgctgcaat accggtccta cgaagcggag
tgcacgaccc gactcgctga gcttgacgag aagatgaacg gcagcacccg cgttccgtct
gagtggttca gcggcgaaga cccgacggcc gaagggggca tctgggcaag ctgggacgtg
tacgagcgtc gggagttcct gagcttcttc cttgactccg tcatggtcga ccgggggcgc
caccctgaga cgaagaaata catccccctg aaggaccgtg tgacgctcaa gtgggcggag
ctgctgaagg aggaagacga agcgagcgaa gccactgagc gggagcttgc ggcgctgtag
cgcacagcgg gaggggtcga gccggcggac ggttcggccc cttttttggc cttgaaatcg
ttagttaggc taactagggt accgtctctg ctttgacaac atgctgtgcg gtgttgtaaa
gtctggtgta ggagaatacg acagcttgtc aaaggagtgt ccatatggga tccagatatc
ctgcaggaat tcctcgagcc gatatccctc tagaccgcgg ccgcccggct gcagcccgta
cctctagtag agtcgagggg gaacccggcc gcgtccggcg cccccgccgc cttcgacgag
atcccgcaaa agcggccttt gactccctgc aagcctcagc gaccgaatat atcggttatg
cgtgggcgat ggttgttgtc attgtcggcg caactatcgg tatcaagctg tttaagaaat
tcacctcgaa agcaagctga taaaccgata caattaaagg ctccttttgg agcctttttt
tttggagatt ttcaacgtga aaaaattatt attcgcaatt cctttagttg ttcctttcta
ttctcactcc gctgaaactg ttgaaagttg tttagcaaaa cctcatacag aaaattcatt
tactaacgtc tggaaagacg acaaaacttt agatcctcta gctagagtcg accggcatgc
aatcgaattc gtaatcatgt catagctgtt tcctgtgtga aattgttatc cgctcacaat
tccacacaac atacgagccg gaagcataaa gtgtaaagcc tggggtgcct aatgagtgag
ctaactcaca ttaattgcgt tgcgctcact gcccgctttc cagtcgggaa acctgtcgtg
ccagctgcat taatgaatcg gccaacgcgc ggggagaggc ggtttgcgta ttgggcgctc
ttccgcttcc tcgctcactg actcgctgcg ctcggtcgtt cggctgcggc gagcggtatc
agctcactca aaggcggtaa tacggttatc cacagaatca ggggataacg caggaaagaa
catgtgagca aaaggccagc aaaaggccag gaaccgtaaa aaggccgcgt tgctggcgtt
tttccatagg ctccgccccc ctgacgagca tcacaaaaat cgacgctcaa gtcagaggtg
gcgaaacccg acaggactat aaagatacca ggcgtttccc cctggaagct ccctcgtgcg
ctctcctgtt ccgaccctgc cgcttaccgg atacctgtcc gcctttctcc cttcgggaag
cgtggcgctt tctcatagct cacgctgtag gtatctcagt tcggtgtagg tcgttcgctc
caagctgggc tgtgtgcacg aaccccccgt tcagcccgac cgctgcgcct tatccggtaa
ctatcgtctt gagtccaacc cggtaagaca cgacttatcg ccactggcag cagccactgg
taacaggatt agcagagcga ggtatgtagg cggtgctaca gagttcttga agtggtggcc
taactacggc tacactagaa gaacagtatt tggtatctgc gctctgctga agccagttac
cttcggaaaa agagttggta gctcttgatc cggcaaacaa accaccgctg gtagcggtgg
tttttttgtt tgcaagcagc agattacgcg cagaaaaaaa ggatctcaag aagatccttt
gatcttttct acggggtctg acgctcagtg gaacgaaaac tcacgttaag ggattttggt
catgagatta tcaaaaagga tcttcaccta gatccttttg gttcatgtgc agctccatca
gcaaaagggg atgataagtt tatcaccacc gactatttgc aacagtgccg ttgatcgtgc
tatgatcgac tgatgtcatc agcggtggag tgcaatgtcg tgcaatacga atggcgaaaa
gccgagctca tcggtcagct tctcaacctt ggggttaccc ccggcggtgt gctgctggtc
cacagctcct tccgtagcgt ccggcccctc gaagatgggc cacttggact gatcgaggcc
ctgcgtgctg cgctgggtcc gggagggacg ctcgtcatgc cctcgtggtc aggtctggac
gacgagccgt tcgatcctgc cacgtcgccc gttacaccgg accttggagt tgtctctgac
acattctggc gcctgccaaa tgtaaagcgc agcgcccatc catttgcctt tgcggcagcg
gggccacagg cagagcagat catctctgat ccattgcccc tgccacctca ctcgcctgca
agcccggtcg cccgtgtcca tgaactcgat gggcaggtac ttctcctcgg cgtgggacac
 
gatgccaaca cgacgctgca tcttgccgag ttgatggcaa aggttcccta tggggtgccg
agacactgca ccattcttca ggatggcaag ttggtacgcg tcgattatct cgagaatgac
cactgctgtg agcgctttgc cttggcggac aggtggctca aggagaagag ccttcagaag
gaaggtccag tcggtcatgc ctttgctcgg ttgatccgct cccgcgacat tgtggcgaca
gccctgggtc aactgggccg agatccgttg atcttcctgc atccgccaga ggcgggatgc
gaagaatgcg atgccgctcg ccagtcgatt ggctgagctc atgagcggag aacgagatga
cgttggaggg gcaaggtcgc gctgattgct ggggcaacac gtggagcgga tcggggattg
tctttcttca gctcgctgat gatatgctga cgctcaatgc cgtttggcct ccgactaacg
aaaatcccgc atttggacgg ctgatccgat tggcacggcg gacggcgaat ggcggagcag
acgctcgtcc gggggcaatg agatatgaaa aagcctgaac tcaccgcgac gtatcggccc
ctggccagct agctagagtc gacctgcagg tccccgggga tcggtcttgc cttgctcgtc
ggtgatgtac ttcaccagct ccgcgaagtc gctcttcttg atggagcgca tggggacgtg
cttggcaatc acgcgcaccc cccggccgtt ttagcggcta aaaaagtcat ggctctgccc
tcgggcggac cacgcccatc atgaccttgc caagctcgtc ctgcttctct tcgatcttcg
ccagcagggc gaggatcgtg gcatcaccga accgcgccgt gcgcgggtcg tcggtgagcc
agagtttcag caggccgccc aggcggccca ggtcgccatt gatgcgggcc agctcgcgga
cgtgctcata gtccacgacg cccgtgattt tgtagccctg gccgacggcc agcaggtagg
ccgacaggct catgccggcc gccgccgcct tttcctcaat cgctcttcgt tcgtctggaa
ggcagtacac cttgataggt gggctgccct tcctggttgg cttggtttca tcagccatcc
gcttgccctc atctgttacg ccggcggtag ccggccagcc tcgcagagca ggattcccgt
tgagcaccgc caggtgcgaa taagggacag tgaagaagga acacccgctc gcgggtgggc
ctacttcacc tatcctgccc ggctgacgcc gttggataca ccaaggaaag tctacacgaa
ccctttggca aaatcctgta tatcgtgcga aaaaggatgg atataccgaa aaaatcgcta
taatgacccc gaagcagggt tatgcagcgg aaaagatccg tcgacctgca ggcatgc
SEQ ID NO: 15 
atgcgtcgca ccctcaaggc cgtgggagca gccgcggcgg cggccacctg cgtcctcgcc
gcgacggcag gcaccgcgca ggccgaggct agcgtctccc acccgttcca ccactcctgc
gccacggtga agccgggtcg ggcgagctgc aatgccctcg tacgcagcga catcgcccag
agcgcggcga ccctcgcgca ccaagcggcc gccccatccg ggctctcgcc ggccaacctg
cagagcgcct acaagctgcc gtcctccacg gccggatccg gccagaccgt cgcgatcgtc
gacgcctatg acgccccgac cgccgaagcg gacttgaacg tgtaccgaag ccagttcgga
ctcggcgcgt gcacgaccgc caacggctgt ttcaagaagg tcgaccagaa cggcggcacg
tcctatccga ggaaggacgg cggctgggcg caggagatct ccctggacct cgacatggtc
tccgcggtct gccccaactg caagatcgtt ctcgtcgagg cgaagaccaa ctcgttcgcc
aacctgggta ccgccgagaa caccgcggcg agtctcgcga acgtcatcag caacagctac
ggcggctcgg acgcctctga cgcgagctat ggctcgtact acaaccaccc gggcaaggcc
atcacggtca gctccggcga cgccggctac ggcgtggagt acccggcctc gtcccactac
gtgaccgccg tcggcggcac ctcgctgcgc accgcgagca ccagccgcgg ctggagcgag
accgcgtgga gcggcgcggg cagtggctgc tcggcctaca acaccgcgct gtccggccag
tccggcctca ccggctgctc ccggcgcgcc gtcgccgacg tctccgccgt ggccgacccg
gccaccggcg tcgccgtcta cgacagcacg gcctaccagg gccagagcgg ctggatggtc
ttcggcggca ccagcgtcgc cgcaccgatc atcggtggcg tgtacggcct cgccgccaac
gccgcgagca tcgacaacaa ctacccctac gcccacacca gctcgctctt cgacgtcacg
tcgggcagca acggcacctg caccaccacc aagtggtgca ccgccggcac cggctgggac
ggccccaccg gcctcggaac gccgaacggc accggagcct tctga
SEQ ID NO: 16 
atgcgtcgca ccctcaaggc cgtgggagca gccgcggcgg cggccacctg cgtcctcgcc
gcgacggcag gcaccgcgca ggccgaggct agcgtctccc acccgttcca ccactcctgc
gccacggtga agccgggtcg ggcgagctgc aatgccctcg tacgcagcga catcgcccag
agcgcggcga ccctcgcgca ccaagcggcc gccccatccg ggctctcgcc ggccaacctg
cagagcgcct acaagctgcc gtcctccacg gccggatccg gccagaccgt cgcgatcgtc
gacgcctatg acgccccgac cgccgaagcg gacttgaacg tgtaccgaag ccagttcgga
ctcggcgcgt gcacgaccgc caacggctgt ttcaagaagg tcgaccagaa cggcggcacg
tcctatccga ggaaggacgg cggctgggcg caggagatct ccctggacct cgacatggtc
tccgcggtct gccccaactg caagatcgtt ctcgtcgagg cgaagaccaa ctcgttcgcc
aacctgggta ccgccgagaa caccgcggcg agtctcgcga acgtcatcag caacagctac
ggcggctcgg acgcctctga cgcgagctat ggctcgtact acaaccaccc gggcaaggcc
 
atcacggtca gctccggcga cgccggctac ggcgtggagt acccggcctc gtcccactac
gtgaccgccg tcggcggcac ctcgctgcgc accgcgagca ccagccgcgg ctggagcgag
accgcgtgga gcggcgcggg cagtggctgc tcggcctaca acaccgcgct gtccggccag
tccggcctca ccggctgctc ccggcgcgcc gtcgccgacg tctccgccgt ggccgacccg
gccaccggcg tcgccgtcta cgacagcacg gcctaccagg gccagagcgg ctggatggtc
ttcggcggca ccagcgtcgc cgcaccgatc atcggtggcg tgtacggcct cgccgccaac
gccgcgagca tcgacaacaa ctacccctac gcccacacca gctcgctctt cgacgtcacg
tcgggcagca acggcacctg caccaccacc aagtggtgca ccgccggcac cggctgggac
ggccccaccg gcctcggaac gccgaacggc accggagcct tcggacacca ccaccaccac
cactga
SEQ ID NO: 17 
gccgccccat ccgggctctc gccggccaac ctgcagagcg cctacaagct gccgtcctcc
acggccggat ccggccagac cgtcgcgatc gtcgacgcct atgacgcccc gaccgccgaa
gcggacttga acgtgtaccg aagccagttc ggactcggcg cgtgcacgac cgccaacggc
tgtttcaaga aggtcgacca gaacggcggc acgtcctatc cgaggaagga cggcggctgg
gcgcaggaga tctccctgga cctcgacatg gtctccgcgg tctgccccaa ctgcaagatc
gttctcgtcg aggcgaagac caactcgttc gccaacctgg gtaccgccga gaacaccgcg
gcgagtctcg cgaacgtcat cagcaacagc tacggcggct cggacgcctc tgacgcgagc
tatggctcgt actacaacca cccgggcaag gccatcacgg tcagctccgg cgacgccggc
tacggcgtgg agtacccggc ctcgtcccac tacgtgaccg ccgtcggcgg cacctcgctg
cgcaccgcga gcaccagccg cggctggagc gagaccgcgt ggagcggcgc gggcagtggc
tgctcggcct acaacaccgc gctgtccggc cagtccggcc tcaccggctg ctcccggcgc
gccgtcgccg acgtctccgc cgtggccgac ccggccaccg gcgtcgccgt ctacgacagc
acggcctacc agggccagag cggctggatg gtcttcggcg gcaccagcgt cgccgcaccg
atcatcggtg gcgtgtacgg cctcgccgcc aacgccgcga gcatcgacaa caactacccc
tacgcccaca ccagctcgct cttcgacgtc acgtcgggca gcaacggcac ctgcaccacc
accaagtggt gcaccgccgg caccggctgg gacggcccca ccggcctcgg aacgccgaac
ggcaccggag ccttctga
SEQ ID NO: 18 
gccgccccat ccgggctctc gccggccaac ctgcagagcg cctacaagct gccgtcctcc
acggccggat ccggccagac cgtcgcgatc gtcgacgcct atgacgcccc gaccgccgaa
gcggacttga acgtgtaccg aagccagttc ggactcggcg cgtgcacgac cgccaacggc
tgtttcaaga aggtcgacca gaacggcggc acgtcctatc cgaggaagga cggcggctgg
gcgcaggaga tctccctgga cctcgacatg gtctccgcgg tctgccccaa ctgcaagatc
gttctcgtcg aggcgaagac caactcgttc gccaacctgg gtaccgccga gaacaccgcg
gcgagtctcg cgaacgtcat cagcaacagc tacggcggct cggacgcctc tgacgcgagc
tatggctcgt actacaacca cccgggcaag gccatcacgg tcagctccgg cgacgccggc
tacggcgtgg agtacccggc ctcgtcccac tacgtgaccg ccgtcggcgg cacctcgctg
cgcaccgcga gcaccagccg cggctggagc gagaccgcgt ggagcggcgc gggcagtggc
tgctcggcct acaacaccgc gctgtccggc cagtccggcc tcaccggctg ctcccggcgc
gccgtcgccg acgtctccgc cgtggccgac ccggccaccg gcgtcgccgt ctacgacagc
acggcctacc agggccagag cggctggatg gtcttcggcg gcaccagcgt cgccgcaccg
atcatcggtg gcgtgtacgg cctcgccgcc aacgccgcga gcatcgacaa caactacccc
tacgcccaca ccagctcgct cttcgacgtc acgtcgggca gcaacggcac ctgcaccacc
accaagtggt gcaccgccgg caccggctgg gacggcccca ccggcctcgg aacgccgaac
ggcaccggag ccttcggaca ccaccaccac caccactga
SEQ ID NO: 19 
atgctgcggc ccgtcgaaac cccgacccgc gagatcaaga agctggacgg cctgtgggcg
ttcagcctcg accgggagaa ctgcggtatc gaccagcggt ggtgggagag cgccctccag
gagtcgcgcg ccatcgccgt ccccggcagc ttcaacgacc agttcgcgga cgcggacatc
cgcaactacg cggggaacgt ctggtatcag cgggaggtgt tcatccccaa gggctgggcc
ggtcagcgca tcgtgctgcg cttcgacgcc gtgacccact acggcaaggt ctgggtcaac
aaccaggaag tgatggagca ccagggcggc tacacgccct tcgaggcgga cgtgaccccg
tacgtcatcg ccggcaagtc ggtccgcatc acggtctgcg tcaacaacga gctcaactgg
cagaccatcc cgcccggcat ggtgatcacg gacgagaacg gcaagaagaa gcagagctac
 
ttccacgact tcttcaacta cgccggcatc caccgctcgg tcatgctgta cacgacgccg
aacacctggg tcgacgacat cacggtggtc acccacgtgg cccaggactg caaccacgcg
tccgtggact ggcaggtcgt cgccaacggc gacgtgagcg tggagctccg cgacgccgag
cagcaggtcg tggcgaccgg gcagggcacc tcggggaccc tccaggtcgt gaacccccac
ctctggcagc cgggtgaggg ctacctgtac gagctgtgcg tgacggcgaa gagccagacc
gagtgcgaca tctaccccct gcgcgtcggc atccggtccg tggcggtcaa gggcgagcag
ttcctgatca accacaagcc gttctacttc acgggcttcg gtcggcacga ggacgccgac
ctccgcggca agggcttcga caacgtcctg atggtccacg accacgccct gatggactgg
atcggcgcca actcgtaccg gacctcgcac tacccgtacg cggaagagat gctggactgg
gcggacgagc acgggatcgt cgtcatcgac gaaaccgccg cggtgggctt caacctgtcc
ctcggcatcg gcttcgaggc cggcaacaag ccgaaagagc tctactccga agaggccgtc
aacggcgaaa cccagcaggc ccacctccag gcgatcaaag agctcatcgc ccgcgacaag
aaccacccgt ccgtcgtgat gtggtcgatc gccaacgagc cggacacccg gccgcagggt
gcccgggagt acttcgcccc gctggccgag gccacccgga agctcgaccc cacccggccc
atcacctgcg tgaacgtgat gttctgcgac gcccacacgg acaccatctc cgacctcttc
gacgtcctgt gcctgaaccg ctactacggc tggtacgtgc agtccgggga cctggaaacg
gccgagaagg tcctggagaa agagctgctg gcgtggcagg agaagctgca ccagccgatc
atcatcaccg agtacggcgt ggacacgctg gccggtctgc actccatgta caccgacatg
tggtccgaag agtaccagtg cgcgtggctg gacatgtacc accgcgtgtt cgaccgcgtc
agcgcggtcg tcggcgagca ggtgtggaac ttcgccgact tcgccacctc ccagggcatc
ctccgcgtcg ggggcaacaa gaagggcatc ttcacccggg accgcaagcc caagtcggcg
gcgttcctgc tccagaagcg gtggaccggg atgaacttcg gggagaagcc gcagcagggc
gggaagcagt g
SEQ ID NO: 20 
gtctctgctt tgacaacatg ctgtgcggtg ttgtaaagtc tggtgtagga gaatacgaca
gcttgtcaaa ggagtgtcca tatgtcacga cgcgtgaccg ggaccatact gggcgggttg
atcctcgcca tggtcccctt cctttccacc gcggccaacg ccgcacccca ggccgcgccg
gcttccgtct cccacccgtt ccaccactcc tgcgccacgg tgaagccggg tcgggcgagc
tgcaatgccc tcgtacgcag cgacatcgcc cagagcgcgg cgaccctcgc gcaccaagcg
gccgccccat ccgggctctc gccggccaac ctgcagagcg cctacaagct gccgtcctcc
acggccggat ccggccagac cgtcgcgatc gtcgacgcct atgacgcccc gaccgccgaa
gcggacttga acgtgtaccg aagccagttc ggactcggcg cgtgcacgac cgccaacggc
tgtttcaaga aggtcgacca gaacggcggc acgtcctatc cgaggaagga cggcggctgg
gcgcaggaga tctccctgga cctcgacatg gtctccgcgg tctgccccaa ctgcaagatc
gttctcgtcg aggcgaagac caactcgttc gccaacctgg gtaccgccga gaacaccgcg
gcgagtctcg cgaacgtcat cagcaacagc tacggcggct cggacgcctc tgacgcgagc
tatggctcgt actacaacca cccgggcaag gccatcacgg tcagctccgg cgacgccggc
tacggcgtgg agtacccggc ctcgtcccac tacgtgaccg ccgtcggcgg cacctcgctg
cgcaccgcga gcaccagccg cggctggagc gagaccgcgt ggagcggcgc gggcagtggc
tgctcggcct acaacaccgc gctgtccggc cagtccggcc tcaccggctg ctcccggcgc
gccgtcgccg acgtctccgc cgtggccgac ccggccaccg gcgtcgccgt ctacgacagc
acggcctacc agggccagag cggctggatg gtcttcggcg gcaccagcgt cgccgcaccg
atcatcggtg gcgtgtacgg cctcgccgcc aacgccgcga gcatcgacaa caactacccc
tacgcccaca ccagctcgct cttcgacgtc acgtcggcca gcaacggcac ctgcaccacc
accaagtggt gcaccgccgg caccggctgg gacggcccca ccggcctcgg aacgccgaac
ggcaccggag ccttctga
SEQ ID NO: 21 
gtctctgctt tgacaacatg ctgtgcggtg ttgtaaagtc tggtgtagga gaatacgaca
gcttgtcaaa ggagtgtcca tatgtcacga cgcgtgaccg ggaccatact gggcgggttg
atcctcgcca tggtcccctt cctttccacc gcggccaacg ccgcacccca ggccgcgccg
gcttccgtct cccacccgtt ccaccactcc tgcgccacgg tgaagccggg tcgggcgagc
tgcaatgccc tcgtacgcag cgacatcgcc cagagcgcgg cgaccctcgc gcaccaagcg
gccgccccat ccgggctctc gccggccaac ctgcagagcg cctacaagct gccgtcctcc
acggccggat ccggccagac cgtcgcgatc gtcgacgcct atgacgcccc gaccgccgaa
gcggacttga acgtgtaccg aagccagttc ggactcggcg cgtgcacgac cgccaacggc
tgtttcaaga aggtcgacca gaacggcggc acgtcctatc cgaggaagga cggcggctgg
 
gcgcaggaga tctccctgga cctcgacatg gtctccgcgg tctgccccaa ctgcaagatc
gttctcgtcg aggcgaagac caactcgttc gccaacctgg gtaccgccga gaacaccgcg
gcgagtctcg cgaacgtcat cagcaacagc tacggcggct cggacgcctc tgacgcgagc
tatggctcgt actacaacca cccgggcaag gccatcacgg tcagctccgg cgacgccggc
tacggcgtgg agtacccggc ctcgtcccac tacgtgaccg ccgtcggcgg cacctcgctg
cgcaccgcga gcaccagccg cggctggagc gagaccgcgt ggagcggcgc gggcagtggc
tgctcggcct acaacaccgc gctgtccggc cagtccggcc tcaccggctg ctcccggcgc
gccgtcgccg acgtctccgc cgtggccgac ccggccaccg gcgtcgccgt ctacgacagc
acggcctacc agggccagag cggctggatg gtcttcggcg gcaccagcgt cgccgcaccg
atcatcggtg gcgtgtacgg cctcgccgcc aacgccgcga gcatcgacaa caactacccc
tacgcccaca ccagctcgct cttcgacgtc acgtcgggca gcaacggcac ctgcaccacc
accaagtggt gcaccgccgg caccggctgg gacggcccca ccggcctcgg aacgccgaac
ggcaccggag ccttcggaca ccaccaccac caccactga
SEQ ID NO: 22 
gtctctgctt tgacaacatg ctgtgcggtg ttgtaaagtc tggtgtagga gaatacgaca
gcttgtcaaa ggagtgtcca tatgcgtcgc accctcaagg ccgtgggagc agccgcggcg
gcggccacct gcgtcctcgc cgcgacggca ggcaccgcgc aggccgaggc tagcgtctcc
cacccgttcc accactcctg cgccacggtg aagccgggtc gggcgagctg caatgccctc
gtacgcagcg acatcgccca gagcgcggcg accctcgcgc accaagcggc cgccccatcc
gggctctcgc cggccaacct gcagagcgcc tacaagctgc cgtcctccac ggccggatcc
ggccagaccg tcgcgatcgt cgacgcctat gacgccccga ccgccgaagc ggacttgaac
gtgtaccgaa gccagttcgg actcggcgcg tgcacgaccg ccaacggctg tttcaagaag
gtcgaccaga acggcggcac gtcctatccg aggaaggacg gcggctgggc gcaggagatc
tccctggacc tcgacatggt ctccgcggtc tgccccaact gcaagatcgt tctcgtcgag
gcgaagacca actcgttcgc caacctgggt accgccgaga acaccgcggc gagtctcgcg
aacgtcatca gcaacagcta cggcggctcg gacgcctctg acgcgagcta tggctcgtac
tacaaccacc cgggcaaggc catcacggtc agctccggcg acgccggcta cggcgtggag
tacccggcct cgtcccacta cgtgaccgcc gtcggcggca cctcgctgcg caccgcgagc
accagccgcg gctggagcga gaccgcgtgg agcggcgcgg gcagtggctg ctcggcctac
aacaccgcgc tgtccggcca gtccggcctc accggctgct cccggcgcgc cgtcgccgac
gtctccgccg tggccgaccc ggccaccggc gtcgccgtct acgacagcac ggcctaccag
ggccagagcg gctggatggt cttcggcggc accagcgtcg ccgcaccgat catcggtggc
gtgtacggcc tcgccgccaa cgccgcgagc atcgacaaca actaccccta cgcccacacc
agctcgctct tcgacgtcac gtcgggcagc aacggcacct gcaccaccac caagtggtgc
accgccggca ccggctggga cggccccacc ggcctcggaa cgccgaacgg caccggagcc
ttctga
SEQ ID NO: 23 
gtctctgctt tgacaacatg ctgtgcggtg ttgtaaagtc tggtgtagga gaatacgaca
gcttgtcaaa ggagtgtcca tatgcgtcgc accctcaagg ccgtgggagc agccgcggcg
gcggccacct gcgtcctcgc cgcgacggca ggcaccgcgc aggccgaggc tagcgtctcc
cacccgttcc accactcctg cgccacggtg aagccgggtc gggcgagctg caatgccctc
gtacgcagcg acatcgccca gagcgcggcg accctcgcgc accaagcggc cgccccatcc
gggctctcgc cggccaacct gcagagcgcc tacaagctgc cgtcctccac ggccggatcc
ggccagaccg tcgcgatcgt cgacgcctat gacgccccga ccgccgaagc ggacttgaac
gtgtaccgaa gccagttcgg actcggcgcg tgcacgaccg ccaacggctg tttcaagaag
gtcgaccaga acggcggcac gtcctatccg aggaaggacg gcggctgggc gcaggagatc
tccctggacc tcgacatggt ctccgcggtc tgccccaact gcaagatcgt tctcgtcgag
gcgaagacca actcgttcgc caacctgggt accgccgaga acaccgcggc gagtctcgcg
aacgtcatca gcaacagcta cggcggctcg gacgcctctg acgcgagcta tggctcgtac
tacaaccacc cgggcaaggc catcacggtc agctccggcg acgccggcta cggcgtggag
tacccggcct cgtcccacta cgtgaccgcc gtcggcggca cctcgctgcg caccgcgagc
accagccgcg gctggagcga gaccgcgtgg agcggcgcgg gcagtggctg ctcggcctac
aacaccgcgc tgtccggcca gtccggcctc accggctgct cccggcgcgc cgtcgccgac
gtctccgccg tggccgaccc ggccaccggc gtcgccgtct acgacagcac ggcctaccag
ggccagagcg gctggatggt cttcggcggc accagcgtcg ccgcaccgat catcggtggc
gtgtacggcc tcgccgccaa cgccgcgagc atcgacaaca actaccccta cgcccacacc
 
agctcgctct tcgacgtcac gtcgggcagc aacggcacct gcaccaccac caagtggtgc
accgccggca ccggctggga cggccccacc ggcctcggaa cgccgaacgg caccggagcc
ttcggacacc accaccacca ccactga
SEQ ID NO: 24 
gtctctgctt tgacaacatg ctgtgcggtg ttgtaaagtc tggtgtagga gaatacgaca
gcttgtcaaa ggagtgtcca tatgctgcgg cccgtcgaaa ccccgacccg cgagatcaag
aagctggacg gcctgtgggc gttcagcctc gaccgggaga actgcggtat cgaccagcgg
tggtgggaga gcgccctcca ggagtcgcgc gccatcgccg tccccggcag cttcaacgac
cagttcgcgg acgcggacat ccgcaactac gcggggaacg tctggtatca gcgggaggtg
ttcatcccca agggctgggc cggtcagcgc atcgtgctgc gcttcgacgc cgtgacccac
tacggcaagg tctgggtcaa caaccaggaa gtgatggagc accagggcgg ctacacgccc
ttcgaggcgg acgtgacccc gtacgtcatc gccggcaagt cggtccgcat cacggtctgc
gtcaacaacg agctcaactg gcagaccatc ccgcccggca tggtgatcac ggacgagaac
ggcaagaaga agcagagcta cttccacgac ttcttcaact acgccggcat ccaccgctcg
gtcatgctgt acacgacgcc gaacacctgg gtcgacgaca tcacggtggt cacccacgtg
gcccaggact gcaaccacgc gtccgtggac tggcaggtcg tcgccaacgg cgacgtgagc
gtggagctcc gcgacgccga gcagcaggtc gtggcgaccg ggcagggcac ctcggggacc
ctccaggtcg tgaaccccca cctctggcag ccgggtgagg gctacctgta cgagctgtgc
gtgacggcga agagccagac cgagtgcgac atctaccccc tgcgcgtcgg catccggtcc
gtggcggtca agggcgagca gttcctgatc aaccacaagc cgttctactt cacgggcttc
ggtcggcacg aggacgccga cctccgcggc aagggcttcg acaacgtcct gatggtccac
gaccacgccc tgatggactg gatcggcgcc aactcgtacc ggacctcgca ctacccgtac
gcggaagaga tgctggactg ggcggacgag cacgggatcg tcgtcatcga cgaaaccgcc
gcggtgggct tcaacctgtc cctcggcatc ggcttcgagg ccggcaacaa gccgaaagag
ctctactccg aagaggccgt caacggcgaa acccagcagg cccacctcca ggcgatcaaa
gagctcatcg cccgcgacaa gaaccacccg tccgtcgtga tgtggtcgat cgccaacgag
ccggacaccc ggccgcaggg tgcccgggag tacttcgccc cgctggccga ggccacccgg
aagctcgacc ccacccggcc catcacctgc gtgaacgtga tgttctgcga cgcccacacg
gacaccatct ccgacctctt cgacgtcctg tgcctgaacc gctactacgg ctggtacgtg
cagtccgggg acctggaaac ggccgagaag gtcctggaga aagagctgct ggcgtggcag
gagaagctgc accagccgat catcatcacc gagtacggcg tggacacgct ggccggtctg
cactccatgt acaccgacat gtggtccgaa gagtaccagt gcgcgtggct ggacatgtac
caccgcgtgt tcgaccgcgt cagcgcggtc gtcggcgagc aggtgtggaa cttcgccgac
ttcgccacct cccagggcat cctccgcgtc gggggcaaca agaagggcat cttcacccgg
gaccgcaagc ccaagtcggc ggcgttcctg ctccagaagc ggtggaccgg gatgaacttc
ggggagaagc cgcagcaggg cgggaagcag tga
SEQ ID NO: 25 
tggcgccgga cggggcttca gacgtttcgg gtgctgggtt gttgtctctg gacagtgatc
catgggaaac tactcagcac caccaatgtt cccaaaagaa agcgcaggtc agcgcccatg
agccaagatc taggcatgtc gcccttcatc gctcccgacg tccctgagca ccttctagac
actgttcgcg tcttcctgta cgcgcgtcag tctaagggcc ggtccgacgg ctcagacgtg
tcgaccgaag cacagctagc ggccggtcgt gcgttggtcg cgtctcgcaa cgcccagggg
ggtgcgcgct gggtcgtggc aggtgagttc gtggacgtcg ggcgctccgg ctgggacccg
aacgtgaccc gtgccgactt cgagcgcatg atgggcgaag tccgcgccgg cgaaggtgac
gttgtcgttg tgaatgagct ttcccggctc actcgcaagg gcgcccatga cgcgctcgaa
atcgacaacg aattgaagaa gcacggcgtg cgcttcatgt cggttcttga gccgttcctt
gacacgtcta cccctatcgg cgtcgccatt ttcgcgctga tcgctgccct tgcgaaacag
gacagtgacc tgaaggcgga gcgcctgaag ggtgcgaaag acgagattgc cgcgctgggt
ggcgttcact cgtcttccgc cccgttcgga atgcgcgccg tgcgcaagaa ggtcgataat
ctcgtgatct ccgttcttga gccggacgaa gacaacccgg atcacgtcga gctagttgag
cgcatggcga aaatgtcgtt cgaaggcgtg tccgacaacg ccattgcaac gaccttcgag
aaggaaaaga tcccgtcgcc cggaatggct gagagacgcg ccacggaaaa gcgtcttgcg
tccgtcaagg cacgtcgcct gaacggcgct gaaaagccga tcatgtggcg cgctcaaacg
gtccgatgga ttctcaacca tcccgcaatc ggcggtttcg cattcgagcg tgtgaagcac
ggtaaggcgc acatcaacgt catacggcgc gaccccggcg gcaagccgct aacgccccac
acgggcattc tcagcggctc gaagtggctt gagcttcaag agaagcgttc cgggaagaat
 
ctcagcgacc ggaagcctgg ggccgaagtc gaaccgacgc ttctgagcgg gtggcgtttc
ctggggtgcc gaatctgcgg cggctcaatg ggtcagtccc agggtggccg taagcgcaac
ggcgaccttg ccgaaggcaa ttacatgtgc gccaacccga aggggcacgg cggcttgtcg
gtcaagcgca gcgaactgga cgaattcgtt gcttcgaagg tgtgggcacg gctccgcaca
gccgacatgg aagatgaaca cgatcaggca tggattgccg ccgctgcgga gcgcttcgcc
cttcagcacg acctagcggg ggtggccgat gagcggcgcg aacaacaggc gcacctagac
aacgtgcggc gctccatcaa ggaccttcag gcggaccgta agcccggtct gtacgtcggg
cgtgaagagc tggaaacgtg gcgctcaacg gtgctgcaat accggtccta cgaagcggag
tgcacgaccc gactcgctga gcttgacgag aagatgaacg gcagcacccg cgttccgtct
gagtggttca gcggcgaaga cccgacggcc gaagggggca tctgggcaag ctgggacgtg
tacgagcgtc gggagttcct gagcttcttc cttgactccg tcatggtcga ccgggggcgc
caccctgaga cgaagaaata catccccctg aaggaccgtg tgacgctcaa gtgggcggag
ctgctgaagg aggaagacga agcgagcgaa gccactgagc gggagcttgc ggcgctgtag
cgcacagcgg gaggggtcga gccggcggac ggttcggccc cttttttggc cttgaaatcg
ttagttaggc taactagggt accgtctctg ctttgacaac atgctgtgcg gtgttgtaaa
gtctggtgta ggagaatacg acagcttgtc aaaggagtgt ccatatgtca cgacgcgtga
ccgggaccat actgggcggg ttgatcctcg ccatggtccc cttcctttcc accgcggcca
acgccgcacc ccaggccgcg ccggcttccg tctcccaccc gttccaccac tcctgcgcca
cggtgaagcc gggtcgggcg agctgcaatg ccctcgtacg cagcgacatc gcccagagcg
cggcgaccct cgcgcaccaa gcggccgccc catccgggct ctcgccggcc aacctgcaga
gcgcctacaa gctgccgtcc tccacggccg gatccggcca gaccgtcgcg atcgtcgacg
cctatgacgc cccgaccgcc gaagcggact tgaacgtgta ccgaagccag ttcggactcg
gcgcgtgcac gaccgccaac ggctgtttca agaaggtcga ccagaacggc ggcacgtcct
atccgaggaa ggacggcggc tgggcgcagg agatctccct ggacctcgac atggtctccg
cggtctgccc caactgcaag atcgttctcg tcgaggcgaa gaccaactcg ttcgccaacc
tgggtaccgc cgagaacacc gcggcgagtc tcgcgaacgt catcagcaac agctacggcg
gctcggacgc ctctgacgcg agctatggct cgtactacaa ccacccgggc aaggccatca
cggtcagctc cggcgacgcc ggctacggcg tggagtaccc ggcctcgtcc cactacgtga
ccgccgtcgg cggcacctcg ctgcgcaccg cgagcaccag ccgcggctgg agcgagaccg
cgtggagcgg cgcgggcagt ggctgctcgg cctacaacac cgcgctgtcc ggccagtccg
gcctcaccgg ctgctcccgg cgcgccgtcg ccgacgtctc cgccgtggcc gacccggcca
ccggcgtcgc cgtctacgac agcacggcct accaggccca gagcggctgg atggtcttcg
gcggcaccag cgtcgccgca ccgatcatcg gtggcgtgta cggcctcgcc gccaacgccg
cgagcatcga caacaactac ccctacgccc acaccagctc gctcttcgac gtcacgtcgg
gcagcaacgg cacctgcacc accaccaagt ggtgcaccgc cggcaccggc tgggacggcc
ccaccggcct cggaacgccg aacggcaccg gagccttctg aagcttacta ggtatctaga
accggtgacg tcaccatggg aagcttcgtg gatccagata tccctctaga ccgcggccgc
ccggctgcag cccgtacctc tagtagagtc gagggggaac ccggccgcgt ccggcgcccc
cgccgccttc gacgagatcc cgcaaaagcg gcctttgact ccctgcaagc ctcagcgacc
gaatatatcg gttatgcgtg ggcgatggtt gttgtcattg tcggcgcaac tatcggtatc
aagctgttta agaaattcac ctcgaaagca agctgataaa ccgatacaat taaaggctcc
ttttggagcc tttttttttg gagattttca acgtgaaaaa attattattc gcaattcctt
tagttgttcc tttctattct cactccgctg aaactgttga aagttgttta gcaaaacctc
atacagaaaa ttcatttact aacgtctgga aagacgacaa aactttagat cctctagcta
gagtcgaccg gcatgcaatc gaattcgtaa tcatgtcata gctgtttcct gtgtgaaatt
gttatccgct cacaattcca cacaacatac gagccggaag cataaagtgt aaagcctggg
gtgcctaatg agtgagctaa ctcacattaa ttgcgttgcg ctcactgccc gctttccagt
cgggaaacct gtcgtgccag ctgcattaat gaatcggcca acgcgcgggg agaggcggtt
tgcgtattgg gcgctcttcc gcttcctcgc tcactgactc gctgcgctcg gtcgttcggc
tgcggcgagc ggtatcagct cactcaaagg cggtaatacg gttatccaca gaatcagggg
ataacgcagg aaagaacatg tgagcaaaag gccagcaaaa ggccaggaac cgtaaaaagg
ccgcgttgct ggcgtttttc cataggctcc gcccccctga cgagcatcac aaaaatcgac
gctcaagtca gaggtggcga aacccgacag gactataaag ataccaggcg tttccccctg
gaagctccct cgtgcgctct cctgttccga ccctgccgct taccggatac ctgtccgcct
ttctcccttc gggaagcgtg gcgctttctc atagctcacg ctgtaggtat ctcagttcgg
tgtaggtcgt tcgctccaag ctgggctgtg tgcacgaacc ccccgttcag cccgaccgct
gcgccttatc cggtaactat cgtcttgagt ccaacccggt aagacacgac ttatcgccac
tggcagcagc cactggtaac aggattagca gagcgaggta tgtaggcggt gctacagagt
 
tcttgaagtg gtggcctaac tacggctaca ctagaagaac agtatttggt atctgcgctc
tgctgaagcc agttaccttc ggaaaaagag ttggtagctc ttgatccggc aaacaaacca
ccgctggtag cggtggtttt tttgtttgca agcagcagat tacgcgcaga aaaaaaggat
ctcaagaaga tcctttgatc ttttctacgg ggtctgacgc tcagtggaac gaaaactcac
gttaagggat tttggtcatg agattatcaa aaaggatctt cacctagatc cttttggttc
atgtgcagct ccatcagcaa aaggggatga taagtttatc accaccgact atttgcaaca
gtgccgttga tcgtgctatg atcgactgat gtcatcagcg gtggagtgca atgtcgtgca
atacgaatgg cgaaaagccg agctcatcgg tcagcttctc aaccttgggg ttacccccgg
cggtgtgctg ctggtccaca gctccttccg tagcgtccgg cccctcgaag atgggccact
tggactgatc gaggccctgc gtgctgcgct gggtccggga gggacgctcg tcatgccctc
gtggtcaggt ctggacgacg agccgttcga tcctgccacg tcgcccgtta caccggacct
tggagttgtc tctgacacat tctggcgcct gccaaatgta aagcgcagcg cccatccatt
tgcctttgcg gcagcggggc cacaggcaga gcagatcatc tctgatccat tgcccctgcc
acctcactcg cctgcaagcc cggtcgcccg tgtccatgaa ctcgatgggc aggtacttct
cctcggcgtg ggacacgatg ccaacacgac gctgcatctt gccgagttga tggcaaaggt
tccctatggg gtgccgagac actgcaccat tcttcaggat ggcaagttgg tacgcgtcga
ttatctcgag aatgaccact gctgtgagcg ctttgccttg gcggacaggt ggctcaagga
gaagagcctt cagaaggaag gtccagtcgg tcatgccttt gctcggttga tccgctcccg
cgacattgtg gcgacagccc tgggtcaact gggccgagat ccgttgatct tcctgcatcc
gccagaggcg ggatgcgaag aatgcgatgc cgctcgccag tcgattggct gagctcatga
gcggagaacg agatgacgtt ggaggggcaa ggtcgcgctg attgctgggg caacacgtgg
agcggatcgg ggattgtctt tcttcagctc gctgatgata tgctgacgct caatgccgtt
tggcctccga ctaacgaaaa tcccgcattt ggacggctga tccgattggc acggcggacg
gcgaatggcg gagcagacgc tcgtccgggg gcaatgagat atgaaaaagc ctgaactcac
cgcgacgtat cgggccctgg ccagctagct agagtcgacc tgcaggtccc cggggatcgg
tcttgccttg ctcgtcggtg atgtacttca ccagctccgc gaagtcgctc ttcttgatgg
agcgcatggg gacgtgcttg gcaatcacgc gcaccccccg gccgttttag cggctaaaaa
agtcatggct ctgccctcgg gcggaccacg cccatcatga ccttgccaag ctcgtcctgc
ttctcttcga tcttcgccag cagggcgagg atcgtggcat caccgaaccg cgccgtgcgc
gggtcgtcgg tgagccagag tttcagcagg ccgcccaggc ggcccaggtc gccattgatg
cgggccagct cgcggacgtg ctcatagtcc acgacgcccg tgattttgta gccctggccg
acggccagca ggtaggccga caggctcatg ccggccgccg ccgccttttc ctcaatcgct
cttcgttcgt ctggaaggca gtacaccttg ataggtgggc tgcccttcct ggttggcttg
gtttcatcag ccatccgctt gccctcatct gttacgccgg cggtagccgg ccagcctcgc
agagcaggat tcccgttgag caccgccagg tgcgaataag ggacagtgaa gaaggaacac
ccgctcgcgg gtgggcctac ttcacctatc ctgcccggct gacgccgttg gatacaccaa
ggaaagtcta cacgaaccct ttggcaaaat cctgtatatc gtgcgaaaaa ggatggatat
accgaaaaaa tcgctataat gaccccgaag cagggttatg cagcggaaaa gatccgtcga
cctgcaggca tgc
SEQ ID NO: 26 
tggcgccgga cggggcttca gacgtttcgg gtgctgggtt gttgtctctg gacagtgatc
catgggaaac tactcagcac caccaatgtt cccaaaagaa agcgcaggtc agcgcccatg
agccaagatc taggcatgtc gcccttcatc gctcccgacg tccctgagca ccttctagac
actgttcgcg tcttcctgta cgcgcgtcag tctaagggcc ggtccgacgg ctcagacgtg
tcgaccgaag cacagctagc ggccggtcgt gcgttggtcg cgtctcgcaa cgcccagggg
ggtgcgcgct gggtcgtggc aggtgagttc gtggacgtcg ggcgctccgg ctgggacccg
aacgtgaccc gtgccgactt cgagcgcatg atgggcgaag tccgcgccgg cgaaggtgac
gttgtcgttg tgaatgagct ttcccggctc actcgcaagg gcgcccatga cgcgctcgaa
atcgacaacg aattgaagaa gcacggcgtg cgcttcatgt cggttcttga gccgttcctt
gacacgtcta cccctatcgg cgtcgccatt ttcgcgctga tcgctgccct tgcgaaacag
gacagtgacc tgaaggcgga gcgcctgaag ggtgcgaaag acgagattgc cgcgctgggt
ggcgttcact cgtcttccgc cccgttcgga atgcgcgccg tgcgcaagaa ggtcgataat
ctcgtgatct ccgttcttga gccggacgaa gacaacccgg atcacgtcga gctagttgag
cgcatggcga aaatgtcgtt cgaaggcgtg tccgacaacg ccattgcaac gaccttcgag
aaggaaaaga tcccgtcgcc cggaatggct gagagacgcg ccacggaaaa gcgtcttgcg
tccgtcaagg cacgtcgcct gaacggcgct gaaaagccga tcatgtggcg cgctcaaacg
gtccgatgga ttctcaacca tcccgcaatc ggcggtttcg cattcgagcg tgtgaagcac
 
ggtaaggcgc acatcaacgt catacggcgc gaccccggcg gcaagccgct aacgccccac
acgggcattc tcagcggctc gaagtggctt gagcttcaag agaagcgttc cgggaagaat
ctcagcgacc ggaagcctgg ggccgaagtc gaaccgacgc ttctgagcgg gtggcgtttc
ctggggtgcc gaatctgcgg cggctcaatg ggtcagtccc agggtggccg taagcgcaac
ggcgaccttg ccgaaggcaa ttacatgtgc gccaacccga aggggcacgg cggcttgtcg
gtcaagcgca gcgaactgga cgaattcgtt gcttcgaagg tgtgggcacg gctccgcaca
gccgacatgg aagatgaaca cgatcaggca tggattgccg ccgctgcgga gcgcttcgcc
cttcagcacg acctagcggg ggtggccgat gagcggcgcg aacaacaggc gcacctagac
aacgtgcggc gctccatcaa ggaccttcag gcggaccgta agcccggtct gtacgtcggg
cgtgaagagc tggaaacgtg gcgctcaacg gtgctgcaat accggtccta cgaagcggag
tgcacgaccc gactcgctga gcttgacgag aagatgaacg gcagcacccg cgttccgtct
gagtggttca gcggcgaaga cccgacggcc gaagggggca tctgggcaag ctgggacgtg
tacgagcgtc gggagttcct gagcttcttc cttgactccg tcatggtcga ccgggggcgc
caccctgaga cgaagaaata catccccctg aaggaccgtg tgacgctcaa gtgggcggag
ctgctgaagg aggaagacga agcgagcgaa gccactgagc gggagcttgc ggcgctgtag
cgcacagcgg gaggggtcga gccggcggac ggttcggccc cttttttggc cttgaaatcg
ttagttaggc taactagggt accgtctctg ctttgacaac atgctgtgcg gtgttgtaaa
gtctggtgta ggagaatacg acagcttgtc aaaggagtgt ccatatgtca cgacgcgtga
ccgggaccat actgggcggg ttgatcctcg ccatggtccc cttcctttcc accgcggcca
acgccgcacc ccaggccgcg ccggcttccg tctcccaccc gttccaccac tcctgcgcca
cggtgaagcc gggtcgggcg agctgcaatg ccctcgtacg cagcgacatc gcccagagcg
cggcgaccct cgcgcaccaa gcggccgccc catccgggct ctcgccggcc aacctgcaga
gcgcctacaa gctgccgtcc tccacggccg gatccggcca gaccgtcgcg atcgtcgacg
cctatgacgc cccgaccgcc gaagcggact tgaacgtgta ccgaagccag ttcggactcg
gcgcgtgcac gaccgccaac ggctgtttca agaaggtcga ccagaacggc ggcacgtcct
atccgaggaa ggacggcggc tgggcgcagg agatctccct ggacctcgac atggtctccg
cggtctgccc caactgcaag atcgttctcg tcgaggcgaa gaccaactcg ttcgccaacc
tgggtaccgc cgagaacacc gcggcgagtc tcgcgaacgt catcagcaac agctacggcg
gctcggacgc ctctgacgcg agctatggct cgtactacaa ccacccgggc aaggccatca
cggtcagctc cggcgacgcc ggctacggcg tggagtaccc ggcctcgtcc cactacgtga
ccgccgtcgg cggcacctcg ctgcgcaccg cgagcaccag ccgcggctgg agcgagaccg
cgtggagcgg cgcgggcagt ggctgctcgg cctacaacac cgcgctgtcc ggccagtccg
gcctcaccgg ctgctcccgg cgcgccgtcg ccgacgtctc cgccgtggcc gacccggcca
ccggcgtcgc cgtctacgac agcacggcct accaggccca gagcggctgg atggtcttcg
gcggcaccag cgtcgccgca ccgatcatcg gtggcgtgta cggcctcgcc gccaacgccg
cgagcatcga caacaactac ccctacgccc acaccagctc gctcttcgac gtcacgtcgg
gcagcaacgg cacctgcacc accaccaagt ggtgcaccgc cggcaccggc tgggacggcc
ccaccggcct cggaacgccg aacggcaccg gagccttcgg acaccaccac caccaccact
gaagcttact aggtatctag aaccggtgac gtcaccatgg gaagcttcgt ggatccagat
atccctctag accgcggccg cccggctgca gcccgtacct ctagtagagt cgagggggaa
cccggccgcg tccggcgccc ccgccgcctt cgacgagatc ccgcaaaagc ggcctttgac
tccctgcaag cctcagcgac cgaatatatc ggttatgcgt gggcgatggt tgttgtcatt
gtcggcgcaa ctatcggtat caagctgttt aagaaattca cctcgaaagc aagctgataa
accgatacaa ttaaaggctc cttttggagc cttttttttt ggagattttc aacgtgaaaa
aattattatt cgcaattcct ttagttgttc ctttctattc tcactccgct gaaactgttg
aaagttgttt agcaaaacct catacagaaa attcatttac taacgtctgg aaagacgaca
aaactttaga tcctctagct agagtcgacc ggcatgcaat cgaattcgta atcatgtcat
agctgtttcc tgtgtgaaat tgttatccgc tcacaattcc acacaacata cgagccggaa
gcataaagtg taaagcctgg ggtgcctaat gagtgagcta actcacatta attgcgttgc
gctcactgcc cgctttccag tcgggaaacc tgtcgtgcca gctgcattaa tgaatcggcc
aacgcgcggg gagaggcggt ttgcgtattg ggcgctcttc cgcttcctcg ctcactgact
cgctgcgctc ggtcgttcgg ctgcggcgag cggtatcagc tcactcaaag gcggtaatac
ggttatccac agaatcaggg gataacgcag gaaagaacat gtgagcaaaa ggccagcaaa
aggccaggaa ccgtaaaaag gccgcgttgc tggcgttttt ccataggctc cgcccccctg
acgagcatca caaaaatcga cgctcaagtc agaggtggcg aaacccgaca ggactataaa
gataccaggc gtttccccct ggaagctccc tcgtgcgctc tcctgttccg accctgccgc
ttaccggata cctgtccgcc tttctccctt cgggaagcgt ggcgctttct catagctcac
gctgtaggta tctcagttcg gtgtaggtcg ttcgctccaa gctgggctgt gtgcacgaac
 
cccccgttca gcccgaccgc tgcgccttat ccggtaacta tcgtcttgag tccaacccgg
taagacacga cttatcgcca ctggcagcag ccactggtaa caggattagc agagcgaggt
atgtaggcgg tgctacagag ttcttgaagt ggtggcctaa ctacggctac actagaagaa
cagtatttgg tatctgcgct ctgctgaagc cagttacctt cggaaaaaga gttggtagct
cttgatccgg caaacaaacc accgctggta gcggtggttt ttttgtttgc aagcagcaga
ttacgcgcag aaaaaaagga tctcaagaag atcctttgat cttttctacg gggtctgacg
ctcagtggaa cgaaaactca cgttaaggga ttttggtcat gagattatca aaaaggatct
tcacctagat ccttttggtt catgtgcagc tccatcagca aaaggggatg ataagtttat
caccaccgac tatttgcaac agtgccgttg atcgtgctat gatcgactga tgtcatcagc
ggtggagtgc aatgtcgtgc aatacgaatg gcgaaaagcc gagctcatcg gtcagcttct
caaccttggg gttacccccg gcggtgtgct gctggtccac agctccttcc gtagcgtccg
gcccctcgaa gatgggccac ttggactgat cgaggccctg cgtgctgcgc tgggtccggg
agggacgctc gtcatgccct cgtggtcagg tctggacgac gagccgttcg atcctgccac
gtcgcccgtt acaccggacc ttggagttgt ctctgacaca ttctggcgcc tgccaaatgt
aaagcgcagc gcccatccat ttgcctttgc ggcagcgggg ccacaggcag agcagatcat
ctctgatcca ttgcccctgc cacctcactc gcctgcaagc ccggtcgccc gtgtccatga
actcgatggg caggtacttc tcctcggcgt gggacacgat gccaacacga cgctgcatct
tgccgagttg atggcaaagg ttccctatgg ggtgccgaga cactgcacca ttcttcagga
tggcaagttg gtacgcgtcg attatctcga gaatgaccac tgctgtgagc gctttgcctt
ggcggacagg tggctcaagg agaagagcct tcagaaggaa ggtccagtcg gtcatgcctt
tgctcggttg atccgctccc gcgacattgt ggcgacagcc ctgggtcaac tgggccgaga
tccgttgatc ttcctgcatc cgccagaggc gggatgcgaa gaatgcgatg ccgctcgcca
gtcgattggc tgagctcatg agcggagaac gagatgacgt tggaggggca aggtcgcgct
gattgctggg gcaacacgtg gagcggatcg gggattgtct ttcttcagct cgctgatgat
atgctgacgc tcaatgccgt ttggcctccg actaacgaaa atcccgcatt tggacggctg
atccgattgg cacggcggac ggcgaatggc ggagcagacg ctcgtccggg ggcaatgaga
tatgaaaaag cctgaactca ccgcgacgta tcgggccctg gccagctagc tagagtcgac
ctgcaggtcc ccggggatcg gtcttgcctt gctcgtcggt gatgtacttc accagctccg
cgaagtcgct cttcttgatg gagcgcatgg ggacgtgctt ggcaatcacg cgcacccccc
ggccgtttta gcggctaaaa aagtcatggc tctgccctcg ggcggaccac gcccatcatg
accttgccaa gctcgtcctg cttctcttcg atcttcgcca gcagggcgag gatcgtggca
tcaccgaacc gcgccgtgcg cgggtcgtcg gtgagccaga gtttcagcag gccgcccagg
cggcccaggt cgccattgat gcgggccagc tcgcggacgt gctcatagtc cacgacgccc
gtgattttgt agccctggcc gacggccagc aggtaggccg acaggctcat gccggccgcc
gccgcctttt cctcaatcgc tcttcgttcg tctggaaggc agtacacctt gataggtggg
ctgcccttcc tggttggctt ggtttcatca gccatccgct tgccctcatc tgttacgccg
gcggtagccg gccagcctcg cagagcagga ttcccgttga gcaccgccag gtgcgaataa
gggacagtga agaaggaaca cccgctcgcg ggtgggccta cttcacctat cctgcccggc
tgacgccgtt ggatacacca aggaaagtct acacgaaccc tttggcaaaa tcctgtatat
cgtgcgaaaa aggatggata taccgaaaaa atcgctataa tgaccccgaa gcagggttat
gcagcggaaa agatccgtcg acctgcaggc atgc
SEQ ID NO: 27 
tggcgccgga cggggcttca gacgtttcgg gtgctgggtt gttgtctctg gacagtgatc
catgggaaac tactcagcac caccaatgtt cccaaaagaa agcgcaggtc agcgcccatg
agccaagatc taggcatgtc gcccttcatc gctcccgacg tccctgagca ccttctagac
actgttcgcg tcttcctgta cgcgcgtcag tctaagggcc ggtccgacgg ctcagacgtg
tcgaccgaag cacagctagc ggccggtcgt gcgttggtcg cgtctcgcaa cgcccagggg
ggtgcgcgct gggtcgtggc aggtgagttc gtggacgtcg ggcgctccgg ctgggacccg
aacgtgaccc gtgccgactt cgagcgcatg atgggcgaag tccgcgccgg cgaaggtgac
gttgtcgttg tgaatgagct ttcccggctc actcgcaagg gcgcccatga cgcgctcgaa
atcgacaacg aattgaagaa gcacggcgtg cgcttcatgt cggttcttga gccgttcctt
gacacgtcta cccctatcgg cgtcgccatt ttcgcgctga tcgctgccct tgcgaaacag
gacagtgacc tgaaggcgga gcgcctgaag ggtgcgaaag acgagattgc cgcgctgggt
ggcgttcact cgtcttccgc cccgttcgga atgcgcgccg tgcgcaagaa ggtcgataat
ctcgtgatct ccgttcttga gccggacgaa gacaacccgg atcacgtcga gctagttgag
cgcatggcga aaatgtcgtt cgaaggcgtg tccgacaacg ccattgcaac gaccttcgag
aaggaaaaga tcccgtcgcc cggaatggct gagagacgcg ccacggaaaa gcgtcttgcg
 
tccgtcaagg cacgtcgcct gaacggcgct gaaaagccga tcatgtggcg cgctcaaacg
gtccgatgga ttctcaacca tcccgcaatc ggcggtttcg cattcgagcg tgtgaagcac
ggtaaggcgc acatcaacgt catacggcgc gaccccggcg gcaagccgct aacgccccac
acgggcattc tcagcggctc gaagtggctt gagcttcaag agaagcgttc cgggaagaat
ctcagcgacc ggaagcctgg ggccgaagtc gaaccgacgc ttctgagcgg gtggcgtttc
ctggggtgcc gaatctgcgg cggctcaatg ggtcagtccc agggtggccg taagcgcaac
ggcgaccttg ccgaaggcaa ttacatgtgc gccaacccga aggggcacgg cggcttgtcg
gtcaagcgca gcgaactgga cgaattcgtt gcttcgaagg tgtgggcacg gctccgcaca
gccgacatgg aagatgaaca cgatcaggca tggattgccg ccgctgcgga gcgcttcgcc
cttcagcacg acctagcggg ggtggccgat gagcggcgcg aacaacaggc gcacctagac
aacgtgcggc gctccatcaa ggaccttcag gcggaccgta agcccggtct gtacgtcggg
cgtgaagagc tggaaacgtg gcgctcaacg gtgctgcaat accggtccta cgaagcggag
tgcacgaccc gactcgctga gcttgacgag aagatgaacg gcagcacccg cgttccgtct
gagtggttca gcggcgaaga cccgacggcc gaagggggca tctgggcaag ctgggacgtg
tacgagcgtc gggagttcct gagcttcttc cttgactccg tcatggtcga ccgggggcgc
caccctgaga cgaagaaata catccccctg aaggaccgtg tgacgctcaa gtgggcggag
ctgctgaagg aggaagacga agcgagcgaa gccactgagc gggagcttgc ggcgctgtag
cgcacagcgg gaggggtcga gccggcggac ggttcggccc cttttttggc cttgaaatcg
ttagttaggc taactagggt accgtctctg ctttgacaac atgctgtgcg gtgttgtaaa
gtctggtgta ggagaatacg acagcttgtc aaaggagtgt ccatatgcgt cgcaccctca
aggccgtggg agcagccgcg gcggcggcca cctgcgtcct cgccgcgacg gcaggcaccg
cgcaggccga ggctagcgtc tcccacccgt tccaccactc ctgcgccacg gtgaagccgg
gtcgggcgag ctgcaatgcc ctcgtacgca gcgacatcgc ccagagcgcg gcgaccctcg
cgcaccaagc ggccgcccca tccgggctct cgccggccaa cctgcagagc gcctacaagc
tgccgtcctc cacggccgga tccggccaga ccgtcgcgat cgtcgacgcc tatgacgccc
cgaccgccga agcggacttg aacgtgtacc gaagccagtt cggactcggc gcgtgcacga
ccgccaacgg ctgtttcaag aaggtcgacc agaacggcgg cacgtcctat ccgaggaagg
acggcggctg ggcgcaggag atctccctgg acctcgacat ggtctccgcg gtctgcccca
actgcaagat cgttctcgtc gaggcgaaga ccaactcgtt cgccaacctg ggtaccgccg
agaacaccgc ggcgagtctc gcgaacgtca tcagcaacag ctacggcggc tcggacgcct
ctgacgcgag ctatggctcg tactacaacc acccgggcaa ggccatcacg gtcagctccg
gcgacgccgg ctacggcgtg gagtacccgg cctcgtccca ctacgtgacc gccgtcggcg
gcacctcgct gcgcaccgcg agcaccagcc gcggctggag cgagaccgcg tggagcggcg
cgggcagtgg ctgctcggcc tacaacaccg cgctgtccgg ccagtccggc ctcaccggct
gctcccggcg cgccgtcgcc gacgtctccg ccgtggccga cccggccacc ggcgtcgccg
tctacgacag cacggcctac cagggccaga gcggctggat ggtcttcggc ggcaccagcg
tcgccgcacc gatcatcggt ggcgtgtacg gcctcgccgc caacgccgcg agcatcgaca
acaactaccc ctacgcccac accagctcgc tcttcgacgt cacgtcgggc agcaacggca
cctgcaccac caccaagtgg tgcaccgccg gcaccggctg ggacggcccc accggcctcg
gaacgccgaa cggcaccgga gccttctgaa gcttactagg tatctagaac cggtgacgtc
accatgggaa gcttcgtgga tccagatatc cctctagacc gcggccgccc ggctgcagcc
cgtacctcta gtagagtcga gggggaaccc ggccgcgtcc ggcgcccccg ccgccttcga
cgagatcccg caaaagcggc ctttgactcc ctgcaagcct cagcgaccga atatatcggt
tatgcgtggg cgatggttgt tgtcattgtc ggcgcaacta tcggtatcaa gctgtttaag
aaattcacct cgaaagcaag ctgataaacc gatacaatta aaggctcctt ttggagcctt
tttttttgga gattttcaac gtgaaaaaat tattattcgc aattccttta gttgttcctt
tctattctca ctccgctgaa actgttgaaa gttgtttagc aaaacctcat acagaaaatt
catttactaa cgtctggaaa gacgacaaaa ctttagatcc tctagctaga gtcgaccggc
atgcaatcga attcgtaatc atgtcatagc tgtttcctgt gtgaaattgt tatccgctca
caattccaca caacatacga gccggaagca taaagtgtaa agcctggggt gcctaatgag
tgagctaact cacattaatt gcgttgcgct cactgcccgc tttccagtcg ggaaacctgt
cgtgccagct gcattaatga atcggccaac gcgcggggag aggcggtttg cgtattgggc
gctcttccgc ttcctcgctc actgactcgc tgcgctcggt cgttcggctg cggcgagcgg
tatcagctca ctcaaaggcg gtaatacggt tatccacaga atcaggggat aacgcaggaa
agaacatgtg agcaaaaggc cagcaaaagg ccaggaaccg taaaaaggcc gcgttgctgg
cgtttttcca taggctccgc ccccctgacg agcatcacaa aaatcgacgc tcaagtcaga
ggtggcgaaa cccgacagga ctataaagat accaggcgtt tccccctgga agctccctcg
tgcgctctcc tgttccgacc ctgccgctta ccggatacct gtccgccttt ctcccttcgg
 
gaagcgtggc gctttctcat agctcacgct gtaggtatct cagttcggtg taggtcgttc
gctccaagct gggctgtgtg cacgaacccc ccgttcagcc cgaccgctgc gccttatccg
gtaactatcg tcttgagtcc aacccggtaa gacacgactt atcgccactg gcagcagcca
ctggtaacag gattagcaga gcgaggtatg taggcggtgc tacagagttc ttgaagtggt
ggcctaacta cggctacact agaagaacag tatttggtat ctgcgctctg ctgaagccag
ttaccttcgg aaaaagagtt ggtagctctt gatccggcaa acaaaccacc gctggtagcg
gtggtttttt tgtttgcaag cagcagatta cgcgcagaaa aaaaggatct caagaagatc
ctttgatctt ttctacgggg tctgacgctc agtggaacga aaactcacgt taagggattt
tggtcatgag attatcaaaa aggatcttca cctagatcct tttggttcat gtgcagctcc
atcagcaaaa ggggatgata agtttatcac caccgactat ttgcaacagt gccgttgatc
gtgctatgat cgactgatgt catcagcggt ggagtgcaat gtcgtgcaat acgaatggcg
aaaagccgag ctcatcggtc agcttctcaa ccttggggtt acccccggcg gtgtgctgct
ggtccacagc tccttccgta gcgtccggcc cctcgaagat gggccacttg gactgatcga
ggccctgcgt gctgcgctgg gtccgggagg gacgctcgtc atgccctcgt ggtcaggtct
ggacgacgag ccgttcgatc ctgccacgtc gcccgttaca ccggaccttg gagttgtctc
tgacacattc tggcgcctgc caaatgtaaa gcgcagcgcc catccatttg cctttgcggc
agcggggcca caggcagagc agatcatctc tgatccattg cccctgccac ctcactcgcc
tgcaagcccg gtcgcccgtg tccatgaact cgatgggcag gtacttctcc tcggcgtggg
acacgatgcc aacacgacgc tgcatcttgc cgagttgatg gcaaaggttc cctatggggt
gccgagacac tgcaccattc ttcaggatgg caagttggta cgcgtcgatt atctcgagaa
tgaccactgc tgtgagcgct ttgccttggc ggacaggtgg ctcaaggaga agagccttca
gaaggaaggt ccagtcggtc atgcctttgc tcggttgatc cgctcccgcg acattgtggc
gacagccctg ggtcaactgg gccgagatcc gttgatcttc ctgcatccgc cagaggcggg
atgcgaagaa tgcgatgccg ctcgccagtc gattggctga gctcatgagc ggagaacgag
atgacgttgg aggggcaagg tcgcgctgat tgctggggca acacgtggag cggatcgggg
attgtctttc ttcagctcgc tgatgatatg ctgacgctca atgccgtttg gcctccgact
aacgaaaatc ccgcatttgg acggctgatc cgattggcac ggcggacggc gaatggcgga
gcagacgctc gtccgggggc aatgagatat gaaaaagcct gaactcaccg cgacgtatcg
ggccctggcc agctagctag agtcgacctg caggtccccg gggatcggtc ttgccttgct
cgtcggtgat gtacttcacc agctccgcga agtcgctctt cttgatggag cgcatgggga
cgtgcttggc aatcacgcgc acccccggcc gttttagcgg ctaaaaaag tcatggctct
gccctcgggc ggaccacgcc catcatgacc ttgccaagct cgtcctgctt ctcttcgatc
ttcgccagca gggcgaggat cgtggcatca ccgaaccgcg ccgtgcgcgg gtcgtcggtg
agccagagtt tcagcaggcc gcccaggcgg cccaggtcgc cattgatgcg ggccagctcg
cggacgtgct catagtccac gacgcccgtg attttgtagc cctggccgac ggccagcagg
taggccgaca ggctcatgcc ggccgccgcc gccttttcct caatcgctct tcgttcgtct
ggaaggcagt acaccttgat aggtgggctg cccttcctgg ttggcttggt ttcatcagcc
atccgcttgc cctcatctgt tacgccggcg gtagccggcc agcctcgcag agcaggattc
ccgttgagca ccgccaggtg cgaataaggg acagtgaaga aggaacaccc gctcgcgggt
gggcctactt cacctatcct gcccggctga cgccgttgga tacaccaagg aaagtctaca
cgaacccttt ggcaaaatcc tgtatatcgt gcgaaaaagg atggatatac cgaaaaaatc
gctataatga ccccgaagca gggttatgca gcggaaaaga tccgtcgacc tgcaggcatg
c
SEQ ID NO: 28 
tggcgccgga cggggcttca gacgtttcgg gtgctgggtt gttgtctctg gacagtgatc
catgggaaac tactcagcac caccaatgtt cccaaaagaa agcgcaggtc agcgcccatg
agccaagatc taggcatgtc gcccttcatc gctcccgacg tccctgagca ccttctagac
actgttcgcg tcttcctgta cgcgcgtcag tctaagggcc ggtccgacgg ctcagacgtg
tcgaccgaag cacagctagc ggccggtcgt gcgttggtcg cgtctcgcaa cgcccagggg
ggtgcgcgct gggtcgtggc aggtgagttc gtggacgtcg ggcgctccgg ctgggacccg
aacgtgaccc gtgccgactt cgagcgcatg atgggcgaag tccgcgccgg cgaaggtgac
gttgtcgttg tgaatgagct ttcccggctc actcgcaagg gcgcccatga cgcgctcgaa
atcgacaacg aattgaagaa gcacggcgtg cgcttcatgt cggttcttga gccgttcctt
gacacgtcta cccctatcgg cgtcgccatt ttcgcgctga tcgctgccct tgcgaaacag
gacagtgacc tgaaggcgga gcgcctgaag ggtgcgaaag acgagattgc cgcgctgggt
ggcgttcact cgtcttccgc cccgttcgga atgcgcgccg tgcgcaagaa ggtcgataat
ctcgtgatct ccgttcttga gccggacgaa gacaacccgg atcacgtcga gctagttgag
cgcatggcga aaatgtcgtt cgaaggcgtg tccgacaacg ccattgcaac gaccttcgag
 
aaggaaaaga tcccgtcgcc cggaatggct gagagacgcg ccacggaaaa gcgtcttgcg
tccgtcaagg cacgtcgcct gaacggcgct gaaaagccga tcatgtggcg cgctcaaacg
gtccgatgga ttctcaacca tcccgcaatc ggcggtttcg cattcgagcg tgtgaagcac
ggtaaggcgc acatcaacgt catacggcgc gaccccggcg gcaagccgct aacgccccac
acgggcattc tcagcggctc gaagtggctt gagcttcaag agaagcgttc cgggaagaat
ctcagcgacc ggaagcctgg ggccgaagtc gaaccgacgc ttctgagcgg gtggcgtttc
ctggggtgcc gaatctgcgg cggctcaatg ggtcagtccc agggtggccg taagcgcaac
ggcgaccttg ccgaaggcaa ttacatgtgc gccaacccga aggggcacgg cggcttgtcg
gtcaagcgca gcgaactgga cgaattcgtt gcttcgaagg tgtgggcacg gctccgcaca
gccgacatgg aagatgaaca cgatcaggca tggattgccg ccgctgcgga gcgcttcgcc
cttcagcacg acctagcggg ggtggccgat gagcggcgcg aacaacaggc gcacctagac
aacgtgcggc gctccatcaa ggaccttcag gcggaccgta agcccggtct gtacgtcggg
cgtgaagagc tggaaacgtg gcgctcaacg gtgctgcaat accggtccta cgaagcggag
tgcacgaccc gactcgctga gcttgacgag aagatgaacg gcagcacccg cgttccgtct
gagtggttca gcggcgaaga cccgacggcc gaagggggca tctgggcaag ctgggacgtg
tacgagcgtc gggagttcct gagcttcttc cttgactccg tcatggtcga ccgggggcgc
caccctgaga cgaagaaata catccccctg aaggaccgtg tgacgctcaa gtgggcggag
ctgctgaagg aggaagacga agcgagcgaa gccactgagc gggagcttgc ggcgctgtag
cgcacagcgg gaggggtcga gccggcggac ggttcggccc cttttttggc cttgaaatcg
ttagttaggc taactagggt accgtctctg ctttgacaac atgctgtgcg gtgttgtaaa
gtctggtgta ggagaatacg acagcttgtc aaaggagtgt ccatatgcgt cgcaccctca
aggccgtggg agcagccgcg gcggcggcca cctgcgtcct cgccgcgacg gcaggcaccg
cgcaggccga ggctagcgtc tcccacccgt tccaccactc ctgcgccacg gtgaagccgg
gtcgggcgag ctgcaatgcc ctcgtacgca gcgacatcgc ccagagcgcg gcgaccctcg
cgcaccaagc ggccgcccca tccgggctct cgccggccaa cctgcagagc gcctacaagc
tgccgtcctc cacggccgga tccggccaga ccgtcgcgat cgtcgacgcc tatgacgccc
cgaccgccga agcggacttg aacgtgtacc gaagccagtt cggactcggc gcgtgcacga
ccgccaacgg ctgtttcaag aaggtcgacc agaacggcgg cacgtcctat ccgaggaagg
acggcggctg ggcgcaggag atctccctgg acctcgacat ggtctccgcg gtctgcccca
actgcaagat cgttctcgtc gaggcgaaga ccaactcgtt cgccaacctg ggtaccgccg
agaacaccgc ggcgagtctc gcgaacgtca tcagcaacag ctacggcggc tcggacgcct
ctgacgcgag ctatggctcg tactacaacc acccgggcaa ggccatcacg gtcagctccg
gcgacgccgg ctacggcgtg gagtacccgg cctcgtccca ctacgtgacc gccgtcggcg
gcacctcgct gcgcaccgcg agcaccagcc gcggctggag cgagaccgcg tggagcggcg
cgggcagtgg ctgctcggcc tacaacaccg cgctgtccgg ccagtccggc ctcaccggct
gctcccggcg cgccgtcgcc gacgtctccg ccgtggccga cccggccacc ggcgtcgccg
tctacgacag cacggcctac cagggccaga gcggctggat ggtcttcggc ggcaccagcg
tcgccgcacc gatcatcggt ggcgtgtacg gcctcgccgc caacgccgcg agcatcgaca
acaactaccc ctacgcccac accagctcgc tcttcgacgt cacgtcgggc agcaacggca
cctgcaccac caccaagtgg tgcaccgccg gcaccggctg ggacggcccc accggcctcg
gaacgccgaa cggcaccgga gccttcggac accaccacca ccaccactga agcttactag
gtatctagaa ccggtgacgt caccatggga agcttcgtgg atccagatat ccctctagac
cgcggccgcc cggctgcagc ccgtacctct agtagagtcg agggggaacc cggccgcgtc
cggcgccccc gccgccttcg acgagatccc gcaaaagcgg cctttgactc cctgcaagcc
tcagcgaccg aatatatcgg ttatgcgtgg gcgatggttg ttgtcattgt cggcgcaact
atcggtatca agctgtttaa gaaattcacc tcgaaagcaa gctgataaac cgatacaatt
aaaggctcct tttggagcct ttttttttgg agattttcaa cgtgaaaaaa ttattattcg
caattccttt agttgttcct ttctattctc actccgctga aactgttgaa agttgtttag
caaaacctca tacagaaaat tcatttacta acgtctggaa agacgacaaa actttagatc
ctctagctag agtcgaccgg catgcaatcg aattcgtaat catgtcatag ctgtttcctg
tgtgaaattg ttatccgctc acaattccac acaacatacg agccggaagc ataaagtgta
aagcctgggg tgcctaatga gtgagctaac tcacattaat tgcgttgcgc tcactgcccg
ctttccagtc gggaaacctg tcgtgccagc tgcattaatg aatcggccaa cgcgcgggga
gaggcggttt gcgtattggg cgctcttccg cttcctcgct cactgactcg ctgcgctcgg
tcgttcggct gcggcgagcg gtatcagctc actcaaaggc ggtaatacgg ttatccacag
aatcagggga taacgcagga aagaacatgt gagcaaaagg ccagcaaaag gccaggaacc
gtaaaaaggc cgcgttgctg gcgtttttcc ataggctccg cccccctgac gagcatcaca
aaaatcgacg ctcaagtcag aggtggcgaa acccgacagg actataaaga taccaggcgt
 
ttccccctgg aagctccctc gtgcgctctc ctgttccgac cctgccgctt accggatacc
tgtccgcctt tctcccttcg ggaagcgtgg cgctttctca tagctcacgc tgtaggtatc
tcagttcggt gtaggtcgtt cgctccaagc tgggctgtgt gcacgaaccc cccgttcagc
ccgaccgctg cgccttatcc ggtaactatc gtcttgagtc caacccggta agacacgact
tatcgccact ggcagcagcc actggtaaca ggattagcag agcgaggtat gtaggcggtg
ctacagagtt cttgaagtgg tggcctaact acggctacac tagaagaaca gtatttggta
tctgcgctct gctgaagcca gttaccttcg gaaaaagagt tggtagctct tgatccggca
aacaaaccac cgctggtagc ggtggttttt ttgtttgcaa gcagcagatt acgcgcagaa
aaaaaggatc tcaagaagat cctttgatct tttctacggg gtctgacgct cagtggaacg
aaaactcacg ttaagggatt ttggtcatga gattatcaaa aaggatcttc acctagatcc
ttttggttca tgtgcagctc catcagcaaa aggggatgat aagtttatca ccaccgacta
tttgcaacag tgccgttgat cgtgctatga tcgactgatg tcatcagcgg tggagtgcaa
tgtcgtgcaa tacgaatggc gaaaagccga gctcatcggt cagcttctca accttggggt
tacccccggc ggtgtgctgc tggtccacag ctccttccgt agcgtccggc ccctcgaaga
tgggccactt ggactgatcg aggccctgcg tgctgcgctg ggtccgggag ggacgctcgt
catgccctcg tggtcaggtc tggacgacga gccgttcgat cctgccacgt cgcccgttac
accggacctt ggagttgtct ctgacacatt ctggcgcctg ccaaatgtaa agcgcagcgc
ccatccattt gcctttgcgg cagcgggccc acaggcagag cagatcatct ctgatccatt
gcccctgcca cctcactcgc ctgcaagccc ggtcgcccgt gtccatgaac tcgatgggca
ggtacttctc ctcggcgtgg gacacgatgc caacacgacg ctgcatcttg ccgagttgat
ggcaaaggtt ccctatgggg tgccgagaca ctgcaccatt cttcaggatg gcaagttggt
acgcgtcgat tatctcgaga atgaccactg ctgtgagcgc tttgccttgg cggacaggtg
gctcaaggag aagagccttc agaaggaagg tccagtcggt catgcctttg ctcggttgat
ccgctcccgc gacattgtgg cgacagccct gggtcaactg ggccgagatc cgttgatctt
cctgcatccg ccagaggcgg gatgcgaaga atgcgatgcc gctcgccagt cgattggctg
agctcatgag cggagaacga gatgacgttg gaggggcaag gtcgcgctga ttgctggggc
aacacgtgga gcggatcggg gattgtcttt cttcagctcg ctgatgatat gctgacgctc
aatgccgttt ggcctccgac taacgaaaat cccgcatttg gacggctgat ccgattggca
cggcggacgg cgaatggcgg agcagacgct cgtccggggg caatgagata tgaaaaagcc
tgaactcacc gcgacgtatc gggccctggc cagctagcta gagtcgacct gcaggtcccc
ggggatcggt cttgccttgc tcgtcggtga tgtacttcac cagctccgcg aagtcgctct
tcttgatgga gcgcatgggg acgtgcttgg caatcacgcg caccccccgg ccgttttagc
ggctaaaaaa gtcatggctc tgccctcggg cggaccacgc ccatcatgac cttgccaagc
tcgtcctgct tctcttcgat cttcgccagc agggcgagga tcgtggcatc accgaaccgc
gccgtgcgcg ggtcgtcggt gagccagagt ttcagcaggc cgcccaggcg gcccaggtcg
ccattgatgc gggccagctc gcggacgtgc tcatagtcca cgacgcccgt gattttgtag
ccctggccga cggccagcag gtaggccgac aggctcatgc cggccgccgc cgccttttcc
tcaatcgctc ttcgttcgtc tggaaggcag tacaccttga taggtgggct gcccttcctg
gttggcttgg tttcatcagc catccgcttg ccctcatctg ttacgccggc ggtagccggc
cagcctcgca gagcaggatt cccgttgagc accgccaggt gcgaataagg gacagtgaag
aaggaacacc cgctcgcggg tgggcctact tcacctatcc tgcccggctg acgccgttgg
atacaccaag gaaagtctac acgaaccctt tggcaaaatc ctgtatatcg tgcgaaaaag
gatggatata ccgaaaaaat cgctataatg accccgaagc agggttatgc agcggaaaag
atccgtcgac ctgcaggcat gc
SEQ ID NO: 29 
tggcgccgga cggggcttca gacgtttcgg gtgctgggtt gttgtctctg gacagtgatc
catgggaaac tactcagcac caccaatgtt cccaaaagaa agcgcaggtc agcgcccatg
agccaagatc taggcatgtc gcccttcatc gctcccgacg tccctgagca ccttctagac
actgttcgcg tcttcctgta cgcgcgtcag tctaagggcc ggtccgacgg ctcagacgtg
tcgaccgaag cacagctagc ggccggtcgt gcgttggtcg cgtctcgcaa cgcccagggg
ggtgcgcgct gggtcgtggc aggtgagttc gtggacgtcg ggcgctccgg ctgggacccg
aacgtgaccc gtgccgactt cgagcgcatg atgggcgaag tccgcgccgg cgaaggtgac
gttgtcgttg tgaatgagct ttcccggctc actcgcaagg gcgcccatga cgcgctcgaa
atcgacaacg aattgaagaa gcacggcgtg cgcttcatgt cggttcttga gccgttcctt
gacacgtcta cccctatcgg cgtcgccatt ttcgcgctga tcgctgccct tgcgaaacag
gacagtgacc tgaaggcgga gcgcctgaag ggtgcgaaag acgagattgc cgcgctgggt
ggcgttcact cgtcttccgc cccgttcgga atgcgcgccg tgcgcaagaa ggtcgataat
 
ctcgtgatct ccgttcttga gccggacgaa gacaacccgg atcacgtcga gctagttgag
cgcatggcga aaatgtcgtt cgaaggcgtg tccgacaacg ccattgcaac gaccttcgag
aaggaaaaga tcccgtcgcc cggaatggct gagagacgcg ccacggaaaa gcgtcttgcg
tccgtcaagg cacgtcgcct gaacggcgct gaaaagccga tcatgtggcg cgctcaaacg
gtccgatgga ttctcaacca tcccgcaatc ggcggtttcg cattcgagcg tgtgaagcac
ggtaaggcgc acatcaacgt catacggcgc gaccccggcg gcaagccgct aacgccccac
acgggcattc tcagcggctc gaagtggctt gagcttcaag agaagcgttc cgggaagaat
ctcagcgacc ggaagcctgg ggccgaagtc gaaccgacgc ttctgagcgg gtggcgtttc
ctggggtgcc gaatctgcgg cggctcaatg ggtcagtccc agggtggccg taagcgcaac
ggcgaccttg ccgaaggcaa ttacatgtgc gccaacccga aggggcacgg cggcttgtcg
gtcaagcgca gcgaactgga cgaattcgtt gcttcgaagg tgtgggcacg gctccgcaca
gccgacatgg aagatgaaca cgatcaggca tggattgccg ccgctgcgga gcgcttcgcc
cttcagcacg acctagcggg ggtggccgat gagcggcgcg aacaacaggc gcacctagac
aacgtgcggc gctccatcaa ggaccttcag gcggaccgta agcccggtct gtacgtcggg
cgtgaagagc tggaaacgtg gcgctcaacg gtgctgcaat accggtccta cgaagcggag
tgcacgaccc gactcgctga gcttgacgag aagatgaacg gcagcacccg cgttccgtct
gagtggttca gcggcgaaga cccgacggcc gaagggggca tctgggcaag ctgggacgtg
tacgagcgtc gggagttcct gagcttcttc cttgactccg tcatggtcga ccgggggcgc
caccctgaga cgaagaaata catccccctg aaggaccgtg tgacgctcaa gtgggcggag
ctgctgaagg aggaagacga agcgagcgaa gccactgagc gggagcttgc ggcgctgtag
cgcacagcgg gaggggtcga gccggcggac ggttcggccc cttttttggc cttgaaatcg
ttagttaggc taactagggt accgtctctg ctttgacaac atgctgtgcg gtgttgtaaa
gtctggtgta ggagaatacg acagcttgtc aaaggagtgt ccatatgctg cggcccgtcg
aaaccccgac ccgcgagatc aagaagctgg acggcctgtg ggcgttcagc ctcgaccggg
agaactgcgg tatcgaccag cggtggtggg agagcgccct ccaggagtcg cgcgccatcg
ccgtccccgg cagcttcaac gaccagttcg cggacgcgga catccgcaac tacgcgggga
acgtctggta tcagcgggag gtgttcatcc ccaagggctg ggccggtcag cgcatcgtgc
tgcgcttcga cgccgtgacc cactacggca aggtctgggt caacaaccag gaagtgatgg
agcaccaggg cggctacacg cccttcgagg cggacgtgac cccgtacgtc atcgccggca
agtcggtccg catcacggtc tgcgtcaaca acgagctcaa ctggcagacc atcccgcccg
gcatggtgat cacggacgag aacggcaaga agaagcagag ctacttccac gacttcttca
actacgccgg catccaccgc tcggtcatgc tgtacacgac gccgaacacc tgggtcgacg
acatcacggt ggtcacccac gtggcccagg actgcaacca cgcgtccgtg gactggcagg
tcgtcgccaa cggcgacgtg agcgtggagc tccgcgacgc cgagcagcag gtcgtggcga
ccgggcaggg cacctcgggg accctccagg tcgtgaaccc ccacctctgg cagccgggtg
agggctacct gtacgagctg tgcgtgacgg cgaagagcca gaccgagtgc gacatctacc
ccctgcgcgt cggcatccgg tccgtggcgg tcaagggcga gcagttcctg atcaaccaca
agccgttcta cttcacgggc ttcggtcggc acgaggacgc cgacctccgc ggcaagggct
tcgacaacgt cctgatggtc cacgaccacg ccctgatgga ctggatcggc gccaactcgt
accggacctc gcactacccg tacgcggaag agatgctgga ctgggcggac gagcacggga
tcgtcgtcat cgacgaaacc gccgcggtgg gcttcaacct gtccctcggc atcggcttcg
aggccggcaa caagccgaaa gagctctact ccgaagaggc cgtcaacggc gaaacccagc
aggcccacct ccaggcgatc aaagagctca tcgcccgcga caagaaccac ccgtccgtcg
tgatgtggtc gatcgccaac gagccggaca cccggccgca gggtgcccgg gagtacttcg
ccccgctggc cgaggccacc cggaagctcg accccacccg gcccatcacc tgcgtgaacg
tgatgttctg cgacgcccac acggacacca tctccgacct cttcgacgtc ctgtgcctga
accgctacta cggctggtac gtgcagtccg gggacctgga aacggccgag aaggtcctgg
agaaagagct gctggcgtgg caggagaagc tgcaccagcc gatcatcatc accgagtacg
gcgtggacac gctggccggt ctgcactcca tgtacaccga catgtggtcc gaagagtacc
agtgcgcgtg gctggacatg taccaccgcg tgttcgaccg cgtcagcgcg gtcgtcggcg
agcaggtgtg gaacttcgcc gacttcgcca cctcccaggg catcctccgc gtcgggggca
acaagaaggg catcttcacc cgggaccgca agcccaagtc ggcggcgttc ctgctccaga
agcggtggac cgggatgaac ttcggggaga agccgcagca gggcgggaag cagtgataag
cattacccgg atccagatat cctgcaggaa ttcctcgagc cgatatccct ctagaccgcg
gccgcccggc tgcagcccgt acctctagta gagtcgaggg ggaacccggc cgcgtccggc
gcccccgccg ccttcgacga gatcccgcaa aagcggcctt tgactccctg caagcctcag
cgaccgaata tatcggttat gcgtgggcga tggttgttgt cattgtcggc gcaactatcg
gtatcaagct gtttaagaaa ttcacctcga aagcaagctg ataaaccgat acaattaaag
 
gctccttttg gagccttttt ttttggagat tttcaacgtg aaaaaattat tattcgcaat
tcctttagtt gttcctttct attctcactc cgctgaaact gttgaaagtt gtttagcaaa
acctcataca gaaaattcat ttactaacgt ctggaaagac gacaaaactt tagatcctct
agctagagtc gaccggcatg caatcgaatt cgtaatcatg tcatagctgt ttcctgtgtg
aaattgttat ccgctcacaa ttccacacaa catacgagcc ggaagcataa agtgtaaagc
ctggggtgcc taatgagtga gctaactcac attaattgcg ttgcgctcac tgcccgcttt
ccagtcggga aacctgtcgt gccagctgca ttaatgaatc ggccaacgcg cggggagagg
cggtttgcgt attgggcgct cttccgcttc ctcgctcact gactcgctgc gctcggtcgt
tcggctgcgg cgagcggtat cagctcactc aaaggcggta atacggttat ccacagaatc
aggggataac gcaggaaaga acatgtgagc aaaaggccag caaaaggcca ggaaccgtaa
aaaggccgcg ttgctggcgt ttttccatag gctccgcccc cctgacgagc atcacaaaaa
tcgacgctca agtcagaggt ggcgaaaccc gacaggacta taaagatacc aggcgtttcc
ccctggaagc tccctcgtgc gctctcctgt tccgaccctg ccgcttaccg gatacctgtc
cgcctttctc ccttcgggaa gcgtggcgct ttctcatagc tcacgctgta ggtatctcag
ttcggtgtag gtcgttcgct ccaagctggg ctgtgtgcac gaaccccccg ttcagcccga
ccgctgcgcc ttatccggta actatcgtct tgagtccaac ccggtaagac acgacttatc
gccactggca gcagccactg gtaacaggat tagcagagcg aggtatgtag gcggtgctac
agagttcttg aagtggtggc ctaactacgg ctacactaga agaacagtat ttggtatctg
cgctctgctg aagccagtta ccttcggaaa aagagttggt agctcttgat ccggcaaaca
aaccaccgct ggtagcggtg gtttttttgt ttgcaagcag cagattacgc gcagaaaaaa
aggatctcaa gaagatcctt tgatcttttc tacggggtct gacgctcagt ggaacgaaaa
ctcacgttaa gggattttgg tcatgagatt atcaaaaagg atcttcacct agatcctttt
ggttcatgtg cagctccatc agcaaaaggg gatgataagt ttatcaccac cgactatttg
caacagtgcc gttgatcgtg ctatgatcga ctgatgtcat cagcggtgga gtgcaatgtc
gtgcaatacg aatggcgaaa agccgagctc atcggtcagc ttctcaacct tggggttacc
cccggcggtg tgctgctggt ccacagctcc ttccgtagcg tccggcccct cgaagatggg
ccacttggac tgatcgaggc cctgcgtgct gcgctgggtc cgggagggac gctcgtcatg
ccctcgtggt caggtctgga cgacgagccg ttcgatcctg ccacgtcgcc cgttacaccg
gaccttggag ttgtctctga cacattctgg cgcctgccaa atgtaaagcg cagcgcccat
ccatttgcct ttgcggcagc ggggccacag gcagagcaga tcatctctga tccattgccc
ctgccacctc actcgcctgc aagcccggtc gcccgtgtcc atgaactcga tgggcaggta
cttctcctcg gcgtgggaca cgatgccaac acgacgctgc atcttgccga gttgatggca
aaggttccct atggggtgcc gagacactgc accattcttc aggatggcaa gttggtacgc
gtcgattatc tcgagaatga ccactgctgt gagcgctttg ccttggcgga caggtggctc
aaggagaaga gccttcagaa ggaaggtcca gtcggtcatg cctttgctcg gttgatccgc
tcccgcgaca ttgtggcgac agccctgggt caactgggcc gagatccgtt gatcttcctg
catccgccag aggcgggatg cgaagaatgc gatgccgctc gccagtcgat tggctgagct
catgagcgga gaacgagatg acgttggagg ggcaaggtcg cgctgattgc tggggcaaca
cgtggagcgg atcggggatt gtctttcttc agctcgctga tgatatgctg acgctcaatg
ccgtttggcc tccgactaac gaaaatcccg catttggacg gctgatccga ttggcacggc
ggacggcgaa tggcggagca gacgctcgtc cgggggcaat gagatatgaa aaagcctgaa
ctcaccgcga cgtatcgggc cctggccagc tagctagagt cgacctgcag gtccccgggg
atcggtcttg ccttgctcgt cggtgatgta cttcaccagc tccgcgaagt cgctcttctt
gatggagcgc atggggacgt gcttggcaat cacgcgcacc ccccggccgt tttagcggct
aaaaaagtca tggctctgcc ctcgggcgga ccacgcccat catgaccttg ccaagctcgt
cctgcttctc ttcgatcttc gccagcaggg cgaggatcgt ggcatcaccg aaccgcgccg
tgcgcgggtc gtcggtgagc cagagtttca gcaggccgcc caggcggccc aggtcgccat
tgatgcgggc cagctcgcgg acgtgctcat agtccacgac gcccgtgatt ttgtagccct
ggccgacggc cagcaggtag gccgacaggc tcatgccggc cgccgccgcc ttttcctcaa
tcgctcttcg ttcgtctgga aggcagtaca ccttgatagg tgggctgccc ttcctggttg
gcttggtttc atcagccatc cgcttgccct catctgttac gccggcggta gccggccagc
ctcgcagagc aggattcccg ttgagcaccg ccaggtgcga ataagggaca gtgaagaagg
aacacccgct cgcgggtggg cctacttcac ctatcctgcc cggctgacgc cgttggatac
accaaggaaa gtctacacga accctttggc aaaatcctgt atatcgtgcg aaaaaggatg
gatataccga aaaaatcgct ataatgaccc cgaagcaggg ttatgcagcg gaaaagatcc
gtcgacctgc aggcatgc
SEQ ID NO: 30
   gttgtgggca caatcgtgcc ggttggtagg atccagcg

## Claims

1. A recombinant vector for heterologous expression of a recombinant protein in a *Streptomyces* host cell bearing an expression cassette comprising:
a promoter, a regulatory element, and a polynucleotide encoding a recombinant protein operably linked to said promoter and regulatory element; wherein:
- said promoter is an engineered *kasO* promoter (*kasOp**) having sequence SEQ ID NO: 10;
- said regulatory element is an optimized synthetic SR40 ribosome-binding site (RBS) having sequence SEQ ID NO: 11;
- the polynucleotide encoding for the recombinant protein of interest encodes for a recombinant protein that includes a N-terminal signal peptide; and
- the recombinant vector is a single-site integrating vector.

2. The recombinant vector of claim 1, wherein the recombinant protein is an *Actinoallomurus* endopeptidase of sequence comprising or consisting of SEQ ID NO: 1; a biologically active fragment of E40; a naturally occurring allelic variant of E40; or an endopeptidase of sequence having at least 60%, 70%, 80%, 90% or 95% of identity to SEQ ID NO: 1.

3. The recombinant vector of claim 2, wherein the recombinant protein is a histidine tagged *Actinoallomurus* endopeptidase of sequence comprising SEQ ID NO: 2.

4. The recombinant vector of any one of claims 1 to 2, wherein the signal peptide is vsi signal peptide of subtilisin inhibitor of *Streptomyces venezuelae,* having sequence SEQ ID NO: 12.

5. The recombinant vector of any one of claims 1 to 4, wherein the polynucleotide encoding the recombinant protein is of sequence comprising SEQ ID NO: 5, 6, 15, 16, 17, or 18.

6. The recombinant vector of any one of claims 1 to 4, wherein the expression cassette has sequence SEQ ID NO: 20, 21, 22 or 23.

7. The recombinant vector of any one of claims 1 to 6 being a vector integrating at *attB* site of *Streptomyces.*

8. The expression vector of claim 7 being a vector of sequence SEQ ID NO: 25, 26, 27 or 28.

9. A *Streptomyces* host cell, preferably a *S. lividans* host cell, for heterologous expression of a recombinant protein comprising the expression vector of claims 1-8.

10. The host cell of claim 9 being a *S. lividans* host cell of strain Tk24 or of strain RedStrep 1.3, 1.6 or 1.9.

11. The host cell of claim 9 being *a S. lividans* host cell of strain DSM 33930 or DSM 33931.

12. Use of the recombinant vector of claims 1-8 or of the host cell of claims 9-11 for the production of a recombinant protein, wherein the recombinant protein is secreted in the culture medium of the host cell.

13. Method of producing a recombinant protein, comprising:
i. providing a recombinant host cell comprising the recombinant expression vector of claims 1-8;
ii. culturing the recombinant host cell in a culture medium under suitable growth conditions;
iii. recovering the recombinant protein expressed by the recombinant host cell from the culture medium.

## Patentansprüche

1. Rekombinanter Vektor für die heterologe Expression eines rekombinanten Proteins in einer *Streptomyces*-Wirtszelle, der eine Expressionskassette trägt, die Folgendes umfasst:
einen Promotor, ein regulatorisches Element und ein Polynukleotid, das ein rekombinantes Protein kodiert, das funktionell mit dem Promotor und dem regulatorischen Element verbunden ist; wobei:
- der Promotor ein technisch veränderter *kasO*-Promotor *(kasOp*)* mit der Sequenz SEQ ID NO: 10 ist;
- das regulatorische Element eine optimierte synthetische SR40-Ribosomenbindungsstelle (RBS) mit der Sequenz SEQ ID NO: 11 ist;
- das Polynukleotid, das für das rekombinante Protein von Interesse kodiert, für ein rekombinantes Protein kodiert, das ein N-terminales Signalpeptid beinhaltet; und
- der rekombinante Vektor ein Single-Site-Integrationsvektor ist.

2. Rekombinanter Vektor nach Anspruch 1, wobei das rekombinante Protein eine *Actinoallomurus*-Endopeptidase einer Sequenz, die SEQ ID NO: 1 umfasst oder daraus besteht; ein biologisch aktives Fragment von E40; eine natürlich vorkommende allele Variante von E40; oder eine Endopeptidase einer Sequenz mit einer Identität von mindestens 60 %, 70 %, 80 %, 90 % oder 95 % mit SEQ ID NO: 1 ist.

3. Rekombinanter Vektor nach Anspruch 2, wobei das rekombinante Protein eine Histidinmarkierte *Actinoallomurus*-Endopeptidase einer Sequenz ist, die SEQ ID NO: 2 umfasst.

4. Rekombinanter Vektor nach einem der Ansprüche 1 bis 2, wobei das Signalpeptid ein vsi-Signalpeptid eines Subtilisininhibitors von *Streptomyces venezuelae* mit der Sequenz SEQ ID NO: 12 ist.

5. Rekombinanter Vektor nach einem der Ansprüche 1 bis 4, wobei das Polynukleotid, das das rekombinante Protein kodiert, eine Sequenz ist, die SEQ ID NO: 5, 6, 15, 16, 17 oder 18 umfasst.

6. Rekombinanter Vektor nach einem der Ansprüche 1 bis 4, wobei die Expressionskassette die Sequenz SEQ ID NO: 20, 21, 22 oder 23 aufweist.

7. Rekombinanter Vektor nach einem der Ansprüche 1 bis 6, der ein Vektor ist, der an der *attB*-Site von *Streptomyces* integriert.

8. Expressionsvektor nach Anspruch 7, der ein Vektor der Sequenz SEQ ID NO: 25, 26, 27 oder 28 ist.

9. *Streptomyces-Wirtszelle,* vorzugsweise *S. lividans*-Wirtszelle, zur heterologen Expression eines rekombinanten Proteins, umfassend den Expressionsvektor nach den Ansprüchen 1-8.

10. Wirtszelle nach Anspruch 9, die eine *S. lividans*-Wirtszelle des Stamms Tk24 oder des Stamms RedStrep 1.3, 1.6 oder 1.9 ist.

11. Wirtszelle nach Anspruch 9, die eine *S. lividans*-Wirtszelle des Stamms DSM 33930 oder DSM 33931 ist.

12. Verwendung des rekombinanten Vektors nach den Ansprüchen 1-8 oder der Wirtszelle nach den Ansprüchen 9-11 zur Herstellung eines rekombinanten Proteins, wobei das rekombinante Protein in dem Kulturmedium der Wirtszelle sekretiert wird.

13. Verfahren zum Herstellen eines rekombinanten Proteins, umfassend:
i. Bereitstellen einer rekombinanten Wirtszelle, die den rekombinanten Expressionsvektor nach den Ansprüchen 1-8 umfasst;
ii. Kultivieren der rekombinanten Wirtszelle in einem Kulturmedium unter geeigneten Wachstumsbedingungen;
iii. Gewinnen des rekombinanten Proteins, das durch die rekombinante Wirtszelle exprimiert wird, aus dem Kulturmedium.

## Revendications

1. Vecteur recombinant pour l'expression hétérologue d'une protéine recombinante dans une cellule hôte de *Streptomyces* portant une cassette d'expression comprenant :
un promoteur, un élément régulateur et un polynucléotide codant pour une protéine recombinante liée de manière opérationnelle audit promoteur et audit élément régulateur ; dans lequel :
- ledit promoteur est un promoteur *kasO* modifié (*kasOp**) ayant la séquence SEQ ID NO : 10 ;
- ledit élément régulateur est un site de fixation du ribosome (RBS) SR40 synthétique optimisé ayant la séquence SEQ ID NO : 11 ;
- le polynucléotide codant pour la protéine recombinante d'intérêt code pour une protéine recombinante qui comprend un peptide signal N-terminal ; et
- le vecteur recombinant est un vecteur intégrateur à site unique.

2. Vecteur recombinant selon la revendication 1, dans lequel la protéine recombinante est une endopeptidase d'*Actinoallomurus* de séquence comprenant ou consistant en SEQ ID NO : 1 ; un fragment biologiquement actif de E40 ; un variant allélique naturel de E40 ; ou une endopeptidase de séquence ayant au moins 60 %, 70 %, 80 %, 90 % ou 95 % d'identité avec la SEQ ID NO : 1.

3. Vecteur recombinant selon la revendication 2, dans lequel la protéine recombinante est une endopeptidase d'*Actinoallomurus* marquée à l'histidine de séquence comprenant ou consistant en SEQ ID NO : 2.

4. Vecteur recombinant selon l'une quelconque des revendications 1 à 2, dans lequel le peptide signal est le peptide signal vsi de l'inhibiteur de subtilisine de *Streptomyces venezuelae,* ayant la séquence SEQ ID NO : 12.

5. Vecteur recombinant selon l'une quelconque des revendications 1 à 4, dans lequel le polynucléotide codant pour la protéine recombinante a la séquence comprenant SEQ ID NO : 5, 6, 15, 16, 17 ou 18.

6. Vecteur recombinant selon l'une quelconque des revendications 1 à 4, dans lequel la cassette d'expression a la séquence SEQ ID NO : 20, 21, 22 ou 23.

7. Vecteur recombinant selon l'une quelconque des revendications 1 à 6 qui est un vecteur d'intégration au site *attB* de *Streptomyces.*

8. Vecteur d'expression selon la revendication 7 qui est un vecteur de séquence SEQ ID NO : 25, 26, 27 ou 28.

9. Cellule hôte de *Streptomyces,* de préférence une cellule hôte de *S. lividans,* pour l'expression hétérologue d'une protéine recombinante comprenant le vecteur d'expression selon les revendications 1 à 8.

10. Cellule hôte selon la revendication 9 qui est une cellule hôte de *S. lividans* de la souche Tk24 ou de la souche RedStrep 1.3, 1.6 ou 1.9.

11. Cellule hôte selon la revendication 9 qui est une cellule hôte de *S. lividans* de la souche DSM 33930 ou DSM 33931.

12. Utilisation du vecteur recombinant selon les revendications 1 à 8 ou de la cellule hôte selon les revendications 9 à 11 pour la production d'une protéine recombinante, ladite protéine recombinante étant sécrétée dans le milieu de culture de la cellule hôte.

13. Procédé de production d'une protéine recombinante, comprenant :
i. la fourniture d'une cellule hôte recombinante comprenant le vecteur d'expression recombinant selon les revendications 1 à 8 ;
ii. la culture de la cellule hôte recombinante dans un milieu de culture dans des conditions de croissance appropriées ;
iii. la récupération de la protéine recombinante exprimée par la cellule hôte recombinante dans le milieu de culture.
